# EUROPEAN PATENT APPLICATION

(11) **EP 4 365 201 A1**
(43) Date of publication of application: **08.05.2024**
(21) Application number: 22832089.1
(22) Date of filing: 29.06.2022
(51) Int. Cl.: C07K 16/28, C12N 15/13, C12N 15/63, A61K 39/395, A61P 35/00, A61P 3/04

(54) **ANTI-GFRAL ANTIBODY AND APPLICATION THEREOF**

(30) Priority: 30.06.2021 CN 202110739877
(71) Applicant: Shanghai JMT-Bio Technology Co., Ltd., Shanghai 200032 (CN)
(72) Inventor: LIU, Qian, Shanghai 201318 (CN); REN, Baolan, Shanghai 201318 (CN); FENG, Xu, Shanghai 201318 (CN); SONG, Liping, Shanghai 201318 (CN); FAN, Yi, Shanghai 201318 (CN); YANG, Li, Shanghai 201318 (CN)
(74) Representative: Mewburn Ellis LLP
(86) International application number: PCT/CN2022/102174
(87) International publication number: WO 2023/274276

(57) **Abstract**

An anti-GFRAL antibody and a use thereof. The anti-GFRAL antibody or an antigen-binding portion thereof can be used for treating or preventing GFRAL-mediated or GFRAL-RET-GDF 15 signaling pathway-mediated diseases or abnormalities, particularly cachexia syndrome.

## Description

### CROSS REFERECE OF RELATED APPLICATION

The present application claims priority to Chinese Patent Application No. 202110739877.2 filed on June 30, 2021, which is hereby incorporated by reference in its entirety for all purposes.

### TECHNICAL FIELD

The present application generally relates to the biopharmaceutical field, and in particular, to novel anti-GFRAL antibodies or antigen-binding fragments thereof, pharmaceutical compositions comprising the antibodies or antigen-binding fragments thereof, and medical and pharmaceutical uses of the antibodies or antigen-binding fragments thereof.

### BACKGROUND

Growth Differentiation Factor 15 (hereinafter referred to as GDF15) is a member of the transforming growth factor β (TGF-β) superfamily. There are more than 30 members in the TGF-β superfamily, such as transforming growth factor β, activin, bone morphogenetic protein (BMP), and growth differentiation factor (GDF). They play an important role in regulating development, cell differentiation, and tissue repair in various organs¹.

Under normal physiological conditions, GDF15 is weakly expressed in most tissues. When inflammation or tissue damage occurs, the expression of GDF15 is significantly elevated. GDF15 is therefore considered to be an inflammatory marker and is also involved in the pathological processes of multiple diseases, such as tumors, ischemic diseases, metabolic disorders, and neurodegenerative diseases.

The researchers have found that GDF15 can cause anorexia and weight loss in preclinical animal models. It has also been clinically shown that GDF15 levels are significantly elevated in the blood of patients with various advanced tumors, and that GDF15 levels are significantly associated with tumor cachexia and poor survival. In 2017, several research groups simultaneously discovered GFRAL (GDNF family receptor alpha-like), a receptor of GDF15 on brain stem neurons, and demonstrated that GFRAL-RET signaling complexes mediated GDF15-induced weight loss^{2,3,4}. GFRAL is specifically expressed in the end region of the brain stem (AP, Area postrema) and in the solitary tract nucleus (STN). It has been found that GDF15 binds GFRAL and forms a six-membered complex with its co-receptor RET, causing activation of RET and further activation of downstream extracellular signal-regulated kinases (Erk1/2, extracellular signal-regulated kinase), protein kinase B (PKB/AKT, Ak strain tranforming), and phospholipase gamma (PLCγ: phospholipase C-gamma) signaling pathways ^{2, 3, 4}. *In vivo* experimental data indicate that activation of the GFRAL-RET-GDF15 signaling pathway can induce expression of lipid metabolism related genes in adipose tissues through peripheral sympathetic nerves, resulting in decreased mass and function of fat and muscle in tumor-bearing mice. Accordingly, blocking the GFRAL-RET-GDF15 signaling pathway has the potential to inhibit weight loss in cancer cachexia patients.

CACS (Cancer anorexia-cachexia syndrome) is a complex metabolic syndrome clinically characterized by uncontrolled weight loss in cancer patients, including weight loss due to anorexia, muscle mass reduction, and decreased adipose tissue. Cachexia often occurs in end-stage cancer patients, is mainly associated with poor life quality and reduced survival in cancer patients, and accounts for about one quarter of all cancer-related deaths.

At present, no single drug has been widely accepted by major drug regulatory agencies in the world for the treatment of cachexia. For tumor cachexia patients, only progesterone, progesterone analogs and glucocorticoids are recommended as short-term intervention regimen in the 2020 US Clinical Oncology Guidelines ⁵.

The etiology and pathology of cachexia are complex. According to related studies, GDF15 (Growth differentiation factor 15) is a mediator of cachexia caused by cancer (including a cancer with reduced sensitivity to chemotherapeutic agents) and chronic diseases. Epidemic studies have shown that GDF15 serum levels are positively correlated with the severity of cachexia. Clinical study data indicate that, in tissue damage, inflammation, and various disease conditions, including a cancer, cardiovascular diseases, and kidney diseases, GDF15 is significantly increased in the blood circulation, and elevated serum levels of GDF15 are associated with uncontrolled weight loss and poor prognosis.

The GFRAL-RET-GDF15 signaling pathway is a potential therapeutic target for the above-mentioned conditions, and development and use of anti-GFRAL antibodies is urgently needed in the art.

### SUMMARY OF THE INVENTION

In a first aspect, the present application provides an anti-GFRAL antibody or an antigen-binding portion thereof comprising a heavy chain variable region and/or a light chain variable region, wherein the heavy chain variable region comprises a heavy chain CDR1 (HCDR1), a heavy chain CDR2 (HCDR2), and a heavy chain CDR3 (HCDR3), and wherein the light chain variable region comprises a light chain CDR1 (LCDR1), a light chain CDR2 (LCDR2), and a light chain CDR3 (LCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO. 116 or has at least 80%, 85%, 90% or 95% identity to the sequence as set forth in SEQ ID NO.76 or SEQ ID NO. 116, the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99, the sequence of the light chain CDR1 is the sequence as set forth in SEQ ID NO.79 or SEQ ID NO. 126 or SEQ ID NO. 128 or has at least 80%, 85%, 90% or 95% identity to the sequence as set forth in SEQ ID NO.79 or SEQ ID NO. 126 or SEQ ID NO. 128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO. 123 or has at least 80%, 85%, 90% or 95% identity to the sequence as set forth in SEQ ID NO.50 or SEQ ID NO. 123; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, the sequence of the heavy chain CDR3 is the sequence as set forth in SEQ ID NO.70 or has at least 80%, 85%, 90% or 95% identity to the sequence as set forth in SEQ ID NO.70, the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, the sequence of the heavy chain CDR3 is the sequence as set forth in SEQ ID NO.3 or has at least 80%, 85%, 90% or 95% identity to the sequence as set forth in SEQ ID NO.3, the sequence of the light chain CDR1 is the sequence as set forth in SEQ ID NO.5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO.10 or has at least 80%, 85%, 90% or 95% identity to the sequence as set forth in SEQ ID NO.10, the sequence of the heavy chain CDR3 is the sequence as set forth in SEQ ID NO.11 or has at least 80%, 85%, 90% or 95% identity to the sequence as set forth in SEQ ID NO.11, the sequence of the light chain CDR1 is the sequence as set forth in SEQ ID NO.13 or has at least 80%, 85%, 90% or 95% identity to the sequence as set forth in SEQ ID NO.13 the sequence of the light chain CDR2 is the sequence as set forth in SEQ ID NO.14 or has at least 80%, 85%, 90% or 95% identity to the sequence as set forth in SEQ ID NO.14, and the sequence of the light chain CDR3 is the sequence as set forth in SEQ ID NO.15 or has at least 80%, 85%, 90% or 95% identity to the sequence as set forth in SEQ ID NO.15; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, the sequence of the heavy chain CDR3 is the sequence as set forth in SEQ ID NO.19 or has at least 80%, 85%, 90% or 95% identity to the sequence as set forth in SEQ ID NO.19, the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, the sequence of the heavy chain CDR3 is the sequence as set forth in SEQ ID NO.26 or has at least 80%, 85%, 90% or 95% identity to the sequence as set forth in SEQ ID NO.26, the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, the sequence of the heavy chain CDR3 is the sequence as set forth in SEQ ID NO.33 or has at least 80%, 85%, 90% or 95% identity to the sequence as set forth in SEQ ID NO.33, the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, the sequence of the heavy chain CDR3 is the sequence as set forth in SEQ ID NO.41 or has at least 80%, 85%, 90% or 95% identity to the sequence as set forth in SEQ ID NO.41, the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, the sequence of the heavy chain CDR3 is the sequence as set forth in SEQ ID NO.47 or has at least 80%, 85%, 90% or 95% identity to the sequence as set forth in SEQ ID NO.47, the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, the sequence of the heavy chain CDR3 is the sequence as set forth in SEQ ID NO.54 or has at least 80%, 85%, 90% or 95% identity to the sequence as set forth in SEQ ID NO.54, the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, the sequence of the heavy chain CDR3 is the sequence as set forth in SEQ ID NO.62 or has at least 80%, 85%, 90% or 95% identity to the sequence as set forth in SEQ ID NO.62, the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, the sequence of the heavy chain CDR3 is the sequence as set forth in SEQ ID NO.84 or has at least 80%, 85%, 90% or 95% identity to the sequence as set forth in SEQ ID NO.84, the sequence of the light chain CDR1 is the sequence as set forth in SEQ ID NO.86 or has at least 80%, 85%, 90% or 95% identity to the sequence as set forth in SEQ ID NO.86 the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the heavy chain CDR1 to 3 and the light chain CDR1 to 3 are defined according to the IMGT definition.

In a second aspect, the present application provides an anti-GFRAL antibody or an antigen-binding portion thereof, comprising a heavy chain variable region and a light chain variable region, wherein:
the heavy chain variable region has the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121, or has an amino acid sequence having at least 80%, at least 90%, at least 95% or at least 99% identity to the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121; and/or
the light chain variable region has the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134 or 135, or has an amino acid sequence having at least 80%, at least 90%, at least 95% or at least 99% identity to the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134, or 135.

In a third aspect, the present application provides an anti-GFRAL antibody or an antigen-binding portion thereof comprising a heavy chain variable region, wherein the heavy chain variable region comprises a heavy chain CDR1 (HCDR1), a heavy chain CDR2 (HCDR2), and a heavy chain CDR3 (HCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO.116 or has at least 80%, 85%, 90% or 95% identity to the sequence as set forth in SEQ ID NO.76 or SEQ ID NO.116, and the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, and the sequence of the heavy chain CDR3 is the sequence as set forth in SEQ ID NO.70 or has at least 80%, 85%, 90% or 95% identity to the sequence as set forth in SEQ ID NO.70; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, and the sequence of the heavy chain CDR3 is the sequence as set forth in SEQ ID NO.3 or has at least 80%, 85%, 90% or 95% identity to the sequence as set forth in SEQ ID NO.3; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO.10 or has at least 80%, 85%, 90% or 95% identity to the sequence as set forth in SEQ ID NO.10, and the sequence of the heavy chain CDR3 is the sequence as set forth in SEQ ID NO.11 or has at least 80%, 85%, 90% or 95% identity to the sequence as set forth in SEQ ID NO.11; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, and the sequence of the heavy chain CDR3 is the sequence as set forth in SEQ ID NO.19 or has at least 80%, 85%, 90% or 95% identity to the sequence as set forth in SEQ ID NO.19; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, and the sequence of the heavy chain CDR3 is the sequence as set forth in SEQ ID NO.26 or has at least 80%, 85%, 90% or 95% identity to the sequence as set forth in SEQ ID NO.26; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, and the sequence of the heavy chain CDR3 is the sequence as set forth in SEQ ID NO.33 or has at least 80%, 85%, 90% or 95% identity to the sequence as set forth in SEQ ID NO.33; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, and the sequence of the heavy chain CDR3 is the sequence as set forth in SEQ ID NO.41 or has at least 80%, 85%, 90% or 95% identity to the sequence as set forth in SEQ ID NO.41; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, and the sequence of the heavy chain CDR3 is the sequence as set forth in SEQ ID NO.47 or has at least 80%, 85%, 90% or 95% identity to the sequence as set forth in SEQ ID NO.47; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, and the sequence of the heavy chain CDR3 is the sequence as set forth in SEQ ID NO.54 or has at least 80%, 85%, 90% or 95% identity to the sequence as set forth in SEQ ID NO.54; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, and the sequence of the heavy chain CDR3 is the sequence as set forth in SEQ ID NO.62 or has at least 80%, 85%, 90% or 95% identity to the sequence as set forth in SEQ ID NO.62; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, and the sequence of the heavy chain CDR3 is the sequence as set forth in SEQ ID NO.84 or has at least 80%, 85%, 90% or 95% identity to the sequence as set forth in SEQ ID NO.84;
wherein the amino acid sequences of the heavy chain CDR1 to 3 are defined according to the IMGT definition.

In a fourth aspect, the present application provides an anti-GFRAL antibody or an antigen-binding portion thereof comprising a light chain variable region, wherein the light chain variable region comprises a light chain CDR1 (HCDR1), a light chain CDR2 (HCDR2), and a light chain CDR3 (HCDR3), wherein:
(1) the sequence of the light chain CDR1 is the sequence as set forth in SEQ ID NO.79 or SEQ ID NO.126 or SEQ ID NO.128 or has at least 80%, 85%, 90% or 95% identity to the sequence as set forth in SEQ ID NO.79 or SEQ ID NO.126 or SEQ ID NO.128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO.123 or has at least 80%, 85%, 90% or 95% identity to the sequence as set forth in SEQ ID NO.50 or SEQ ID NO. 123; or
(2) the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the light chain CDR1 is the sequence as set forth in SEQ ID NO.5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the light chain CDR1 is the sequence as set forth in SEQ ID NO. 13 or has at least 80%, 85%, 90% or 95% identity to the sequence as set forth in SEQ ID NO. 13 the sequence of the light chain CDR2 is the sequence as set forth in SEQ ID NO. 14 or has at least 80%, 85%, 90% or 95% identity to the sequence as set forth in SEQ ID NO. 14, and the sequence of the light chain CDR3 is the sequence as set forth in SEQ ID NO. 15 or has at least 80%, 85%, 90% or 95% identity to the sequence as set forth in SEQ ID NO. 15; or
(5) the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the light chain CDR1 is the sequence as set forth in SEQ ID NO.86 or has at least 80%, 85%, 90% or 95% identity to the sequence as set forth in SEQ ID NO.86 the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the light chain CDR1 to 3 are defined according to the IMGT definition.

In a fifth aspect, the present application provides an anti-GFRAL antibody or an antigen-binding portion thereof comprising a heavy chain variable region and a light chain variable region, wherein:
(i) the heavy chain variable region comprises three heavy chain CDR regions, wherein the amino acid sequence of at least one of the heavy chain CDR regions is selected from the group consisting of SEQ ID NO.1, SEQ ID NO.9, SEQ ID NO.17, SEQ ID NO.24, SEQ ID NO.31, SEQ ID NO.39, SEQ ID NO.45, SEQ ID NO.60, SEQ ID NO.68, SEQ ID NO.75, SEQ ID NO.82, SEQ ID NO.2, SEQ ID NO.10, SEQ ID NO.18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99, or has at least 80% (preferably 85%, 90%, 95%, 98% or 99%) sequence identity to an amino acid sequence selected from the group consisting of SEQ ID NO.1, SEQ ID NO.9, SEQ ID NO. 17, SEQ ID NO.24, SEQ ID NO.31, SEQ ID NO.39, SEQ ID NO.45, SEQ ID NO.60, SEQ ID NO.68, SEQ ID NO.75, SEQ ID NO.82, SEQ ID NO.2, SEQ ID NO. 10, SEQ ID NO. 18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99; and/or
(Ii) the light chain variable region comprises three light chain CDR regions, wherein the amino acid sequence of at least one of the light chain CDR regions is selected from the group consisting of SEQ ID NO.5, SEQ ID NO.13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, SEQ ID NO.128, SEQ ID NO.6, SEQ ID NO.14, SEQ ID NO.36, SEQ ID NO.57, SEQ ID NO.65, SEQ ID NO.80, SEQ ID NO.87, SEQ ID NO.7, SEQ ID NO. 15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO. 123, or has at least 80% (preferably 85%, 90%, 95%, 98% or 99%) sequence identity to an amino acid sequence selected from the group consisting of SEQ ID NO.5, SEQ ID NO. 13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, SEQ ID NO.128, SEQ ID NO.6, SEQ ID NO.14, SEQ ID NO.36, SEQ ID NO.57, SEQ ID NO.65, SEQ ID NO.80, SEQ ID NO.87, SEQ ID NO.7, SEQ ID NO. 15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO. 123;
wherein the amino acid sequences of the heavy chain CDR1 to 3 and the light chain CDR1 to 3 are defined according to the IMGT definition.

In a sixth aspect, the present application provides an isolated nucleic acid molecule encoding the anti-GFRAL antibody or antigen-binding portion thereof of any one of the first to fifth aspects.

In a seventh aspect, the present application provides a vector comprising the nucleic acid molecule of the sixth aspect.

In an eighth aspect, the present application provides a host cell comprising the nucleic acid molecule of the sixth aspect or the vector of the seventh aspect.

In a ninth aspect, the present application provides an antibody-drug conjugate comprising the anti-GFRAL antibody or antigen-binding portion thereof of any one embodiment of the first to fifth aspects conjugated with a therapeutic agent.

In a tenth aspect, the present application provides a pharmaceutical composition comprising the anti-GFRAL antibody or an antigen-binding portion thereof of any one embodiment of the first to fifth aspects or the antibody-drug conjugate of the ninth aspect, and a pharmaceutically acceptable carrier.

In an eleventh aspect, the present application provides the use of the antibody or antigen-binding portion thereof of any one embodiment of the first to fifth aspects, the nucleic acid molecule of the sixth aspect, the vector of the seventh aspect, the host cell of the eighth aspect, or the antibody-drug conjugate of the ninth aspect in the manufacture of a medicament for the treatment or prevention of a GFRAL-mediated or GFRAL-RET-GDF15 signaling pathway-mediated disease or abnormality, e.g., cachexia syndrome, a tumor (including a cancer), a cardiovascular disease, a renal disease, an ischemic disease, a metabolic disorder, a neurodegenerative disease, anorexia, or weight loss due to anorexia.

In a twelfth aspect, the present application provides a method of treating a GFRAL-mediated or GFRAL-RET-GDF15 signaling pathway-mediated disease or abnormality in a subject, the method comprising administering to the subject a therapeutically effective amount of the antibody or antigen-binding portion thereof of any one embodiment of the first to fifth aspects, the antibody-drug conjugate of the ninth aspect, or the pharmaceutical composition of the tenth aspect, for example, the disease or abnormality is a cachexia syndrome, a tumor (including a cancer), a cardiovascular disease, a renal disease, an ischemic disease, a metabolic disorder, a neurodegenerative disease, anorexia or weight loss caused by anorexia.

In a thirteenth aspect, the present application provides a conjugate comprising the anti-GFRAL antibody or antigen-binding portion thereof of any one embodiment of the first to fifth aspects and a detectable label.

In a fourteenth aspect, the present application provides a fusion protein comprising the anti-GFRAL antibody or antigen-binding portion thereof of any one embodiment of the first to fifth aspects.

### BRIEF DESCRIPTION OF THE DRAWINGS

FIGs. 1A to 1D show the results of the binding kinetics tests of exemplary antibodies of the present application, in which FIG. 1A shows the result from 168A7 hGFRAL antibody, FIG. 1B shows the result from 8E10 hGFRAL antibody, FIG. 1C shows the result from 26D7 hGFRAL antibody, and FIG. 1D shows the result from 26D7 mGFRAL antibody.
FIG. 2 is a graph showing the percent changes in overall body weights of individual experimental groups over the treatment period in a human fibrosarcoma HT-1080 tumor-bearing model.
FIG. 3 shows the results of gastrocnemius muscle weight detection for the anti-GFRAL antibody in a human fibrosarcoma HT-1080 tumor-bearing model.
FIG. 4 shows the results of subcutaneous fat tissue weight detection for the anti-GFRAL antibody in a human fibrosarcoma HT-1080 tumor-bearing model.
FIG. 5 shows the results of weight loss monitoring for the anti-GFRAL antibody in an AAV-GDF15 model.
FIG. 6 shows the results of weight loss monitoring for the humanized anti-GFRAL antibody (26D7H3aL2c) in an AAV-GDF15 model.
FIG. 7 shows the results of food intake monitoring for the humanized anti-GFRAL antibody (26D7H3aL2c) in an AAV-GDF15 model.
FIG. 8 shows the results of weight loss monitoring for the humanized anti-GFRAL antibody (26D7H5bL5a) in an AAV-GDF15 model.
FIG. 9 shows the results of food intake monitoring for the humanized anti-GFRAL antibody (26D7H5bL5a) in an AAV-GDF15 model.
FIG. 10 is a graph showing the percent changes in overall body weights of individual experimental groups over the treatment period in a human fibrosarcoma HT-1080 tumor-bearing model.
FIG. 11 shows the percent changes in net body weights of individual experimental groups over the treatment period in a human fibrosarcoma HT-1080 tumor-bearing model.

### DETAILED DESCRIPTION OF THE INVENTION

### DEFINITIONS

Unless defined otherwise, all scientific and technical terms used herein have the same meaning as understood by one of ordinary skill in the art. For definitions and terms in the art, a person skilled in the art can refer specifically to Current Protocols in Molecular Biology (Ausubel). Abbreviations for amino acid residues are standard 3-letter and/or 1-letter codes used in the art to refer to one of the 20 common L-amino acids.

Although the numerical ranges and parameter approximations are shown in broad ranges in the present application, the numerical values shown in the specific embodiments are described as accurately as possible. However, any numerical values inherently contain certain errors due to the standard deviation present in their respective measurements. Additionally, all ranges disclosed herein are to be understood as encompassing any and all subranges contained therein. For example, a range of "1 to 10" should be considered to encompass any and all subranges between the minimum value of 1 and the maximum value of 10, inclusive, i.e., all subranges starting with a minimum value of 1 or more, such as 1 to 6.1, and subranges ending with a maximum value of 10 or less, such as 5.5 to 10. Additionally, any reference referred to as "incorporated herein" is to be understood as being incorporated in its entirety.

As used herein, the term "subject" or "individual" refers to mammal, such as human, as well as other animals, such as wild animals, domestic animals, or experimental animals (e.g., gorillas, monkeys, rats, mice, rabbits, guinea pigs, woodchucks, or ground squirrels).

An "antibody", in its broad sense, refer to an immunoglobulin molecule that is capable of specifically binding to a target via at least one antigen recognition site located in the variable region of the immunoglobulin molecule, and thus encompasses an intact antibody/full-length antibody, a single chain of an antibody, or any antigen binding fragment of an antibody (also referred to as an "antigen-binding portion"). When "antibody" and "antigen-binding fragment/antigen-binding portion" appear in the same context, an "antibody" can be understood as the entirety of an "antigen-binding fragment/antigen-binding portion", and they together correspond to the concept of an "antibody" in its broad sense.

A "full-length/intact antibody" refers to a protein comprising at least two heavy (H) chains and two light (L) chains interconnected by disulfide bonds. Each heavy chain contains a heavy chain variable region (VH) and a heavy chain constant region containing three domains CH1, CH2 and CH3. Each light chain contains a light chain variable region (VL) and a light chain constant region containing one domain CL. The VH and VL regions can be further divided into a plurality of regions with high variability, referred to as complementarity determining regions (CDRs). Among CDRs, there are more conservative regions referred to as framework regions (FRs). Each VH or VL consists of three CDRs and four FRs arranged from the amino terminus to the carboxy terminus in the following order: FR1, CDR1, FR2, CDR2, FR3, CDR3, and FR4. These variable regions of heavy and light chains contain binding domains that interact with antigens. The constant region of an antibody can mediate binding of immunoglobulins to tissues or factors in hosts, including various cells of the immune system (e.g., effector cells) and the first component of the classical complement system (Clq). A full-length/intact antibody can be any type of antibodies, such as IgD, IgE, IgG, IgA or IgM antibodies (or subclasses of the above), but not belonging to any particular class. Immunoglobulins can be assigned to different classes depending on the amino acid sequences of the heavy chain constant domains of antibodies. Generally, immunoglobulins have five main classes, i.e., IgA, IgD, IgE, IgG and IgM. Several of these classes can be further classified into subclasses (isotypes), such as IgG1, IgG2, IgG3, IgG4, IgA1 and IgA2. Heavy chain constant domains corresponding to various classes of immunoglobulins are referred to as α, δ, ε, γ and µ, respectively. Subunit structures and three-dimensional structures of various classes of immunoglobulins are well known. Chimeric or humanized antibodies are also encompassed by antibodies according to the present application. It is well known to those skilled in the art that complementarity determining regions (CDRs, typically including CDR1, CDR2 and CDR3) are the subregions in variable regions that most impact on the affinity and specificity of an antibody. CDR sequences in a VH or VL can be defined in multiple common ways, including IMGT, the Chothia definition, and the Kabat definition. For a variable region sequence of a given antibody, the CDR sequences in the VH and VL sequences can be defined according to different definitions. In embodiments of the present application, the CDR amino acid sequences are defined according to the IMGT definition. For the variable region amino acid sequence of a given antibody, the CDR amino acid sequences in the variable region amino acid sequence can be analyzed in a variety of ways.

The term "murine antibody" refers to an antibody derived from the fusion of B cells of immunized mice with myeloma cells, the selection of murine hybridized fusion cells capable of both infinitely proliferating and secreting antibodies, and the screening, preparation and purification of the resulting antibodies. A murine antibody generally has immunogenicity and therefore needs to be subsequently humanized.

The term "humanized antibody" refers to an antibody obtained by grafting CDR sequences derived from another mammal species, such as a mouse species, onto human framework sequences. In order to retain binding affinity, some residues of the backbone segments (referred to as FR) can be modified. Humanized antibodies or fragments thereof according to the present application can be prepared by techniques known to those skilled in the art.

The term "chimeric antibody" refers to an antibody in which the variable region sequence is from one species and the constant region sequence is from another species, e.g., an antibody in which the variable region sequence is from a murine antibody and the constant region sequence is from a human antibody. Chimeric antibodies or fragments thereof according to the present application can be prepared by using genetic recombination techniques. For example, a chimeric antibody can be produced by cloning a recombinant DNA comprising a promoter and a sequence encoding a variable region of a non-human, particularly murine, monoclonal antibody according to the present application, and a sequence encoding a constant region of a human antibody. Chimeric antibodies of the present application encoded by such recombinant genes will be, for example, a murine-human chimera, and its specificity is determined by the variable regions derived from murine DNA and its isoforms are determined by the constant regions derived from human DNA.

The term "partially humanized antibody" refers to an antibody containing a constant region from a human and a variable region (including CDRs) from a non-human, such as a mouse.

The term "semi-humanized antibody" is one type of humanized antibodies, and refers to an antibody in which one antibody chain comprises a murine variable region and the other antibody chain comprises a humanized variable region, i.e., a half humanized antibody.

The term "monoclonal antibody" refers to an antibody obtained from a population of substantially homogeneous antibodies. That is, individual antibodies constituting the population are the same except that naturally occurring mutations can be present in a small number of individuals.

As used herein, the term "antigen-binding fragment" or "antigen-binding portion" are used interchangeably, and in particular refer to antibody fragments such as Fv, Fab, F (ab')₂, or Fab', or any fragment which is capable of increasing the half-life by chemical modification or by incorporation into liposomes, such as addition of poly(alkylene)glycols, such as polyethylene glycol ("pegylated") (referred to as pegylated fragments Fv-PEG, scFv-PEG, Fab-PEG, F(ab')₂-PEG or Fab'-PEG) ("PEG" represents polyethylene glycol), in which the fragments have GFRAL-binding activities. Preferably, a functional fragment consists of or comprises a partial sequence of the heavy or light chain variable region of the antibody from which it is derived. The partial sequence is sufficient to retain the same binding specificity and sufficient affinity as the antibody from which it is derived. Such a functional fragment can comprise at least 5 amino acids, preferably 10, 15, 25, 50 and 100 contiguous amino acids of the antibody sequence from which it is derived. Examples of antigen-binding fragments include, but are not limited to, (1) Fab fragments, which can be monovalent fragments having VL-CL chains and VH-CH1 chains; (2) F(ab')₂ fragments, which can be divalent fragments having two Fab' fragments linked by disulfide bridges of the hinge region (i.e., dimers of Fab'); and (3) Fv fragments having VL and VH domains from a single arm of an antibody.

The term "single chain fragment variable (scFv) refers to a single polypeptide chain formed by a VH domain and a VL domain linked via a peptide linker. A (scFv)₂ comprises two VH domains linked by a peptide linker and two VL domains that are combined with the two VH domains via disulfide bridges.

The term "Fc fragment", "Fc domain", "Fc moiety" or the like refers to a portion of the constant region of an antibody heavy chain, including the hinge region, the CH2 fragment and CH3 fragment of the constant region.

The term "specific binding" refers to a non-random binding reaction between two molecules, such as binding of an antibody to an antigenic epitope.

The term "multi-antibody", also referred to as "multi-specific antibody", is a molecule having binding specificity for at least two different antigens, in which a molecule that binds to only two antigens is also referred to as a dual antibody (i.e., a bispecific antibody, BsAb).

The term "bispecific antibody" refers to an antibody having binding capacities for two antigenic epitopes. The two epitopes can be on different antigens or on a same antigen. Bispecific antibodies can have a variety of structural configurations. For example, a bispecific antibody can consist of two Fc fragments and two binding moieties fused thereto, respectively (similar to a native antibody, except that the two arms bind to different antigenic targets or epitopes). An antigen binding moiety can be in the form of a single chain fragment variable (scfv) or a Fab fragment.

Generally, in order to prepare a monoclonal antibody or a functional fragment thereof, in particular a murine monoclonal antibody or a functional fragment thereof, guidelines can be found in the techniques described in "Antibodies" manual (Harlow and Lane, Antibodies: A Laboratory Manual, Cold Spring Harbor Laboratory, Cold Spring Harbor NY, pp.726, 1988) or techniques for preparing a monoclonal antibody from hybridoma cells described by Kohler and Milstein (Nature, 256: 495-497, 1975).

The term "conservative variation" or "conservative amino acid substitutions" refers to a substitution that does not substantially affect or reduce the affinity of a protein, such as the affinity of an antibody for GFRAL. For example, a human antibody that specifically binds to GFRAL can include up to about 1, up to about 2, up to about 5, up to about 10, or up to about 15 conservative substitutions, and specifically binds to the GFRAL polypeptide. The term "conservative variation" also includes the use of a substituted amino acid in place of an unsubstituted parent amino acid as long as the antibody specifically binds to GFRAL. Non-conservative substitutions are those that decrease activity or GFRAL binding.

The term "isolated" biological components (e.g., nucleic acids, proteins (including antibodies) or organelles) have been substantially isolated or purified from other biological components (i.e., other chromosomal and additional chromosomal DNA and RNA, proteins and organelles) in the environment in which the components naturally occur (e.g., cells). Nucleic acids and proteins that have been "isolated" include those purified by standard purification methods. The term also includes nucleic acids and proteins prepared by recombinant expression in host cells as well as chemically synthesized nucleic acids.

The term "derived sequence" refers to a sequence that has at least 80% (preferably 85%, 90%, 95%, 98% or 99%) sequence identity to a sequence of interest and still has the same or similar function as the sequence of interest.

As used herein, the term "pharmaceutical composition" refers to a combination of at least one drug and, optionally, a pharmaceutically acceptable carrier or adjuvant that are combined together to achieve a particular purpose. In certain embodiments, the pharmaceutical composition includes a combination that is temporally and/or spatially separated, so long as it is capable of acting together to achieve the purpose of the present application. For example, the ingredients contained in the pharmaceutical composition (e.g., antibodies, nucleic acid molecules, nucleic acid molecule combinations and/or conjugates according to the present application) can be administered to a subject together or separately. When the ingredients contained in the pharmaceutical composition are separately administered to a subject, the ingredients can be administered to the subject simultaneously or sequentially. Preferably, the pharmaceutically acceptable carrier is water, a buffered aqueous solution, an isotonic salt solution such as PBS (phosphate buffer), glucose, mannitol, dextrose, lactose, starch, magnesium stearate, cellulose, magnesium carbonate, 0.3% glycerol, hyaluronic acid, ethanol, or polyalkylene glycols such as polypropylene glycol, or triglycerides. The type of a pharmaceutically acceptable carrier depends, inter alia, on whether the composition according to the present application is formulated for oral, nasal, intradermal, subcutaneous, intramuscular or intravenous administration. The compositions according to the present application can comprise wetting agents, emulsifying agents or buffer substances as additives. The pharmaceutical compositions or pharmaceutical formulations according to the present application can be administered by any suitable route, for example orally, nasally, intradermally, subcutaneously, intramuscularly or intravenously.

As used herein, "a therapeutically effective amount" or "an effective amount" refers to a dose sufficient to show benefit to a subject being administered. The actual administration dose, rate and course will depend on the condition and disease severity of the subject to be treated. The therapeutic prescription (e.g., prescribed dose) is ultimately the responsibility of and dependent on the general practitioner or other physicians, and generally takes into account the disease being treated, the condition of individual patient, the delivery site, the administration method, and other factors known to a physician.

As used herein, the term "fusion protein", in the general context, is a protein consisting of at least two domains with individual domains not associated with to each other in a natural state, and encoded by separate genes with individual genes linked to each other and transcribed and translated in an entirety, resulting in a single protein. In the technical context of the present application, a "fusion protein" comprising an antibody or antigen-binding portion refers to a product obtained by fusion of the antibody or antigen-binding portion to another biologically active protein using genetic engineering techniques, and such antibody fusion proteins have both the antigen-binding capacity of the antibody and the unique biological characteristics of the biologically active protein fused to the antibody.

The term "identity/homology/identity" in the context of an amino acid or nucleic acid sequence is defined as the percentage of identical residues in an amino acid or nucleotide sequence variant that, after alignment and introduction of gaps, achieves maximum percent homology, if desired. Methods and computer programs for alignment are well known in the art.

Based on blocking or inhibition of the GDF15/GFRAL/RET signaling pathway as a novel strategy for the treatment of cancer cachexia, the present inventors have sought to find effective GFRAL-targeting antagonist antibodies that are expected to block the formation of the GDF15/GFRAL/RET protein complex or the GDF15 signaling, thereby treating, preventing or alleviating the occurrence, frequency or severity of GFRAL-mediated or GFRAL-RET-GDF15 signaling pathway-mediated diseases or abnormalities, particularly uncontroled weight loss in cachexia, or cancer induced by or associated with GDF15 protein with reduced susceptibility to chemotherapeutic agents, such as the anti-tumor antibody trastuzumab, thereby breaking the deadlock of no approved drug for cachexia, thereby establishing the various invention in the present application.

In a first aspect, the present application provides an anti-GFRAL antibody or an antigen-binding portion thereof comprising a heavy chain variable region and a light chain variable region, wherein the heavy chain variable region comprises a heavy chain CDR1 (HCDR1), a heavy chain CDR2 (HCDR2), and a heavy chain CDR3 (HCDR3), and wherein the light chain variable region comprises a light chain CDR1 (LCDR1), a light chain CDR2 (LCDR2), and a light chain CDR3 (LCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO. 116 or has at least 80%, 85%, 90% or 95% identity to the sequence as set forth in SEQ ID NO.76 or SEQ ID NO. 116, the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99, the sequence of the light chain CDR1 is the sequence as set forth in SEQ ID NO.79 or SEQ ID NO. 126 or SEQ ID NO. 128 or has at least 80%, 85%, 90% or 95% identity to the sequence as set forth in SEQ ID NO.79 or SEQ ID NO. 126 or SEQ ID NO. 128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO. 123 or has at least 80%, 85%, 90% or 95% identity to the sequence as set forth in SEQ ID NO.50 or SEQ ID NO. 123; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, the sequence of the heavy chain CDR3 is the sequence as set forth in SEQ ID NO.70 or has at least 80%, 85%, 90% or 95% identity to the sequence as set forth in SEQ ID NO.70, the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, the sequence of the heavy chain CDR3 is the sequence as set forth in SEQ ID NO.3 or has at least 80%, 85%, 90% or 95% identity to the sequence as set forth in SEQ ID NO.3, the sequence of the light chain CDR1 is the sequence as set forth in SEQ ID NO.5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO. 10 or has at least 80%, 85%, 90% or 95% identity to the sequence as set forth in SEQ ID NO. 10, the sequence of the heavy chain CDR3 is the sequence as set forth in SEQ ID NO.11 or has at least 80%, 85%, 90% or 95% identity to the sequence as set forth in SEQ ID NO. 11, the sequence of the light chain CDR1 is the sequence as set forth in SEQ ID NO. 13 or has at least 80%, 85%, 90% or 95% identity to the sequence as set forth in SEQ ID NO.13 the sequence of the light chain CDR2 is the sequence as set forth in SEQ ID NO.14 or has at least 80%, 85%, 90% or 95% identity to the sequence as set forth in SEQ ID NO.14, and the sequence of the light chain CDR3 is the sequence as set forth in SEQ ID NO.15 or has at least 80%, 85%, 90% or 95% identity to the sequence as set forth in SEQ ID NO.15; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, the sequence of the heavy chain CDR3 is the sequence as set forth in SEQ ID NO.19 or has at least 80%, 85%, 90% or 95% identity to the sequence as set forth in SEQ ID NO.19, the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, the sequence of the heavy chain CDR3 is the sequence as set forth in SEQ ID NO.26 or has at least 80%, 85%, 90% or 95% identity to the sequence as set forth in SEQ ID NO.26, the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, the sequence of the heavy chain CDR3 is the sequence as set forth in SEQ ID NO.33 or has at least 80%, 85%, 90% or 95% identity to the sequence as set forth in SEQ ID NO.33, the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, the sequence of the heavy chain CDR3 is the sequence as set forth in SEQ ID NO.41 or has at least 80%, 85%, 90% or 95% identity to the sequence as set forth in SEQ ID NO.41, the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, the sequence of the heavy chain CDR3 is the sequence as set forth in SEQ ID NO.47 or has at least 80%, 85%, 90% or 95% identity to the sequence as set forth in SEQ ID NO.47, the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, the sequence of the heavy chain CDR3 is the sequence as set forth in SEQ ID NO.54 or has at least 80%, 85%, 90% or 95% identity to the sequence as set forth in SEQ ID NO.54, the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, the sequence of the heavy chain CDR3 is the sequence as set forth in SEQ ID NO.62 or has at least 80%, 85%, 90% or 95% identity to the sequence as set forth in SEQ ID NO.62, the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, the sequence of the heavy chain CDR3 is the sequence as set forth in SEQ ID NO.84 or has at least 80%, 85%, 90% or 95% identity to the sequence as set forth in SEQ ID NO.84, the sequence of the light chain CDR1 is the sequence as set forth in SEQ ID NO.86 or has at least 80%, 85%, 90% or 95% identity to the sequence as set forth in SEQ ID NO.86 the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the heavy chain CDR1 to 3 and the light chain CDR1 to 3 are defined according to the IMGT definition.

In some embodiments,
(1) the sequence of the heavy chain CDR1 is the sequence as set forth in SEQ ID NO.1, the sequence of the heavy chain CDR2 is the sequence as set forth in SEQ ID NO.2, the sequence of heavy chain CDR3 is the sequence as set forth in SEQ ID NO.3, the sequence of light chain CDR1 is the sequence as set forth in SEQ ID NO.5, the sequence of the light chain CDR2 is the sequence as set forth in SEQ ID NO.6, and the sequence of light chain CDR3 is the sequence as set forth in SEQ ID NO.7; or
(2) the sequence of the heavy chain CDR1 is the sequence as set forth in SEQ ID NO.9, the sequence of the heavy chain CDR2 is the sequence as set forth in SEQ ID NO.10, the sequence of the heavy chain CDR3 is the sequence as set forth in SEQ ID NO. 11, the sequence of the light chain CDR1 is the sequence as set forth in SEQ ID NO. 13, the sequence of the light chain CDR2 is the sequence as set forth in SEQ ID NO. 14, and the light chain CDR3 is the sequence as set forth in SEQ ID NO. 15; or
(3) the sequence of the heavy chain CDR1 is the sequence as set forth in SEQ ID NO. 17, the sequence of the heavy chain CDR2 is the sequence as set forth in SEQ ID NO. 18, the sequence of the heavy chain CDR3 is the sequence as set forth in SEQ ID NO. 19, light chain CDR1 is the sequence as set forth in SEQ ID NO.21. The sequence of the light chain CDR2 is the sequence as set forth in SEQ ID NO.6, and the sequence of light chain CDR3 is the sequence as set forth in SEQ ID NO.22; or
(4) the sequence of the heavy chain CDR1 is the sequence as set forth in SEQ ID NO.24, the sequence of the heavy chain CDR2 is the sequence as set forth in SEQ ID NO.25, the sequence of the heavy chain CDR3 is the sequence as set forth in SEQ ID NO.26, the sequence of the light chain CDR1 is the sequence as set forth in SEQ ID NO.28, the sequence of the light chain CDR2 is the sequence as set forth in SEQ ID NO.6, and the sequence of light chain CDR3 is the sequence as set forth in SEQ ID NO.29; or
(5) the sequence of the heavy chain CDR1 is the sequence as set forth in SEQ ID NO.31, the sequence of the heavy chain CDR2 is the sequence as set forth in SEQ ID NO.32, the sequence of the heavy chain CDR3 is the sequence as set forth in SEQ ID NO.33, the sequence of light chain CDR1 is the sequence as set forth in SEQ ID NO.35, and the sequence of the light chain CDR2 is the sequence as set forth in SEQ ID NO.36, light chain CDR3 SEQ ID NO.37; or
(6) the sequence of the heavy chain CDR1 is the sequence as set forth in SEQ ID NO.39, the sequence of the heavy chain CDR2 is the sequence as set forth in SEQ ID NO.40, the sequence of the heavy chain CDR3 is the sequence as set forth in SEQ ID NO.41, light chain CDR1 is the sequence as set forth in SEQ ID NO.43, the sequence of the light chain CDR2 is the sequence as set forth in SEQ ID NO.6, and the sequence of light chain CDR3 is the sequence as set forth in SEQ ID NO.22; or
(7) the sequence of the heavy chain CDR1 is the sequence as set forth in SEQ ID NO.45, the sequence of the heavy chain CDR2 is the sequence as set forth in SEQ ID NO.46, the sequence of the heavy chain CDR3 is the sequence as set forth in SEQ ID NO.47, the sequence of light chain CDR1 is the sequence as set forth in SEQ ID NO.49, the sequence of the light chain CDR2 is the sequence as set forth in SEQ ID NO.6, and the sequence of light chain CDR3 is the sequence as set forth in SEQ ID NO.50; or
(8) the sequence of the heavy chain CDR1 is the sequence as set forth in SEQ ID NO.31, the sequence of the heavy chain CDR2 is the sequence as set forth in SEQ ID NO.53, the sequence of the heavy chain CDR3 is the sequence as set forth in SEQ ID NO. 54, the sequence of the light chain CDR1 is the sequence as set forth in SEQ ID NO.56, the sequence of the light chain CDR2 is the sequence as set forth in SEQ ID NO.57, and the sequence of light chain CDR3 is the sequence as set forth in SEQ ID NO.58; or
(9) the sequence of the heavy chain CDR1 is the sequence as set forth in SEQ ID NO.60, the sequence of the heavy chain CDR2 is the sequence as set forth in SEQ ID NO.61, the sequence of the heavy chain CDR3 is the sequence as set forth in SEQ ID NO.62, the sequence of light chain CDR1 is the sequence as set forth in SEQ ID NO.64, the sequence of light chain CDR2 is the sequence as set forth in SEQ ID NO.65, and the sequence of the light chain CDR3 is the sequence as set forth in SEQ ID NO.66; or
(10) the sequence of the heavy chain CDR1 is the sequence as set forth in SEQ ID NO.68, the sequence of the heavy chain CDR2 is the sequence as set forth in SEQ ID NO.69 or SEQ ID NO.91, the sequence of the heavy chain CDR3 is the sequence as set forth in SEQ ID NO.70, the sequence of the light chain CDR1 is the sequence as set forth in SEQ ID NO.72 or SEQ ID NO.95, light chain CDR2 is the sequence as set forth in SEQ ID NO.65, and the sequence of the light chain CDR3 is the sequence as set forth in SEQ ID NO.73; or
(11) the sequence of the heavy chain CDR1 is the sequence as set forth in SEQ ID NO.75, the sequence of the heavy chain CDR2 is the sequence as set forth in SEQ ID NO.76 or SEQ ID NO.116, the sequence of the heavy chain CDR3 is the sequence as set forth in SEQ ID NO.77 or SEQ ID NO.99, the sequence of the light chain CDR1 is the sequence as set forth in SEQ ID NO.79 or SEQ ID NO.126 or SEQ ID NO.128, the sequence of the light chain CDR2 is the sequence as set forth in SEQ ID NO.80, and the sequence of the light chain CDR3 is the sequence as set forth in SEQ ID NO.50 or SEQ ID NO.123; or
(12) the sequence of the heavy chain CDR1 is the sequence as set forth in SEQ ID NO.82, the sequence of the heavy chain CDR2 is the sequence as set forth in SEQ ID NO.83, the sequence of the heavy chain CDR3 is the sequence as set forth in SEQ ID NO. 84, the sequence of the light chain CDR1 is the sequence as set forth in SEQ ID NO.86, the sequence of the light chain CDR2 is the sequence as set forth in SEQ ID NO.87, light chain CDR3 SEQ ID NO.88;
wherein the amino acid sequences of the heavy chain CDR1 to 3 and the light chain CDR1 to 3 are defined according to the IMGT definition.

In some embodiments, the heavy chain variable region has the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121, or has an amino acid sequence having at least 80%, at least 90%, at least 95% or at least 99% identity to the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121.

In some embodiments, the light chain variable region has the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134 or 135, or has an amino acid sequence having at least 80%, at least 90%, at least 95% or at least 99% identity to the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134, or 135.

In some embodiments:
(1) the sequence of the heavy chain variable region is the sequence as set forth in SEQ ID NO.4, and the sequence of the light chain variable region is the sequence as set forth in SEQ ID NO.8;
(2) the sequence of the heavy chain variable region is the sequence as set forth in SEQ ID NO.12, and the sequence of the light chain variable region is the sequence as set forth in SEQ ID NO.16;
(3) the sequence of the heavy chain variable region is the sequence as set forth in SEQ ID NO.20, and the sequence of the light chain variable region is the sequence as set forth in SEQ ID NO.23;
(4) the sequence of the heavy chain variable region is the sequence as set forth in SEQ ID NO.27, and the sequence of the light chain variable region is the sequence as set forth in SEQ ID NO.30;
(5) the sequence of the heavy chain variable region is the sequence as set forth in SEQ ID NO.34, and the sequence of the light chain variable region is the sequence as set forth in SEQ ID NO.38;
(6) the sequence of the heavy chain variable region is the sequence as set forth in SEQ ID NO.42, and the sequence of the light chain variable region is the sequence as set forth in SEQ ID NO.44;
(7) the sequence of the heavy chain variable region is the sequence as set forth in SEQ ID NO.48, and the sequence of the light chain variable region is the sequence as set forth in SEQ ID NO.51;
(8) the sequence of the heavy chain variable region is the sequence as set forth in SEQ ID NO.52, and the sequence of the light chain variable region is the sequence as set forth in SEQ ID NO.51;
(9) the sequence of the heavy chain variable region is the sequence as set forth in SEQ ID NO.55, and the sequence of the light chain variable region is the sequence as set forth in SEQ ID NO.59;
(10) the sequence of the heavy chain variable region is the sequence as set forth in SEQ ID NO.63, and the sequence of the light chain variable region is the sequence as set forth in SEQ ID NO.67;
(11) the sequence of the heavy chain variable region is the sequence as set forth in SEQ ID NO.71, and the sequence of the light chain variable region is the sequence as set forth in SEQ ID NO.74;
(12) the sequence of the heavy chain variable region is the sequence as set forth in SEQ ID NO.78, and the sequence of the light chain variable region is the sequence as set forth in SEQ ID NO.81;
(13) the sequence of the heavy chain variable region is the sequence as set forth in SEQ ID NO.85, and the sequence of the light chain variable region is the sequence as set forth in SEQ ID NO.89;
(14) the sequence of the heavy chain variable region is the sequence as set forth in SEQ ID NO.98, and the sequence of the light chain variable region is the sequence as set forth in SEQ ID NO. 122;
(15) the sequence of the heavy chain variable region is the sequence as set forth in SEQ ID NO.98, and the sequence of the light chain variable region is the sequence as set forth in SEQ ID NO. 124;
(16) the sequence of the heavy chain variable region is the sequence as set forth in SEQ ID NO. 100, and the sequence of the light chain variable region is the sequence as set forth in SEQ ID NO. 124;
(17) the sequence of the heavy chain variable region is the sequence as set forth in SEQ ID NO. 101, and the sequence of the light chain variable region is the sequence as set forth in SEQ ID NO. 124;
(18) the sequence of the heavy chain variable region is the sequence as set forth in SEQ ID NO. 102, and the sequence of the light chain variable region is the sequence as set forth in SEQ ID NO. 124;
(19) the sequence of the heavy chain variable region is the sequence as set forth in SEQ ID NO. 103, and the sequence of the light chain variable region is the sequence as set forth in SEQ ID NO. 124;
(20) the sequence of the heavy chain variable region is the sequence as set forth in SEQ ID NO. 104, and the sequence of the light chain variable region is the sequence as set forth in SEQ ID NO. 124;
(21) the sequence of the heavy chain variable region is the sequence as set forth in SEQ ID NO. 105, and the sequence of the light chain variable region is the sequence as set forth in SEQ ID NO. 124;
(22) the sequence of the heavy chain variable region is the sequence as set forth in SEQ ID NO. 112, and the sequence of the light chain variable region is the sequence as set forth in SEQ ID NO. 124;
(23) the sequence of the heavy chain variable region is the sequence as set forth in SEQ ID NO. 113, and the sequence of the light chain variable region is the sequence as set forth in SEQ ID NO. 124;
(24) the sequence of the heavy chain variable region is the sequence as set forth in SEQ ID NO. 105, and the sequence of the light chain variable region is the sequence as set forth in SEQ ID NO. 125;
(25) the sequence of the heavy chain variable region is the sequence as set forth in SEQ ID NO. 105, and the sequence of the light chain variable region is the sequence as set forth in SEQ ID NO. 127;
(26) the sequence of the heavy chain variable region is the sequence as set forth in SEQ ID NO.100, and the sequence of the light chain variable region is the sequence as set forth in SEQ ID NO.129;
(27) the sequence of the heavy chain variable region is the sequence as set forth in SEQ ID NO.101, and the sequence of the light chain variable region is the sequence as set forth in SEQ ID NO.129;
(28) the sequence of the heavy chain variable region is the sequence as set forth in SEQ ID NO.105, and the sequence of the light chain variable region is the sequence as set forth in SEQ ID NO.129;
(29) the sequence of the heavy chain variable region is the sequence as set forth in SEQ ID NO.106, and the sequence of the light chain variable region is the sequence as set forth in SEQ ID NO.129;
(30) the sequence of the heavy chain variable region is the sequence as set forth in SEQ ID NO.107, and the sequence of the light chain variable region is the sequence as set forth in SEQ ID NO.129;
(31) the sequence of the heavy chain variable region is the sequence as set forth in SEQ ID NO.108, and the sequence of the light chain variable region is the sequence as set forth in SEQ ID NO.129;
(32) the sequence of the heavy chain variable region is the sequence as set forth in SEQ ID NO.109, and the sequence of the light chain variable region is the sequence as set forth in SEQ ID NO.129;
(33) the sequence of the heavy chain variable region is the sequence as set forth in SEQ ID NO.110, and the sequence of the light chain variable region is the sequence as set forth in SEQ ID NO.129;
(34) the sequence of the heavy chain variable region is the sequence as set forth in SEQ ID NO.111, and the sequence of the light chain variable region is the sequence as set forth in SEQ ID NO.129;
(35) the sequence of the heavy chain variable region is the sequence as set forth in SEQ ID NO.105, and the sequence of the light chain variable region is the sequence as set forth in SEQ ID NO.130;
(36) the sequence of the heavy chain variable region is the sequence as set forth in SEQ ID NO.105, and the sequence of the light chain variable region is the sequence as set forth in SEQ ID NO.131;
(37) the sequence of the heavy chain variable region is the sequence as set forth in SEQ ID NO.100, and the sequence of the light chain variable region is the sequence as set forth in SEQ ID NO.132;
(38) the sequence of the heavy chain variable region is the sequence as set forth in SEQ ID NO.114, and the sequence of the light chain variable region is the sequence as set forth in SEQ ID NO.132;
(39) the sequence of the heavy chain variable region is the sequence as set forth in SEQ ID NO.115, and the sequence of the light chain variable region is the sequence as set forth in SEQ ID NO.133;
(40) the sequence of the heavy chain variable region is the sequence as set forth in SEQ ID NO.117, and the sequence of the light chain variable region is the sequence as set forth in SEQ ID NO.133;
(41) the sequence of the heavy chain variable region is the sequence as set forth in SEQ ID NO.118, and the sequence of the light chain variable region is the sequence as set forth in SEQ ID NO.133;
(42) the sequence of the heavy chain variable region is the sequence as set forth in SEQ ID NO.119, and the sequence of the light chain variable region is the sequence as set forth in SEQ ID NO.133;
(43) the sequence of the heavy chain variable region is the sequence as set forth in SEQ ID NO.115, and the sequence of the light chain variable region is the sequence as set forth in SEQ ID NO. 134;
(44) the sequence of the heavy chain variable region is the sequence as set forth in SEQ ID NO. 117, and the sequence of the light chain variable region is the sequence as set forth in SEQ ID NO. 134;
(45) the sequence of the heavy chain variable region is the sequence as set forth in SEQ ID NO. 118, and the sequence of the light chain variable region is the sequence as set forth in SEQ ID NO. 134;
(46) the sequence of the heavy chain variable region is the sequence as set forth in SEQ ID NO. 119, and the sequence of the light chain variable region is the sequence as set forth in SEQ ID NO. 134;
(47) the sequence of the heavy chain variable region is the sequence as set forth in SEQ ID NO. 119, and the sequence of the light chain variable region is the sequence as set forth in SEQ ID NO.135;
(48) the sequence of the heavy chain variable region is the sequence as set forth in SEQ ID NO. 120, and the sequence of the light chain variable region is the sequence as set forth in SEQ ID NO.135;
(49) the sequence of the heavy chain variable region is the sequence as set forth in SEQ ID NO.121, and the sequence of the light chain variable region is the sequence as set forth in SEQ ID NO.135;
(50) the sequence of the heavy chain variable region is the sequence as set forth in SEQ ID NO.90, and the sequence of the light chain variable region is the sequence as set forth in SEQ ID NO.94;
(51) the sequence of the heavy chain variable region is the sequence as set forth in SEQ ID NO.92, and the sequence of the light chain variable region is the sequence as set forth in SEQ ID NO.94;
(52) the sequence of the heavy chain variable region is the sequence as set forth in SEQ ID NO.90, and the sequence of the light chain variable region is the sequence as set forth in SEQ ID NO.96;
(53) the sequence of the heavy chain variable region is the sequence as set forth in SEQ ID NO.92, and the sequence of the light chain variable region is the sequence as set forth in SEQ ID NO.96;
(54) the sequence of the heavy chain variable region is the sequence as set forth in SEQ ID NO.90, and the sequence of the light chain variable region is the sequence as set forth in SEQ ID NO.97; or
(55) the sequence of the heavy chain variable region is the sequence as set forth in SEQ ID NO.93, and the sequence of the light chain variable region is the sequence as set forth in SEQ ID NO.97.

In a specific embodiment, the sequence of the heavy chain CDR1 is the sequence as set forth in SEQ ID NO.75, the sequence of the heavy chain CDR2 is the sequence as set forth in SEQ ID NO.76, the sequence of the heavy chain CDR3 is the sequence as set forth in SEQ ID NO.99, the sequence of the light chain CDR1 is the sequence as set forth in SEQ ID NO. 128, the sequence of the light chain CDR2 is the sequence as set forth in SEQ ID NO.80, and the sequence of the light chain CDR3 is the sequence as set forth in SEQ ID NO. 123. In a more specific embodiment, the sequence of the heavy chain variable region is the sequence as set forth in SEQ ID NO.105, and the sequence of the light chain variable region is the sequence as set forth in SEQ ID NO.127.

In a second aspect, the present application provides an anti-GFRAL antibody or an antigen-binding portion thereof comprising a heavy chain variable region and a light chain variable region, wherein
the heavy chain variable region has the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121, or has an amino acid sequence having at least 80%, at least 90%, at least 95% or at least 99% identity to the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121; and/or
the light chain variable region has the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134 or 135, or has an amino acid sequence having at least 80%, at least 90%, at least 95% or at least 99% identity to the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134, or 135.

In a third aspect, the present application provides an anti-GFRAL antibody or an antigen-binding portion thereof comprising a heavy chain variable region, wherein the heavy chain variable region comprises a heavy chain CDR1 (HCDR1), a heavy chain CDR2 (HCDR2), and a heavy chain CDR3 (HCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO.116 or has at least 80%, 85%, 90% or 95% identity to the sequence as set forth in SEQ ID NO.76 or SEQ ID NO.116, and the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, and the sequence of the heavy chain CDR3 is the sequence as set forth in SEQ ID NO.70 or has at least 80%, 85%, 90% or 95% identity to the sequence as set forth in SEQ ID NO.70; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, and the sequence of the heavy chain CDR3 is the sequence as set forth in SEQ ID NO.3 or has at least 80%, 85%, 90% or 95% identity to the sequence as set forth in SEQ ID NO.3; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO.10 or has at least 80%, 85%, 90% or 95% identity to the sequence as set forth in SEQ ID NO.10, and the sequence of the heavy chain CDR3 is the sequence as set forth in SEQ ID NO.11 or has at least 80%, 85%, 90% or 95% identity to the sequence as set forth in SEQ ID NO.11; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, and the sequence of the heavy chain CDR3 is the sequence as set forth in SEQ ID NO.19 or has at least 80%, 85%, 90% or 95% identity to the sequence as set forth in SEQ ID NO.19; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, and the sequence of the heavy chain CDR3 is the sequence as set forth in SEQ ID NO.26 or has at least 80%, 85%, 90% or 95% identity to the sequence as set forth in SEQ ID NO.26; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, and the sequence of the heavy chain CDR3 is the sequence as set forth in SEQ ID NO.33 or has at least 80%, 85%, 90% or 95% identity to the sequence as set forth in SEQ ID NO.33; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, and the sequence of the heavy chain CDR3 is the sequence as set forth in SEQ ID NO.41 or has at least 80%, 85%, 90% or 95% identity to the sequence as set forth in SEQ ID NO.41; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, and the sequence of the heavy chain CDR3 is the sequence as set forth in SEQ ID NO.47 or has at least 80%, 85%, 90% or 95% identity to the sequence as set forth in SEQ ID NO.47; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, and the sequence of the heavy chain CDR3 is the sequence as set forth in SEQ ID NO.54 or has at least 80%, 85%, 90% or 95% identity to the sequence as set forth in SEQ ID NO.54; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, and the sequence of the heavy chain CDR3 is the sequence as set forth in SEQ ID NO.62 or has at least 80%, 85%, 90% or 95% identity to the sequence as set forth in SEQ ID NO.62; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, and the sequence of the heavy chain CDR3 is the sequence as set forth in SEQ ID NO.84 or has at least 80%, 85%, 90% or 95% identity to the sequence as set forth in SEQ ID NO.84;
wherein the amino acid sequence of the heavy chain CDR1-3 is defined according to the IMGT definition.

In some embodiments,
(1) the sequence of the heavy chain CDR1 is the sequence as set forth in SEQ ID NO.1, the sequence of the heavy chain CDR2 is the sequence as set forth in SEQ ID NO.2, the sequence of heavy chain CDR3 is the sequence as set forth in SEQ ID NO.3; or
(2) the sequence of the heavy chain CDR1 is the sequence as set forth in SEQ ID NO.9, the sequence of the heavy chain CDR2 is the sequence as set forth in SEQ ID NO.10, and the sequence of the heavy chain CDR3 is the sequence as set forth in SEQ ID NO.11; or
(3) the sequence of the heavy chain CDR1 is the sequence as set forth in SEQ ID NO.17, the sequence of the heavy chain CDR2 is the sequence as set forth in SEQ ID NO.18, and the sequence of the heavy chain CDR3 is the sequence as set forth in SEQ ID NO.19; or
(4) the sequence of the heavy chain CDR1 is the sequence as set forth in SEQ ID NO.24, the sequence of the heavy chain CDR2 is the sequence as set forth in SEQ ID NO.25, and the sequence of the heavy chain CDR3 is the sequence as set forth in SEQ ID NO.26; or
(5) the sequence of the heavy chain CDR1 is the sequence as set forth in SEQ ID NO.31, the sequence of the heavy chain CDR2 is the sequence as set forth in SEQ ID NO.32, and the sequence of the heavy chain CDR3 is the sequence as set forth in SEQ ID NO.33; or
(6) the sequence of the heavy chain CDR1 is the sequence as set forth in SEQ ID NO.39, the sequence of the heavy chain CDR2 is the sequence as set forth in SEQ ID NO.40, and the sequence of the heavy chain CDR3 is the sequence as set forth in SEQ ID NO.41; or
(7) the sequence of the heavy chain CDR1 is the sequence as set forth in SEQ ID NO.45, the sequence of the heavy chain CDR2 is the sequence as set forth in SEQ ID NO.46, and the sequence of the heavy chain CDR3 is the sequence as set forth in SEQ ID NO.47; or
(8) the sequence of the heavy chain CDR1 is the sequence as set forth in SEQ ID NO.31, the sequence of the heavy chain CDR2 is the sequence as set forth in SEQ ID NO.53, and the sequence of the heavy chain CDR3 is the sequence as set forth in SEQ ID NO. 54; or
(9) the sequence of the heavy chain CDR1 is the sequence as set forth in SEQ ID NO.60, the sequence of the heavy chain CDR2 is the sequence as set forth in SEQ ID NO.61, and the sequence of the heavy chain CDR3 is the sequence as set forth in SEQ ID NO.62; or
(10) the sequence of the heavy chain CDR1 is the sequence as set forth in SEQ ID NO.68, the sequence of the heavy chain CDR2 is the sequence as set forth in SEQ ID NO.69 or SEQ ID NO.91, and the sequence of the heavy chain CDR3 is the sequence as set forth in SEQ ID NO.70; or
(11) the sequence of the heavy chain CDR1 is the sequence as set forth in SEQ ID NO.75, the sequence of the heavy chain CDR2 is the sequence as set forth in SEQ ID NO.76 or SEQ ID NO. 116, and the sequence of the heavy chain CDR3 is the sequence as set forth in SEQ ID NO.77 or SEQ ID NO.99; or
(12) the sequence of the heavy chain CDR1 is the sequence as set forth in SEQ ID NO.82, the sequence of the heavy chain CDR2 is the sequence as set forth in SEQ ID NO.83, and the sequence of the heavy chain CDR3 is the sequence as set forth in SEQ ID NO. 84;
wherein the amino acid sequences of the heavy chain CDR1 to 3 are defined according to the IMGT definition.

One example of the antibodies according to the third aspect is a single domain antibody, also known as a variable domain of heavy chain of heavy-chain antibody (VHH), which consists of only one heavy chain variable domain, also known as a nano-antibody. A VHH antibody is an antibody with a light chain naturally deleted initially found in the peripheral blood of alpaca, and comprises only one heavy chain variable region (VHH) and two conventional CH2 and CH3 regions. A cloned and expressed VHH structure has structural stability and binding activity to an antigen comparable to that of the original heavy chain antibody, and is the minimum unit capable of binding to a target antigen known so far.

In a fourth aspect, the present application provides an anti-GFRAL antibody or an antigen-binding portion thereof comprising a light chain variable region, wherein the light chain variable region comprises a light chain CDR1 (HCDR1), a light chain CDR2 (HCDR2), and a light chain CDR3 (HCDR3), wherein:
(1) the sequence of the light chain CDR1 is the sequence as set forth in SEQ ID NO.79 or SEQ ID NO. 126 or SEQ ID NO. 128 or has at least 80%, 85%, 90% or 95% identity to the sequence as set forth in SEQ ID NO.79 or SEQ ID NO. 126 or SEQ ID NO. 128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO. 123 or has at least 80%, 85%, 90% or 95% identity to the sequence as set forth in SEQ ID NO.50 or SEQ ID NO. 123; or
(2) the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the light chain CDR1 is the sequence as set forth in SEQ ID NO.5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the light chain CDR1 is the sequence as set forth in SEQ ID NO. 13 or has at least 80%, 85%, 90% or 95% identity to the sequence as set forth in SEQ ID NO. 13 the sequence of the light chain CDR2 is the sequence as set forth in SEQ ID NO. 14 or has at least 80%, 85%, 90% or 95% identity to the sequence as set forth in SEQ ID NO. 14, and the sequence of the light chain CDR3 is the sequence as set forth in SEQ ID NO. 15 or has at least 80%, 85%, 90% or 95% identity to the sequence as set forth in SEQ ID NO. 15; or
(5) the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the light chain CDR1 is the sequence as set forth in SEQ ID NO.86 or has at least 80%, 85%, 90% or 95% identity to the sequence as set forth in SEQ ID NO.86 the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the light chain CDR1 to 3 are defined according to the IMGT definition.

In some embodiments,
(1) the sequence of the light chain CDR1 is the sequence as set forth in SEQ ID NO.5, the sequence of the light chain CDR2 is the sequence as set forth in SEQ ID NO.6, and the sequence of light chain CDR3 is the sequence as set forth in SEQ ID NO.7; or
(2) the sequence of the light chain CDR1 is the sequence as set forth in SEQ ID NO. 13, the sequence of the light chain CDR2 is the sequence as set forth in SEQ ID NO. 14, the light chain CDR3 is the sequence as set forth in SEQ ID NO. 15; or
(3) the sequence of the light chain CDR1 is the sequence as set forth in SEQ ID NO.21. The sequence of the light chain CDR2 is the sequence as set forth in SEQ ID NO.6, and the sequence of light chain CDR3 is the sequence as set forth in SEQ ID NO.22; or
(4) the sequence of the light chain CDR1 is the sequence as set forth in SEQ ID NO.28, the sequence of the light chain CDR2 is the sequence as set forth in SEQ ID NO.6, and the sequence of light chain CDR3 is the sequence as set forth in SEQ ID NO.29; or
(5) the sequence of the light chain CDR1 is the sequence as set forth in SEQ ID NO.35, the sequence of the light chain CDR2 is the sequence as set forth in SEQ ID NO.36, light chain CDR3 SEQ ID NO.37; or
(6) the sequence of the light chain CDR1 is the sequence as set forth in SEQ ID NO.43, the sequence of the light chain CDR2 is the sequence as set forth in SEQ ID NO.6, and the sequence of light chain CDR3 is the sequence as set forth in SEQ ID NO.22; or
(7) the sequence of the light chain CDR1 is the sequence as set forth in SEQ ID NO.49, the sequence of the light chain CDR2 is the sequence as set forth in SEQ ID NO.6, and the sequence of light chain CDR3 is the sequence as set forth in SEQ ID NO.50; or
(8) the sequence of the light chain CDR1 is the sequence as set forth in SEQ ID NO.56, the sequence of the light chain CDR2 is the sequence as set forth in SEQ ID NO.57, and the sequence of light chain CDR3 is the sequence as set forth in SEQ ID NO.58; or
(9) the sequence of the light chain CDR1 is the sequence as set forth in SEQ ID NO.64, the sequence of light chain CDR2 is the sequence as set forth in SEQ ID NO.65, and the sequence of the light chain CDR3 is the sequence as set forth in SEQ ID NO.66; or
(10) the sequence of the light chain CDR1 is the sequence as set forth in SEQ ID NO.72 or SEQ ID NO.95, light chain CDR2 is the sequence as set forth in SEQ ID NO.65, and the sequence of the light chain CDR3 is the sequence as set forth in SEQ ID NO.73; or
(11) the sequence of the light chain CDR1 is the sequence as set forth in SEQ ID NO.79 or SEQ ID NO. 126 or SEQ ID NO. 128, the sequence of the light chain CDR2 is the sequence as set forth in SEQ ID NO.80, and the sequence of the light chain CDR3 is the sequence as set forth in SEQ ID NO.50 or SEQ ID NO. 123; or
(12) the sequence of the light chain CDR1 is the sequence as set forth in SEQ ID NO.86, the sequence of the light chain CDR2 is the sequence as set forth in SEQ ID NO.87, light chain CDR3 SEQ ID NO.88;
wherein the amino acid sequences of the light chain CDR1 to 3 are defined according to the IMGT definition.

In a fifth aspect, the present application provides an anti-GFRAL antibody or an antigen-binding portion thereof comprising a heavy chain variable region and a light chain variable region, wherein
(i) the heavy chain variable region comprises three heavy chain CDR regions, wherein the amino acid sequence of at least one of the heavy chain CDR regions is selected from the group consisting of SEQ ID NO.1, SEQ ID NO.9, SEQ ID NO. 17, SEQ ID NO.24, SEQ ID NO.31, SEQ ID NO.39, SEQ ID NO.45, SEQ ID NO.60, SEQ ID NO.68, SEQ ID NO.75, SEQ ID NO.82, SEQ ID NO.2, SEQ ID NO.10, SEQ ID NO.18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99, or has at least 80% (preferably 85%, 90%, 95%, 98% or 99%) sequence identity to an amino acid sequence selected from the group consisting of SEQ ID NO.1, SEQ ID NO.9, SEQ ID NO. 17, SEQ ID NO.24, SEQ ID NO.31, SEQ ID NO.39, SEQ ID NO.45, SEQ ID NO.60, SEQ ID NO.68, SEQ ID NO.75, SEQ ID NO.82, SEQ ID NO.2, SEQ ID NO.10, SEQ ID NO. 18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99; and/or
(ii) the light chain variable region comprises three light chain CDR regions, wherein the amino acid sequence of at least one of the light chain CDR regions is selected from the group consisting of SEQ ID NO.5, SEQ ID NO.13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, SEQ ID NO.128, SEQ ID NO.6, SEQ ID NO.14, SEQ ID NO.36, SEQ ID NO.57, SEQ ID NO.65, SEQ ID NO.80, SEQ ID NO.87, SEQ ID NO.7, SEQ ID NO. 15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO. 123, or has at least 80% (preferably 85%, 90%, 95%, 98% or 99%) sequence identity to an amino acid sequence selected from the group consisting of SEQ ID NO.5, SEQ ID NO. 13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, SEQ ID NO.128, SEQ ID NO.6, SEQ ID NO.14, SEQ ID NO.36, SEQ ID NO.57, SEQ ID NO.65, SEQ ID NO.80, SEQ ID NO.87, SEQ ID NO.7, SEQ ID NO. 15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO. 123;
wherein the amino acid sequences of the heavy chain CDR1 to 3 and the light chain CDR1 to 3 are defined according to the IMGT definition.

In some embodiments, the antibody comprises a heavy chain variable region and a light chain variable region, and the heavy chain variable region and the light chain variable region comprise CDR1, CDR2, CDR3, respectively, wherein:
the sequence of the heavy chain CDR1 is selected from the group consisting of SEQ ID NO.1, SEQ ID NO.9, SEQ ID NO.17, SEQ ID NO.24, SEQ ID NO.31, SEQ ID NO.39, SEQ ID NO.45, SEQ ID NO.60, SEQ ID NO.68, SEQ ID NO.75, and SEQ ID NO.82, or has at least 80% (preferably 85%, 90%, 95%, 98% or 99%) sequence identity to a sequence selected from the group consisting of SEQ ID NO.1, SEQ ID NO.9, SEQ ID NO.17, SEQ ID NO.24, SEQ ID NO.31, SEQ ID NO.39, SEQ ID NO.45, SEQ ID NO.60, SEQ ID NO.68, SEQ ID NO.75, and SEQ ID NO.82;
the sequence of the heavy chain CDR2 is selected from the group consisting of SEQ ID NO.2, SEQ ID NO.10, SEQ ID NO.18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.91, and SEQ ID NO. 116, or has at least 80% (preferably 85%, 90%, 95%, 98% or 99%) sequence identity to a sequence selected from the group consisting of SEQ ID NO.2, SEQ ID NO.10, SEQ ID NO. 18, SEQ ID NO.25, SEQ ID NO.32, SEQ ID NO.40, SEQ ID NO.46, SEQ ID NO.53, SEQ ID NO.61, SEQ ID NO.69, SEQ ID NO.76, SEQ ID NO.83, SEQ ID NO.91, and SEQ ID NO.116;
the sequence of the heavy chain CDR3 is selected from the group consisting of SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99, or has at least 80% (preferably 85%, 90%, 95%, 98% or 99%) sequence identity to a sequence selected from the group consisting of SEQ ID NO.3, SEQ ID NO.11, SEQ ID NO.19, SEQ ID NO.26, SEQ ID NO.33, SEQ ID NO.41, SEQ ID NO.47, SEQ ID NO.54, SEQ ID NO.62, SEQ ID NO.70, SEQ ID NO.77, SEQ ID NO.84, and SEQ ID NO.99; and/or
the sequence of the light chain CDR1 thereof is selected from the group consisting of SEQ ID NO.5, SEQ ID NO.13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, and SEQ ID NO.128, or has at least 80% (preferably 85%, 90%, 95%, 98% or 99%) sequence identity to a sequence selected from the group consisting of SEQ ID NO.5, SEQ ID NO.13, SEQ ID NO.21, SEQ ID NO.28, SEQ ID NO.35, SEQ ID NO.43, SEQ ID NO.49, SEQ ID NO.56, SEQ ID NO.64, SEQ ID NO.72, SEQ ID NO.79, SEQ ID NO.86, SEQ ID NO.95, SEQ ID NO.126, and SEQ ID NO.128;
the sequence of the light chain CDR2 is selected from the group consisting of SEQ ID NO.6, SEQ ID NO.14, SEQ ID NO.36, SEQ ID NO.57, SEQ ID NO.65, SEQ ID NO.80, and SEQ ID NO.87, or has at least 80% (preferably 85%, 90%, 95%, 98% or 99%) sequence identity to a sequence selected from the group consisting of SEQ ID NO.6, SEQ ID NO.14, SEQ ID NO.36, SEQ ID NO.57, SEQ ID NO.65, SEQ ID NO.80, and SEQ ID NO.87;
the sequence of the light chain CDR3 is selected from the group consisting of SEQ ID NO.7, SEQ ID NO.15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO.123, or has at least 80% (preferably 85%, 90%, 95%, 98% or 99%) sequence identity to a sequence selected from the group consisting of SEQ ID NO.7, SEQ ID NO.15, SEQ ID NO.22, SEQ ID NO.29, SEQ ID NO.37, SEQ ID NO.50, SEQ ID NO.58, SEQ ID NO.66, SEQ ID NO.73, SEQ ID NO.88, and SEQ ID NO.123.

In some embodiments, the antibody comprises a heavy chain variable region and a light chain variable region, wherein
(i) the heavy chain variable region comprises three heavy chain CDR regions, wherein:
   (1) the sequence of CDR1 is the sequence as set forth in SEQ ID NO.1, the sequence of CDR2 is the sequence as set forth in SEQ ID NO.2, and the sequence of CDR3 is the sequence as set forth in SEQ ID NO.3;
   (2) the sequence of CDR1 is the sequence as set forth in SEQ ID NO.9, the sequence of CDR2 is the sequence as set forth in SEQ ID NO.10, and the sequence of CDR3 is the sequence as set forth in SEQ ID NO.11;
   (3) the sequence of CDR1 is the sequence as set forth in SEQ ID NO.17, the sequence of CDR2 is the sequence as set forth in SEQ ID NO.18, and the sequence of CDR3 is the sequence as set forth in SEQ ID NO.19;
   (4) the sequence of CDR1 is the sequence as set forth in SEQ ID NO.24, the sequence of CDR2 is the sequence as set forth in SEQ ID NO.25, and the sequence of CDR3 is the sequence as set forth in SEQ ID NO.26;
   (5) the sequence of CDR1 is the sequence as set forth in SEQ ID NO.31, the sequence of CDR2 is the sequence as set forth in SEQ ID NO.32, and the sequence of CDR3 is the sequence as set forth in SEQ ID NO.33;
   (6) the sequence of CDR1 is the sequence as set forth in SEQ ID NO.39, the sequence of CDR2 is the sequence as set forth in SEQ ID NO.40, and the sequence of CDR3 is the sequence as set forth in SEQ ID NO.41;
   (7) the sequence of CDR1 is the sequence as set forth in SEQ ID NO.45, the sequence of CDR2 is the sequence as set forth in SEQ ID NO.46, and the sequence of CDR3 is the sequence as set forth in SEQ ID NO.47;
   (8) the sequence of CDR1 is the sequence as set forth in SEQ ID NO.31, the sequence of CDR2 is the sequence as set forth in SEQ ID NO.53, and the sequence of CDR3 is the sequence as set forth in SEQ ID NO.54;
   (9) the sequence of CDR1 is the sequence as set forth in SEQ ID NO.60, the sequence of CDR2 is the sequence as set forth in SEQ ID NO.61, and the sequence of CDR3 is the sequence as set forth in SEQ ID NO.62;
   (10) the sequence of CDR1 is the sequence as set forth in SEQ ID NO.68, the sequence of CDR2 is the sequence as set forth in SEQ ID NO.69, and the sequence of CDR3 is the sequence as set forth in SEQ ID NO.70;
   (11) the sequence of CDR1 is the sequence as set forth in SEQ ID NO.75, the sequence of CDR2 is the sequence as set forth in SEQ ID NO.76, and the sequence of CDR3 is the sequence as set forth in SEQ ID NO.77;
   (12) the sequence of CDR1 is the sequence as set forth in SEQ ID NO.82, the sequence of CDR2 is the sequence as set forth in SEQ ID NO.83, and the sequence of CDR3 is the sequence as set forth in SEQ ID NO.84;
(ii) the light chain variable region comprises three light chain CDR regions, wherein:
   (13) the sequence of CDR1 is the sequence as set forth in SEQ ID NO.5, the sequence of CDR2 is the sequence as set forth in SEQ ID NO.6, and the sequence of CDR3 is the sequence as set forth in SEQ ID NO.7;
   (14) the sequence of CDR1 is the sequence as set forth in SEQ ID NO.13, the sequence of CDR2 is the sequence as set forth in SEQ ID NO. 14, and the sequence of CDR3 is the sequence as set forth in SEQ ID NO.15;
   (15) the sequence of CDR1 is the sequence as set forth in SEQ ID NO.21, the sequence of CDR2 is the sequence as set forth in SEQ ID NO.6, and the sequence of CDR3 is the sequence as set forth in SEQ ID NO.22;
   (16) the sequence of CDR1 is the sequence as set forth in SEQ ID NO.28, the sequence of CDR2 is the sequence as set forth in SEQ ID NO.6, and the sequence of CDR3 is the sequence as set forth in SEQ ID NO.29;
   (17) the sequence of CDR1 is the sequence as set forth in SEQ ID NO.35, the sequence of CDR2 is the sequence as set forth in SEQ ID NO.36, and the sequence of CDR3 is the sequence as set forth in SEQ ID NO.37;
   (18) the sequence of CDR1 is the sequence as set forth in SEQ ID NO.43, the sequence of CDR2 is the sequence as set forth in SEQ ID NO.6, and the sequence of CDR3 is the sequence as set forth in SEQ ID NO.22;
   (19) the sequence of CDR1 is the sequence as set forth in SEQ ID NO.49, the sequence of CDR2 is the sequence as set forth in SEQ ID NO.6, and the sequence of CDR3 is the sequence as set forth in SEQ ID NO.50;
   (20) the sequence of CDR1 is the sequence as set forth in SEQ ID NO.56, the sequence of CDR2 is the sequence as set forth in SEQ ID NO.57, and the sequence of CDR3 is the sequence as set forth in SEQ ID NO.58;
   (21) the sequence of CDR1 is the sequence as set forth in SEQ ID NO.64, the sequence of CDR2 is the sequence as set forth in SEQ ID NO.65, and the sequence of CDR3 is the sequence as set forth in SEQ ID NO.66;
   (22) the sequence of CDR1 is the sequence as set forth in SEQ ID NO.72, the sequence of CDR2 is the sequence as set forth in SEQ ID NO.65, and the sequence of CDR3 is the sequence as set forth in SEQ ID NO.73;
   (23) the sequence of CDR1 is the sequence as set forth in SEQ ID NO.79, the sequence of CDR2 is the sequence as set forth in SEQ ID NO.80, and the sequence of CDR3 is the sequence as set forth in SEQ ID NO.50;
   (24) the sequence of CDR1 is the sequence as set forth in SEQ ID NO.86, the sequence of CDR2 is the sequence as set forth in SEQ ID NO.87, and the sequence of CDR3 is the sequence as set forth in SEQ ID NO.88.

In some embodiments of the first to fifth aspects, the anti-GFRAL antibody is an intact antibody, a single chain fragment variable (scFv), or a bispecific antibody. In some embodiments of the first to fifth aspects, the antigen-binding fragment of the anti-GFRAL antibody is Fab, Fab', Fv or F(ab')₂. In some embodiments of the first to fifth aspects, the anti-GFRAL antibody is a murine antibody, a chimeric antibody, a humanized antibody, or a fully human antibody. In some embodiments of the first to fifth aspects, the anti-GFRAL antibody is a monoclonal antibody. In some embodiments of the first to fifth aspects, the monoclonal antibody can be a murine antibody or can be a humanized antibody. In some embodiments of the first to fifth aspects, the anti-GFRAL antibody is a bispecific antibody. In some embodiments of the first to fifth aspects, the anti-GFRAL antibody is of IgG1, IgG2, or IgG4 isotype, i.e., comprising a heavy chain constant region of an IgG1 subtype, a IgG2 subtype, or an IgG4 subtype. In some embodiments, the anti-GFRAL antibody is of IgG1 isotype. In a particular embodiment, the anti-GFRAL antibody or antigen-binding portion thereof comprises a human IgG1 heavy chain constant region and a human kappa light chain constant region. In a particular embodiment, the anti-GFRAL antibody or antigen-binding portion thereof comprises a human IgG4 heavy chain constant region and a human kappa light chain constant region, and the human IgG4 heavy chain constant region includes a wild-type and a mutant type. In some embodiments of the first to fifth aspects, the anti-GFRAL antibody comprises a light chain constant region of kappa subtype or lamda subtype. In some embodiments of the first to fifth aspects, the antibody or antigen-binding portion thereof of the present application is isolated. In some embodiments of the first to fifth aspects, the antibody or antigen-binding portion thereof of the present application can be a chimeric antibody, or can be a humanized antibody, including a semi-humanized antibody, a partially humanized antibody, or a fully human antibody. In some embodiments of the first to fifth aspects, the antibody or antigen-binding portion thereof of the present application has ADCC activity. In some embodiments of the first to fifth aspects, the antibody or antigen-binding portion thereof of the present application has CDC activity.

In a sixth aspect, the present application provides an isolated nucleic acid molecule encoding the anti-GFRAL antibody or antigen-binding portion thereof of any one of the first to fifth aspects. In some embodiments, the present application provides a combination of isolated polynucleotides comprising a polynucleotide encoding a light chain of an antibody of the present application or an antigen-binding portion thereof and a polynucleotide encoding the heavy chain of the antibody of the present application or antigen-binding portion. In some embodiments, the polynucleotide is operably linked to a regulatory sequence that can be recognized by host cells transformed with a vector.

In a seventh aspect, the present application provides a vector (e.g., an expression vector) comprising the nucleic acid molecule or the combination of polynucleotides of the sixth aspect. In some embodiments, an expression vector of the present application comprises a nucleic acid molecule or a combination of polynucleotides as described herein operably linked to a regulatory sequence that allows expression of the polypeptide encoded by the polynucleotides in a host cell or a cell-free expression system. The selection of an expression vector depends on the selection of host cells, and can be selected so as to have the desired expression and regulatory characteristics in the selected host cells.

An "expression vector" is a vector comprising one or more expression regulatory sequences. An "expression regulatory sequence" is a DNA sequence that controls and regulates transcription and/or translation of another DNA sequence.

The nucleic acid in the vector can be operably linked to one or more expression regulatory sequences. As used herein, the term "operably linked" means incorporation into a genetic construct such that the expression regulatory sequence effectively controls the expression of the target coding sequence. Examples of expression regulatory sequences include promoters, enhancers, and transcription termination regions. A promoter is an expression regulatory sequence consisting of a region of a DNA molecule that is typically within 100 nucleotides upstream of the transcriptional initiation site (typically in the vicinity of the start site of RNA polymerase II). In order for the coding sequence to be under the control of the promoter, the translation initiation site of the polypeptide translation reading frame must be located between 1 and about 50 nucleotides downstream of the promoter. Enhancers provide expression specificity in terms of time, position and level. Unlike promoters, enhancers can function at different distances from the transcription site. Enhancers can also be located downstream of the transcription initiation site. When an RNA polymerase is capable of transcribing a coding sequence into an mRNA, which can then be translated into a protein encoded by the coding sequence, the coding sequence is "operably linked" to an expression regulatory sequence in a cell and is under "control" of the expression regulatory sequence.

Suitable expression vectors include, but are not limited to, plasmids and viral vectors derived from, for example, bacteriophages, baculoviruses, tobacco mosaic viruses, herpes viruses, cytomegaloviruses, retroviruses, vaccinia viruses, adenoviruses, and adeno-associated viruses. Many vectors and expression systems are commercially available from companies such as Novagen (Madison, WI), Clontech (Palo Alto, CA), Stratagene (LaJolla, CA), and Invitrogen Life Technologies (Carlsbad, CA).

An expression vector can comprise a tag sequence. A tag sequence is generally expressed as a fusion with the encoded polypeptide. Such tags can be inserted at any position within the polypeptide, including the carboxyl or amino terminus. Examples of useful tags include, but are not limited to, Fc fragments, polyhistidine, green fluorescent protein (GFP), glutathione S-transferase (GST), c-myc, hemagglutinin, FlagTM tags (Kodak, NewHaven, CT), maltose E-binding protein, and protein A. In some embodiments, the nucleic acid molecule encoding the GFRAL fusion polypeptide is present in a vector comprising a nucleic acid encoding one or more domains of an Ig heavy chain constant region, e.g., an amino acid sequence (Fc fragment) corresponding to the hinge region, CH2 region, and CH3 region of the human immunoglobulin Cγ1 chain.

In an eighth aspect, the present application provides a host cell comprising the nucleic acid molecule of the sixth aspect or the vector of the seventh aspect. In certain embodiments of the present application, the host cell can be a prokaryotic host cell, a eukaryotic host cell, or a phage. The prokaryotic host cell can be *Escherichia coli, Bacillus subtilis, Streptomyces* or *Proteus mirabilis.* The eukaryotic host cell can be a fungus such as *Pichia pastoris, Saccharomyces cerevisiae, Schizosaccharomyces cerevisiae*, or *Trichoderma*, an insect cell such as grass armyworm, or a plant cell such as tobacco, or a mammal cell such as a BHK cell, a CHO cell, a COS cell, or a myeloma cell. In some embodiments, the host cells described herein are preferably mammal cells, more preferably BHK cells, CHO cells, NSO cells, or COS cells.

In a ninth aspect, the present application provides an antibody-drug conjugate, also referred to as an antibody-drug conjugate, comprising the anti-GFRAL antibody or antigen-binding portion thereof according to any one embodiment of the first to fifth aspects conjugated with a therapeutic agent.

In some embodiments of the ninth aspect, the therapeutic agent is an anti-tumor drug, e.g., a cytotoxic drug, an immunopotentiator, or a radioisotope.

In some embodiments, the cytotoxic drugs include tubulin inhibitors (e.g., alkaloids), DNA topoisomerase inhibitors, DNA damaging agents, antimetabolites, or antitumor antibiotics.

In some embodiments, the tubulin inhibitors include, but are not limited to, auristatin derivatives (e.g., MMAE (Monomethyl auristatin E), MMAF (Monomethyl auristatin F)) or maytansinoid derivatives (e.g., DM1, DM4, Ansamitocin, Mertansine, or dolastatin and derivatives thereof).

In some embodiments, the DNA topoisomerase inhibitor is a camptothecin analog or a DNA topoisomerase I inhibitor and derivatives thereof, such as, for example, DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxy-xystyridine, topotecan, lertotecan, beilotecan, ecocitracon, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]ami no]-5H-indolo[2,3-a]pyrrolo[3,4-c]carbazole-5,7(6H)-dione, N-[2-(dimethylamino)ethyl]-4-acridinecarboxamidedihydrochloride, N-[2-(dimethylamino)ethyl]-4-acridinecarboxamide.

In some embodiments, the DNA damaging agents include, but are not limited to, calicheamicin, duocarmycin, and pyrrolobenzodiazepine derivatives PBD.

In some embodiments, the antimetabolite includes, but is not limited to, methotrexate, 6-mercaptopurine, or 5-fluorouracil.

In some embodiments, the anti-tumor antibiotics include, but are not limited to, polypeptide antibiotics (e.g., actinomycin D or bleomycin) or anthraquinones (e.g., doxorubicin or mitoxantrone hydrochloride).

In some embodiments, the radioisotope includes, but is not limited to, ²¹¹At, ¹³¹I, ¹²⁵I, ⁹⁰Y, ¹⁸⁶Re, ¹⁸⁸Re, ¹⁵³Sm, ²¹²Bi, ³²P, ⁶⁰Co, or ¹⁷⁷Lu.

In some embodiments, the immunopotentiators include, but are not limited to, levamisole, pidomod, imiquimod, isoinosine, polyinosinic acid, or polyinosinic acid.

In some embodiments, an anti-GFRAL antibody of the present application is covalently linked to a drug moiety through a linker. In some embodiments, the linker is a cleavable linker. In some embodiments, the linker can be cleaved under intracellular conditions. In one embodiment, the linker is hydrolyzable at a pH of less than 5.5. In some embodiments, the linker can be cleaved by an intracellular protease. In some embodiments, the linker is a cathepsin cleavable linker. In some embodiments, the linker comprises a dipeptide. In some embodiments, the dipeptide is valine (Val) -citrulline (Cit). In some embodiments, the antibody is coupled to the linker through a thiol of a cysteine in the antibody. In one embodiment, the antibody is coupled to the linker through an amino group of the antibody, in particular the amino group of the glutamine residue. Non-limiting examples of linkers include mc-Val-Cit-pAB, mc-Val-Cit-pABC, mc-Val-Cit, NH₂-(PEG)ₘ-Val-Cit, NH₂-(PEG)ₘ-Val-Cit-pAB, where m is an integer from 1 to 8.

In a tenth aspect, the present application provides a pharmaceutical composition comprising an anti-GFRAL antibody or an antigen-binding portion thereof according to any one embodiment of the first to fifth aspects or an antibody-drug conjugate according to the ninth aspect, and a pharmaceutically acceptable carrier.

In some embodiments, the pharmaceutical composition can further comprise one or more of lubricants, such as talc, magnesium stearate, and mineral oil, wetting agents, emulsifiers, suspending agents, preservatives such as benzoic acid, sorbic acid and calcium propionate, sweetening agents and/or flavoring agents. In some embodiments, the pharmaceutical compositions herein can be formulated as tablets, pills, powders, lozenges, elixirs, suspensions, emulsions, solutions, syrups, suppositories, or capsules.

In some embodiments, the pharmaceutical compositions of the present application can be delivered using any physiologically acceptable mode of administration including, but not limited to, oral administration, parenteral administration, nasal administration, rectal administration, intraperitoneal administration, intravascular injection, subcutaneous administration, transdermal administration, and inhalation administration.

In some embodiments, the pharmaceutical composition for therapeutic use can be formulated for storage in a lyophilized formulation or in the form of an aqueous solution by mixing the agents having the desired purity with pharmaceutically acceptable carriers or excipients, where appropriate.

In an eleventh aspect, the present application provides the use of the antibody or antigen-binding portion thereof of any one embodiment of the first to fifth aspects, the nucleic acid molecule of the sixth aspect, the vector of the seventh aspect, the host cell of the eighth aspect, or the antibody-drug conjugate of the eighth aspect, for the manufacture of a medicament for the treatment or prevention of a GFRAL-mediated or GFRAL-RET-GDF15 signaling pathway-mediated disease or abnormality, e.g., cachexia syndrome, a tumor (including a cancer), a cardiovascular disease, a renal disease, an ischemic disease, a metabolic disorder, a neurodegenerative disease, anorexia, or weight loss due to anorexia.

In a twelfth aspect, the present application provides a method of treating a GFRAL-mediated or GFRAL-RET-GDF15 signaling pathway-mediated disease or abnormality in a subject, the method comprising administering to the subject a therapeutically effective amount of the antibody or antigen-binding portion thereof of any one embodiment of the first to fifth aspects, the antibody-drug conjugate of the ninth aspect, or the pharmaceutical composition of the tenth aspect. For example, the disease or abnormality is cachexia syndrome, a tumor (including a cancer), a cardiovascular disease, a renal disease, an ischemic disease, a metabolic disorder, a neurodegenerative disease, anorexia, or weight loss due to anorexia. In some embodiments, the cachexia syndrome is a cachexia syndrome caused by a tumor, including a cancer. Tumors, cancers, or abnormally proliferative diseases in the present application include, but are not limited to, cancers including bladder cancer, breast cancer, colon cancer, renal cancer, liver cancer, lung cancer, ovarian cancer, pancreatic cancer, gastric cancer, cervical cancer, thyroid cancer, and skin cancer; squamous cell carcinoma; lymphoblastic hematopoietic tumors including leukemia, acute lymphocytic leukemia, acute lymphoblastic leukemia, B-cell lymphoma, T-cell lymphoma, Burkitt lymphoma; myeloid hematopoietic tumors including acute and chronic myeloid leukemia and promyelocytic leukemia; tumors of mesenchymal origin, including fibrosarcoma and rhabdomyosarcoma; tumors of the central and peripheral nervous systems, including astrocytomas, neuroblastomas, gliomas, and schwannomas; tumors of mesenchymal origin including fibrosarcoma, rhabdomyosarcoma, and osteosarcoma; and other tumors including melanoma, xeroderma pigmentosum, keratoacanthoma, seminoma, thyroid follicular carcinoma, and teratocarcinoma.

In some embodiments, the tumor (including a cancer) includes, but is not limited to, a hematologic tumor or a solid tumor.

In some embodiments, the tumor (including a cancer) is a hematologic tumor, preferably a lymphoma or leukemia, including but not limited to myeloma, B-cell lymphoma, mantle cell lymphoma, non-Hodgkin B-cell lymphoma, non-Hodgkin T-cell lymphoma, cutaneous lymphoma, anaplastic large cell lymphoma, multiple myeloma, inert non-Hodgkin lymphoma, plasmacytoma, chronic lymphocytic leukemia, small lymphocyte lymphoma, and follicular lymphoma. In some embodiments, the hematologic tumor is recurrent or refractory.

In some embodiments, the cancer tumor (including a cancer) is a solid tumor, including, but not limited to, a respiratory tumor, a digestive tract tumor, a urinary tract tumor, a male organ tumor, a female organ tumor, a skin cancer, an endothelial cell tumor, a brain tumor, a nervous system tumor, and an endocrine organ tumor.

In some embodiments, the respiratory tumor includes, but is not limited to, lung cancer, nasopharyngeal cancer and laryngeal cancer.

In some embodiments, the gastrointestinal tumor includes, but is not limited to, esophageal cancer, gastric cancer, colorectal cancer, liver cancer, pancreatic cancer, and bile duct cancer.

In some embodiments, the urological tumor includes, but is not limited to, renal cancer, renal pelvis ureteral cancer, bladder cancer, and urethral cancer.

In some embodiments, the male organ tumor includes, but is not limited to, penile cancer, prostate cancer, and testicular cancer.

In some embodiments, the female organ tumor includes, but is not limited to, breast cancer, vulvar cancer, vaginal cancer, cervical cancer, uterine body cancer, and ovarian cancer.

In some embodiments, the neurological tumor includes, but is not limited to, astrocytoma, oligodendroglioma, ependymoma, medulloblastoma, and meningioma.

In some embodiments, the brain tumor includes, but is not limited to, glioma, neurocytoma, germinal mesenchymal tumor, stromal tumor, epithelial tumor, teratoma, and pinealoma.

In some embodiments, the skin cancer includes, but is not limited to, skin melanoma or non-melanoma skin cancer.

In some embodiments, the cancer is a solid tumor, including, but not limited to, bladder cancer, brain cancer, breast cancer, cervical cancer, thoracic tumors, endometrial cancer, esophageal squamous cell cancer, gastric cancer, head tumors, pancreatic cancer, bile duct cancer, colorectal cancer, ocular cancer, head and neck squamous cell cancer, urothelial cancer, renal cancer, liver cancer, lymph node cancer, lung cancer, oral cancer, neck tumors, ovarian cancer, prostate cancer, testicular cancer, laryngeal cancer, uterine cancer, melanoma, salivary adenocarcinoma, fibrosarcoma, soft tissue sarcoma, and osteosarcoma. In some embodiments, the above cancer is recurrent or refractory.

In some embodiments, the breast cancer includes ductal cancer, lobular cancer, myeloid cancer, glial cancer, tubular cancer, inflammatory breast cancer, and triple negative breast cancer (TNBC).

In some embodiments, the ovarian cancer includes epithelial ovarian tumors such as adenocarcinoma in the ovary and adenocarcinoma migrating from the ovary to the abdominal cavity.

In some embodiments, the leukemia includes acute myeloid leukemia (AML), acute lymphocytic leukemia, chronic lymphocytic leukemia, chronic myeloid leukemia, hairy cell leukemia, myelodysplasia, myeloproliferative disorders, NK cell leukemia (e.g., blastoplasmacytoid dendritic cell tumors), acute myeloid leukemia (AML), chronic myeloid leukemia (CML), mastocytosis, chronic lymphocytic leukemia (CLL), multiple myeloma (MM), and myelodysplastic syndrome (MDS).

In some embodiments, the pancreatic cancer is acinar cell adenocarcinoma of the pancreas, ductal cell adenocarcinoma of the pancreas, or stage IV pancreatic cancer.

In some embodiments, the lung cancer includes non-small cell lung cancer (NSCLC) and small cell lung cancer (SCLC). In some embodiments, non-small cell lung cancer (NSCLC) includes, but is not limited to, squamous cell carcinoma, adenocarcinoma, and large cell carcinoma. In some embodiments, the lung cancer is recurrent. In some embodiments, the lung cancer is recurrent squamous cell lung cancer or (advanced) stage IV squamous cell lung cancer.

In some embodiments, the prostate cancer is metastatic castration-resistant prostate cancer (mCRPC).

In a thirteenth aspect, the present application provides a conjugate comprising the anti-GFRAL antibody or antigen-binding portion thereof of any one embodiment of the first to fifth aspects and a detectable label. The detectable label is, for example, a detectable fluorescent label, a chemiluminescent label, or an isotopic label.

In a fourteenth aspect, the present application provides a fusion protein comprising the anti-GFRAL antibody or antigen-binding portion thereof of any one embodiment of the first to fifth aspects. Examples of antibody fusion proteins include Fab fusion proteins, Fc fusion proteins and single chain fragment variable (scFv) fusion proteins, which are named depending on the site to which the effector protein (e.g., cytokine) is fused.

It is to be understood that the foregoing detailed description is intended only to enable those skilled in the art to have a better understanding of the present application and is not intended to be limiting in any way. Various modifications and alterations can be made to the described embodiments by those skilled in the art.

### EXAMPLES

Specific embodiments of the present application will be described in detail below in connection with the Examples, but those skilled in the art will appreciate that the following Examples are merely illustrative of the inventions of the present application and should not be construed as limiting the scope of the present application.

Where specific conditions are not indicated in the Examples, the Examples are carried out in accordance with conventional conditions or conditions suggested by the manufacturers. The reagents or the instruments used, without indication of the manufacturers, are conventional products which are commercially available.

### EXAMPLE 1: Production of Murine Antibodies

In order to produce an anti-GFRAL antibody, the inventors induced the production of antibodies against human GFRAL by immunizing mice with human GFRAL-His fusion protein (Kactus, Cat. No. GFL-HM401) and cells overexpressing human GFRAL protein.

Preparation of cells overexpressing human GFRAL - HEK293 cells were infected with a lentivirus carrying the pLVX-IRES-puromycin-hGFRAL gene. After 72 h, a monoclonal cell strain was selected by adding puromycin at a final concentration of 1µg/ml, and the cell strain with high expression of human GFRAL was screened by FACS method for animal immunization.

Animals were immunized with human GFRAL-His fusion protein or cells overexpressing human GFRAL in the absence or presence of adjuvants (Freund's complete adjuvant, or Freund's incomplete adjuvant) by subcutaneous or intraperitoneal injection. Animals were continuously induced for antibody production by booster immunization every 2 weeks until desired titers were achieved. Blood was collected from animals after each booster immunization, and antibody titers in serum were detected by ELISA and FACS methods. Four days after the final immunization, the spleens of animals having desired titer were taken and prepared into single cell suspensions. Cells were fused to SP2/0 mouse myeloma cells using electrofusion. The confluent cells were resuspended in a medium containing the hybridoma cell selector thymidine, hypoxanthine and aminopterin (HAT) and then seeded into 96-well plates for culture.

After 7-10 days of incubation, the culture supernatant was collected. Antibody clones capable of binding to human GFRAL and cynomolgus monkey GFRAL proteins were screened by ELISA or FACS methods as described in Example 2 and verified for binding to mouse GFRAL protein. Hybridoma cells were further cultured after addition of a fresh HAT-containing medium. Two days later, culture supernatants from the first round of selected positive clones were collected and assayed for antagonistic functional activities of anti-GFRAL antibodies as described in Example 3. Thereafter, the selected positive clones were further subcloned. After successful subcloning, these antibodies were purified by conventional antibody purification methods, and the antagonistic function of the antibodies was further determined by the antagonistic function assay of anti-GFRAL antibodies described in Example 3. At the same time, the variable regions of some of the desired clones were sequenced.

A total of 5 rounds of fusion screening were performed and approximately 30,000 clones were screened. More than 1,000 clones were screened by ELISA and FACS, and subjected to GFRAL antagonistic function detection. Less than 100 clones were screened for subcloning. Finally, the antibodies of the present application were obtained.

The related sequences of the antibodies in the present application are as follows:

**Table 1: Sequences of Murine Antibodies underlined parts show CDRs)**

| Name | Numbering (IMGT) | |
|---|---|---|
| 61G8 | Heavy chain CDR1: GYGVN | SEQ ID NO. 1 |
| | Heavy chain CDR2: MIWGDGTTDYNSALKS | SEQ ID NO.2 |
| | Heavy chain CDR3: DPKYNYGYAMDY | SEQ ID NO.3 |
| | Heavy chain variable region: | |
| | QVQLKESGPGLVAPSQSLSITCTVSGFSLTGYGVNWVRQSPGKGLEWLGMIWGDGTTDYNSALKS RLTIRKDNSKSQVFLRMNSLQTDDTARYYCARDPKYNYGYAMDYWGQGTSVTVSS SEQ ID NO.4 | |
| | Light chain CDR1: RASESVDNFGNSFMH | SEQ ID NO.5 |
| | Light chain CDR2: RASNLES | SEQ ID NO.6 |
| | Light chain CDR3: QQTNEEPYT | SEQ ID NO.7 |
| | Light chain variable region: | |
| | DIVLTQAPGSLAVSLGQRATISCRASESVDNFGNSFMHWYQQKPGQSPKLLIYRASNLESGIPARF SGSGSRTDFTLTINPVEADDVATYYCQQTNEEPYTFGGGTKLEIE SEQ ID NO.8 | |
| 90E1 | Heavy chain CDR1: SYVIH | SEQ ID NO.9 |
| | Heavy chain CDR2: YINPYNDDINYNEKFKG | SEQ ID NO.10 |
| | Heavy chain CDR3: YDYDWYFDV | SEQ ID NO.11 |
| | Heavy chain variable region: | |
| | EVQLQQSGPELVKPGASVKMSCKASGYTFTSYVIHWVKQEPGQGLEWIGYINPYNDDINYNEKFKGK ATLTSDKSSSTAYMEFSSLTSEDSAVYYCARYDYDWYFDVWGAGTAVTVSS SEQ ID NO.12 | |
| | Light chain CDR1: KSSQSLLDSDGKTYVN | SEQ ID NO.13 |
| | Light chain CDR2: LVSKLGS | SEQ ID NO.14 |
| | Light chain CDR3: WQGTHFPQT | SEQ ID NO.15 |
| | Light chain variable region: | |
| | DVVMTQTPLTLSVTIGQPASISCKSSQSLLDSDGKTYVNWLLQRPGQSPKRLIFLVSKLGSGVPDRFTG SGSGTDFTLKISRVEAEDLGVYYCWQGTHFPQTFGGGTKLEIK SEQ ID NO.16 | |
| 82G8 | Heavy chain CDR1: GHGVN | SEQ ID NO.17 |
| | Heavy chain CDR2: MIWGDGYTDYNSALKS | SEQ ID NO.18 |
| | Heavy chain CDR3: DTYGRTYDYAMDY | SEQ ID NO.19 |
| | Heavy chain variable region: | |
| | QVQLKESGPGLVAPSQSLSITCTVSGFSLTGHGVNWVRQPPGKGLEWLGMIWGDGYTDYNSALKSRLSISKDN | |
| | SKSQVFLKMNSLQTDDTATYYCARDTYGRTYDYAMDYWGQGTSVTVSS SEQ ID NO.20 | |
| | Light chain CDR1: RASESVDNYGNSFMH | SEQ ID NO.21 |
| | Light chain CDR2: RASNLES | SEQ ID NO.6 |
| | Light chain CDR3: QQSNEDPRT | SEQ ID NO.22 |
| | Light chain variable region: | |
| | DIVLTQSPASLAVSLGQRAAISCRASESVDNYGNSFMHWYQQKPGQPPKLLIYRASNLESGIPARFSGSGSRTD FTLTINPVEADDVATYYCQQSNEDPRTFGGGTKLEIK SEQ ID NO.23 | |
| 8E10 | Heavy chain CDR1: NYWMN | SEQ ID NO.24 |
| | Heavy chain CDR2: QIYPGDDDADYNGKFQG | SEQ ID NO.25 |
| | Heavy chain CDR3: SGRRDFDY | SEQ ID NO.26 |
| | Heavy chain variable region: | |
| | QVQLQQSGSELVRPGSSVKISCRASGYTFSNYWMNWVKQRPGQGLEWIGQIYPGDDDADYNGKFQG KATLTADKSSSTAYMQLNTLTSEDSAVYFCTRSGRRDFDYWGQGTTLTVSS SEQ ID NO.27 | |
| | Light chain CDR1: RASESIDNYGNSFMH | SEQ ID NO.28 |
| | Light chain CDR2: RASNLES | SEQ ID NO.6 |
| | Light chain CDR3: QQNNEDPYT | SEQ ID NO.29 |
| | Light chain variable region: | |
| | NIVLTQSPASLAVSLGQRATISCRASESIDNYGNSFMHWYQQKPGQPPKLLIYRASNLESGVPARFSGSGS RTDFTLTIDPVEADDAATYYCQQNNEDPYTFGGGTKLEIK SEQ ID NO.30 | |
| 174E10 | Heavy chain CDR1: SYGVH | SEQ ID NO.31 |
| | Heavy chain CDR2: VIWSGGRIDYNAAFIS | SEQ ID NO.32 |
| | Heavy chain CDR3: KGSSYYGLYYYGMDY | SEQ ID NO.33 |
| | Heavy chain variable region: | |
| | QVQLKQSGPGLVQPSQSLSITCTVSGFSLTSYGVHWVRQSPGKGLEWLGVIWSGGRIDYNAAFISRLSIS KDNSKSQVFFKMNSLQADDTAIYYCARKGSSYYGLYYYGMDYWGQGTSVTVSS SEQ ID NO.34 | |
| | Light chain CDR1: KSSQSLLNSGNQKNYLT | SEQ ID NO.35 |
| | Light chain CDR2: WASTRES | SEQ ID NO.36 |
| | Light chain CDR3: QNDYGYPLT | SEQ ID NO.37 |
| | Light chain variable region: | |
| | DIVMTQSPSSLTVTAGEKVTMSCKSSQSLLNSGNQKNYLTWYQQKSGQTPKLLIYWASTRESGVPDRFT GSGSGTDFTLTISSVQAEDLAVYYCQNDYGYPLTFGAGTKLELK SEQ ID NO.38 | |
| 96C12 | Heavy chain CDR1: GYSVN | SEQ ID NO.39 |
| | Heavy chain CDR2: MIWGDGSTDYNSALKS | SEQ ID NO.40 |
| | Heavy chain CDR3: DRDYRYDGGYIMDS | SEQ ID NO.41 |
| | Heavy chain variable region: | |
| | RVQLRESGPGLVAPSQSLSITCTVSGFSLTGYSVNWVRQPPGKGLEWLGMIWGDGSTDYNSALKSRLSIS KDNSKSQVFLKMNSLQTDDTARYYCARDRDYRYDGGYIMDSWGQGTSVTVSS SEQ ID NO.42 | |
| | Light chain CDR1: RASESVDNFGNSFLH | SEQ ID NO.43 |
| | Light chain CDR2: RASNLES | SEQ ID NO.6 |
| | Light chain CDR3: QQSNEDPRT | SEQ ID NO.22 |
| | Light chain variable region: | |
| | DIVLTQSPGSLAVSLGQRATISCRASESVDNFGNSFLHWYQQKPGQPPKLLIYRASNLESGVPARFSGSGSR TDFTLTISPVEADDVATYYCQQSNEDPRTFGGGTKLEIR SEQ ID NO.44 | |
| 36D1 | Heavy chain CDR1: RNWMH | SEQ ID NO.45 |
| | Heavy chain CDR2: EIDPSDSYTHYNQKFKG | SEQ ID NO.46 |
| | Heavy chain CDR3: VGNYDNGKDRLYAMDY | SEQ ID NO.47 |
| | Heavy chain variable region: | |
| | QVQLQQPGTEFVKPGASVKLSCKASGYTFTRNWMHWLKQRPGQGLEWIGEIDPSDSYTHYNQKFKGKA TLTVDKSSSTAYMQLSSLTSEDSAVYYCARVGNYDNGKDRLYAMDYWGQGTSVTVSS SEQ ID NO.48 | |
| | Light chain CDR1: RASESVDNFGNTFLH | SEQ ID NO.49 |
| | Light chain CDR2: RASNLES | SEQ ID NO.6 |
| | Light chain CDR3: QQNNEDPWT | SEQ ID NO.50 |
| | Light chain variable region: | |
| | DIVLTQSPASLAVSLGQRATISCRASESVDNFGNTFLHWFRQKPGQPPKLLIYRASNLESGIPARFSGSGSRTDF TLTINPVEADDVATYYCQQNNEDPWTFGGGTKLEIK SEQ ID NO.51 | |
| 33B12 | Heavy chain CDR1: RNWMH | SEQ ID NO.45 |
| | Heavy chain CDR2: EIDPSDSYTHYNQKFKG | SEQ ID NO.46 |
| | Heavy chain CDR3: VGNYDNGTDRLYAMDY | SEQ ID NO.47 |
| | Heavy chain variable region: | |
| | QVQLQQPGTELVKPGASVKLSCKASGYTFTRNWMHWLKQRPGQGLEWIGEIDPSDSYTHYNQKFKGKATLTV EMSSSTAYMQLRSLTSEDSAVYYCARVGNYDNGTDRLYAMDYWGQGTSVTVSS SEQ ID NO.52 | |
| | Light chain CDR1: RASESVDNFGNTFLH | SEQ ID NO.49 |
| | Light chain CDR2: RASNLES | SEQ ID NO.6 |
| | Light chain CDR3: QQNNEDPWT | SEQ ID NO.50 |
| | Light chain variable region: | |
| | DIVLTQSPASLAVSLGQRATISCRASESVDNFGNTFLHWFRQKPGQPPKLLIYRASNLESGIPARFSGSGSRTDF TLTINPVEADDVATYYCQQNNEDPWTFGGGTKLEIK SEQ ID NO.51 | |
| 174D6 | Heavy chain CDR1: SYGVH | SEQ ID NO.31 |
| | Heavy chain CDR2: VIWSGGRTDYNAAFIS | SEQ ID NO.53 |
| | Heavy chain CDR3: KGSSYYGLYYYGMDF | SEQ ID NO.54 |
| | Heavy chain variable region: | |
| | QVQLKQSGPGLVQPSQSLSITCTVSGFSLTSYGVHWVRQSPGKGLEWLGVIWSGGRTDYNAAFISRLNISKDS SKSQVFFKMNSLQADDTAIYYCARKGSSYYGLYYYGMDFWGQGTSVTVSS SEQ ID NO.55 | |
| | Light chain CDR1: RASESVDNYGISFMN | SEQ ID NO.56 |
| | Light chain CDR2: VASNQGS | SEQ ID NO.57 |
| | Light chain CDR3: QHSKEVPRT | SEQ ID NO.58 |
| | Light chain variable region: | |
| | DIVLTQSPASLTVSLGQRATISCRASESVDNYGISFMNWFQQKPGQPPKLLIYVASNQGSGVPARFSGSGSGTDFS LNIHPMEEDDTAMYFCQHSKEVPRTFGGGTKLEIK SEQ ID NO.59 | |
| 165C5 | Heavy chain CDR1: SYAMS | SEQ ID NO.60 |
| | Heavy chain CDR2: SITTGGSTYYLDSVRG | SEQ ID NO.61 |
| | Heavy chain CDR3: SLIITATGFDY | SEQ ID NO.62 |
| | Heavy chain variable region: | |
| | EVKLVESGGGLVKPGGSLKLSCAASGFTFSSYAMSWVRQTPEKRLEWVASITTGGSTYYLDSVRGRFTISRDNA RNILSLQMSSLRSEDTAIYYCARSLIITATGFDYWGQGTTLTVSS SEQ ID NO.63 | |
| | Light chain CDR1: RASQDVGTAVA | SEQ ID NO.64 |
| | Light chain CDR2: SASNRYT | SEQ ID NO.65 |
| | Light chain CDR3: QQYSIYYT | SEQ ID NO.66 |
| | Light chain variable region: | |
| | DIVMTQSQKFMSISVGDRVNITCRASQDVGTAVAWYQQKPGQSPKLLIYSASNRYTGVPNRFTGSGSGTDFTLAI SNMQSEDLADYFCQQYSIYYTFGGGTKLEIK SEQ ID NO.67 | |
| 2D4 | Heavy chain CDR1: SFVMS | SEQ ID NO.68 |
| | Heavy chain CDR2: SITSGGTTFYPDSVKG | SEQ ID NO.69 |
| | Heavy chain CDR3: GPPQFSSLSMDY | SEQ ID NO.70 |
| | Heavy chain variable region: | |
| | EVNLVESGGGLVKPGGPLKLSCAASGFTFSSFVMSWVRQTPEKRLEWVASITSGGTTFYPDSVKGRFTISRDNA RNILYLQMSNLRSGDTAMYYCARGPPQFSSLSMDYWGQGTSVTVSS SEQ ID NO.71 | |
| | Light chain CDR1: KASQNVGTAVS | SEQ ID NO.72 |
| | Light chain CDR2: SASNRYT | SEQ ID NO.65 |
| | Light chain CDR3: QQYSSLFT | SEQ ID NO.73 |
| | Light chain variable region: | |
| | DIVMTQSQKFMSTSVGDRITITCKASQNVGTAVSWFQQKPGQVPKLLIYSASNRYTGVPDRFTGSGSGTEFTLTI | |
| | SNIQSEDLADYFCQQYSSLFTFGSGTKLEIK SEQ ID NO.74 | |
| 26D7 | Heavy chain CDR1: GYTFTDYGMN | SEQ ID NO.75 |
| | Heavy chain CDR2: WINTYTGEPTYADDFKD | SEQ ID NO.76 |
| | Heavy chain CDR3: RYGYDAGIDC | SEQ ID NO.77 |
| | Heavy chain variable region: | |
| | QIQLVQSGPELKKPGETVKISCKVSGYTFTDYGMNWVKQTPGKDLKWVGWINTYTGEPTYADDFKDRFAFSLE ASASTAYLQINNLKNEDTATYFCARRYGYDAGIDCWGQGTTLTVSS SEQ ID NO.78 | |
| | Light chain CDR1: RASESVDSYGNNLMH | SEQ ID NO.79 |
| | Light chain CDR2: LASYLES | SEQ ID NO.80 |
| | Light chain CDR3: QQNNEDPWT | SEQ ID NO.50 |
| | Light chain variable region: | |
| | NIVLTQSPPSLAVSLGQRATISCRASESVDSYGNNLMHWYQQKPGQPPKLLIYLASYLESGVPARFSGSGSRTD FTLTIDPVEADDAATYYCQQNNEDPWTFGGGTKLEIK SEQ ID NO.81 | |
| 168A7 | Heavy chain CDR1: TSDMGVG | SEQ ID NO.82 |
| | Heavy chain CDR2: HIWWDDDKYYNPSLKR | SEQ ID NO.83 |
| | Heavy chain CDR3: LYDYDDEYYFDY | SEQ ID NO.84 |
| | Heavy chain variable region: | |
| | QVTLKESGPGILKPSQTLSLTCSFSGFSLSTSDMGVGWIRQPSGKGLEWLAHIWWDDDKYYNPSLKRQLTISKDN SRNQVFLKISSVDTTDTATYYCARLYDYDDEYYFDYWGQGTTLTVSS SEQ ID NO.85 | |
| | Light chain CDR1: RASQDSNNYLN | SEQ ID NO.86 |
| | Light chain CDR2: YTSRLHS | SEQ ID NO.87 |
| | Light chain CDR3: QQGNTIPWT | SEQ ID NO.88 |
| | Light chain variable region: | |
| | DIQMTQTKSSLSASLGDRVTISCRASQDSNNYLNWYQQKPDGTVKLLIYYTSRLHSGVPSRFSGSGSGTDYSLTI SNLEHEDIATYFCQQGNTIPWTFGGGTKLEIK SEQ ID NO.89 | |

### EXAMPLE 2: Screening and Selection of Anti-GFRAL Antibodies

The hybridoma-derived anti-GFRAL antibodies or purified antibodies in the culture supernatant described in Example 1 was assayed for binding to human GFRAL, cynomolgus monkey GFRAL and mouse GFRAL proteins by ELISA or FACS methods.

The ELISA assay had the following protocols. Human GFRAL-His protein, cynomolgus monkey GFRAL-His protein, or mouse GFRAL-Fc protein were coated on 96-well plates, and after blocking the wells with PBS/1%BSA solution, the hybridoma supernatant or purified antibodies were added to the reaction wells. After incubation for 1 hour at room temperature, HRP-labeled anti-mouse IgG (Fab region) antibody was added to the reaction wells to incubate for an additional hour at room temperature. After the substrate and stop solution were added successively, the OD₄₅₀ values were read. The OD₄₅₀ values were used to verify and assess the binding ability of the antibodies to the GFRAL proteins.

The FACS screening assay used a monoclonal cell line over-expressing human GFRAL which was different from that used in animal immunizations. After incubation of the hybridoma supernatant or purified antibodies with the monoclonal cells overexpressing human GFRAL for 1 hour at 4°C, Alexa Fluor 647 dye-labeled anti-mouse Fc antibody was added to the reaction wells to incubate for an additional 1 hour at 4°C. The Alexa Fluor 647 fluorescence signals on the cells were analyzed by flow cytometry to determine whether the hybridoma supernatant contained antibodies against GFRAL or to evaluate the binding ability of the antibodies to the GFRAL protein on cell surfaces.

In the ELISA assay, representative data of binding of purified antibodies described in Example 1 to human GFRAL, cynomolgus monkey GFRAL, and mouse GFRAL proteins are as follows.

**Table 2: ELISA experiments on binding of anti-GFRAL antibodies to different sources of GFRAL proteins**

| | **ELISA EC₅₀ (nM)** | | |
|---|---|---|---|
| **Clone ID** | **Human GFRAL-His** | **Cynomolgus monkey GFRAL-His** | **Mouse GFRAL-Fc** |
| 33B12 | 0.06 | 0.08 | >6.67 |
| 165C5 | 0.27 | 0.03 | 0.12 |
| 36D1 | 0.18 | 0.07 | >6.67 |
| 26D7 | 0.07 | 0.06 | 0.15 |
| 82G8 | 0.03 | 0.04 | >6.67 |
| 8E10 | 0.05 | 0.06 | >6.67 |
| 168A7 | 1.87 | 0.09 | >6.67 |
| 174D6 | 0.28 | 0.07 | 0.14 |
| 61G8 | 0.07 | 0.08 | >6.67 |
| 174E10 | 0.18 | 0.06 | 0.12 |
| 96C12 | 0.03 | 0.03 | >6.67 |
| 2D4 | 0.09 | 0.10 | 0.08 |

### EXAMPLE 3: Antagonistic Function Assay of Anti-GFRAL Antibodies

For example, based on a reporter gene assay, an antagonistic function assay of anti-GFRAL antibodies was performed on the culture supernatants or purified antibodies described in Example 1, for screening or evaluating the function of the antibodies in preventing GDF15 from activating the GFRAL/RET signaling pathway. This method utilized cells stably transfected with GFRAL/RET-related genes, and the principle that GDF15 activates the GFRAL/RET signaling pathway, causing ERK phosphorylation, which further causes Gal4-Elk1 phosphorylation and binding to the USA promoter region on the DNA, followed by initiation of luciferase reporter gene transcription. The activation of the GFRAL/RET signaling pathway by GDF15 and the antagonistic function of antibodies were determined by measuring luciferase levels.

The cell lines stably transfected with the reporter gene were constructed as follows. Two reporter plasmids Gal4-Elk1 and 6XUAS-Luc were first transfected into HEK293 cells, and cell lines stably expressing the target molecule were obtained after a period of corresponding antibiotic screening. The plasmid encoding human GFRAL (hGFRAL) or mouse GFRAL (mGFRAL) and human RET (hRET) or mouse RET (mRET) were then further introduced into the cells, and the cell strain stably transfected with GFRAL/RET/Gal4-Elk1/Luc was obtained after antibiotic screening.

For example, the antagonistic activity of the anti-GFRAL antibody is determined as follows. Cells stably transfected with reporters were seeded into 96-well plates at appropriate densities and incubated with hybridoma supernatants or antibodies to be tested in an incubator at 37 °C with 5% CO₂ and saturated humidity overnight. Cells were stimulated by addition of human GDF 15 or mouse GDF15 at certain concentrations on the following day, and luciferase levels in the cells were measured 5-6 hours later using a One-Glo (Promega) kit.

Representative results of inhibition of human GFRAL/RET signaling transduction by anti-GFRAL antibodies are as follows.

**Table 3: Blocking Activity of Anti-GFRAL Antibodies**

| Mouse source | IC₅₀ (nM) | |
|---|---|---|
| Clone ID | Human GFRAL/human RET | Mouse GFRAL/mouse RET |
| 33B12 | 7.04 | >694 |
| 165C5 | 55.48 | 7.22 |
| 36D1 | 6.89 | >694 |
| 26D7 | 6.35 | 0.76 |
| 82G8 | 5.67 | >694 |
| 8E10 | 3.33 | >694 |
| 168A7 | 3.29 | >694 |
| 174D6 | 104.40 | >694 |
| 61G8 | 11.60 | >694 |
| 174E10 | 37.17 | 104.90 |
| 96C12 | 6.42 | >694 |
| 2D4 | 11.64 | 2.23 |
| 90E1 | 81.37 | 75.50 |

### EXAMPLE 4: Antibody Affinity Assay

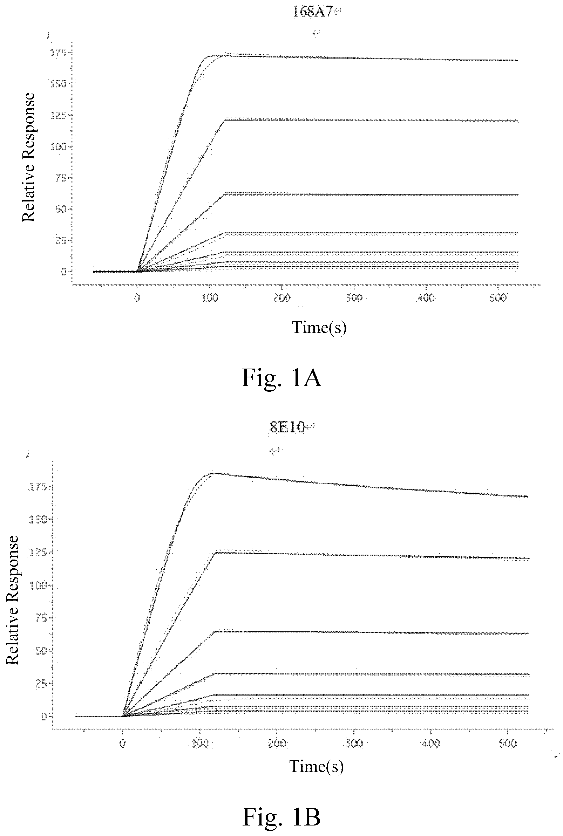

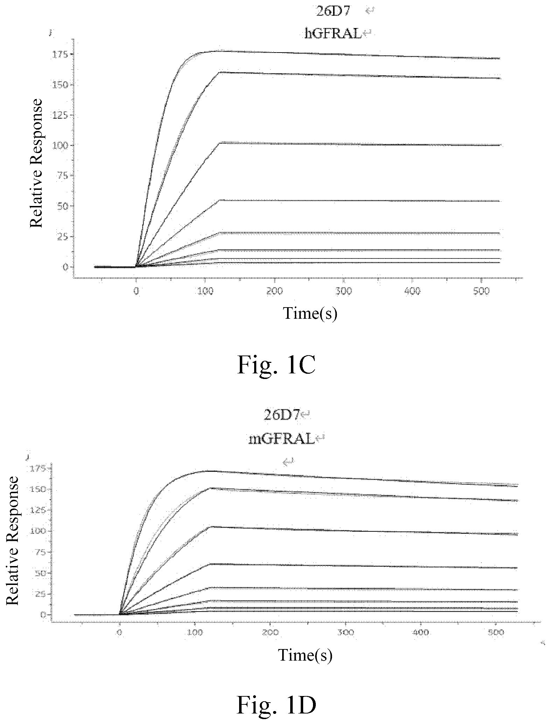

Binding affinity assays were performed for three antibody strains 168A7, 8E10 and 26D7 based on the Biacore affinity assay. The binding affinities of purified antibodies to human or mouse GFRAL were assessed by measuring the equilibrium dissociation constants (KD). The anti-human Fc antibody was immobilized in the flow cell of the CM5 chip according to the instructions of the amino-coupled kit (catalogue number: BR100050, Cytiva). Purified antibodies (~300RU) were captured on flow cells 2, 3 and 4 using flow cell 1 as a reference. Human or mouse GFRAL protein (in an HBS-EP buffer) was then injected at a flow rate of 30µL/min and the binding kinetics were evaluated at 25°C. The results were as follows (FIGs. 1A-1C, Table 4)

**Table 4: Binding Kinetic Data for Three Murine Antibodies**

| | Binding Affinity | | | |
|---|---|---|---|---|
| Murine | Human GFRAL | | Mouse GFRAL | |
| Clone ID | KD (M) | K_{off} (1/s) | KD (nM) | K_{off} (1/s) |
| 168A7 | 1.45E-12 | 7.06E-05 | Not determined | Not determined |
| 8E10 | 1.79E-11 | 4.04E-04 | Not determined | Not determined |
| 26D7 | 1.53E-11 | 9.37E-05 | 1.66E-10 | 2.93E-04 |

Thus, murine antibodies had high affinities for human GFRAL. Clone 26D7 had good binding activity to both human GFRAL and mouse GFRAL, and are suitable for use in experiments on mouse models.

### EXAMPLE 5: Evaluation of Biological Function of Murine Anti-GFRAL Antagonistic Antibodies in Animal Model Study

### 5.1 Evaluation of Effects of Anti-GFRAL Antibodies in Inhibiting Tumor-Induced Weight Loss in Human Fibrosarcoma HT-1080 Tumor-Bearing Mouse Model

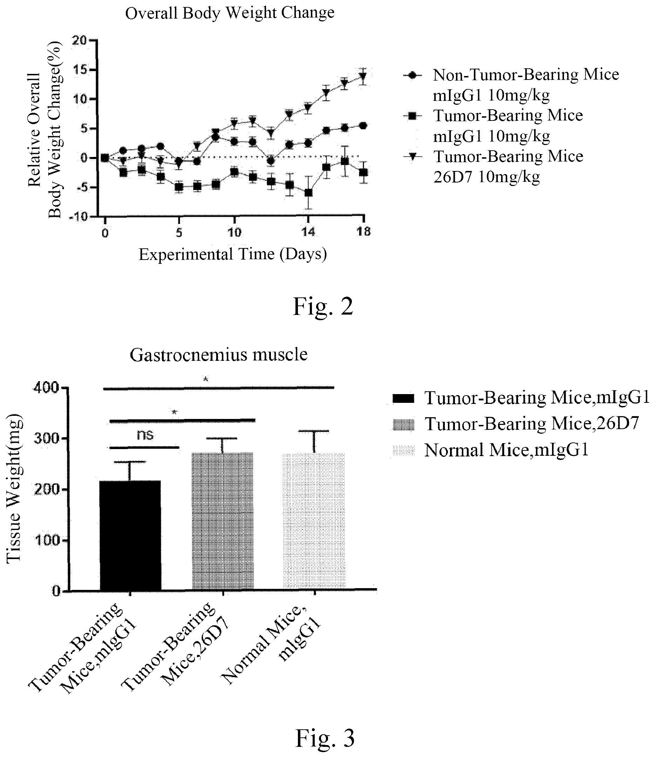

To determine whether the anti-GFRAL antibodies can alleviate tumor-induced weight loss effects in tumor-bearing mice, 6 to 9 weeks old female BALB/c nude mice (GemPharmatech Co., Ltd) were used. The mice were inoculated with an *in vitro* cultured HT1080 cell line. After the mice lost about 7% of their body weight, the inoculated mice were randomly divided into two groups, 10 mice in each group, and the two groups weekly received subcutaneous injections of 10 mg/kg of control antibody (mIgG1, source: Crownbio, a non-GFRAL-targeted negative control antibody) or anti-GFRAL antibody 26D7, respectively. Another group (n = 10) of non-tumor-bearing mice were also injected subcutaneously with 10mg/kg/weeks of control antibody. Daily body weight and food intake were recorded, and body weight data were recorded using Study Director^{™} (version 3.1.399.19) software. The data are summarized as follows. The data are expressed as means ±SEMs of tumor volume, overall body weight, and percent change in body weight (i.e., percent change in body weight relative to baseline body weight). In these experiments, P < 0.05 was considered statistically significant. The results (see FIG. 2 and Table 5) show that anti-GFRAL antibody 26D7 can reverse GDF15-induced weight loss in tumor-bearing mice.

**Table 5: Effect of Anti-GFRAL Antibody 26D7 on Mouse Body Weight and Tumor Size in Human Fibrosarcoma Ht-1080 Tumor-Bearing Model**

| **Group** | **Tumor volume (mm³)** | | **Body Weight (g)** | | **Weight change ^{a} (%)** | ***P^{b}*** |
|---|---|---|---|---|---|---|
| | **Day 0** | **Day 13** | **Day 0** | **Day 13** | **Day 13** | |
| Non-tumor-bearing mice, mIgG1 | N/A | N/A | 21.3±0.3 | 21.7±0.3 | 2.08±0.70 | 0.004 |
| Tumor-bearing mice, mIgG1 | 0 | 1721±171 | 23.1±0.4 | 22.0±0.4 | -4.73±1.91 | - |
| Tumor-bearing mice, 26D7 | 0 | 1921±129 | 23.0±0.3 | 24.6±0.2 | 7.11±0.92 | < 0.001 |

| | | | | | | |
|---|---|---|---|---|---|---|
| Note: Data are expressed as means ±SEMs; a: body weight change = [(body weight on day i - body weight on day 0)/body weight on day 0] × 100%; b: Independent t test by comparing body weight changes from day 0 to day 13 for different groups. | | | | | | |

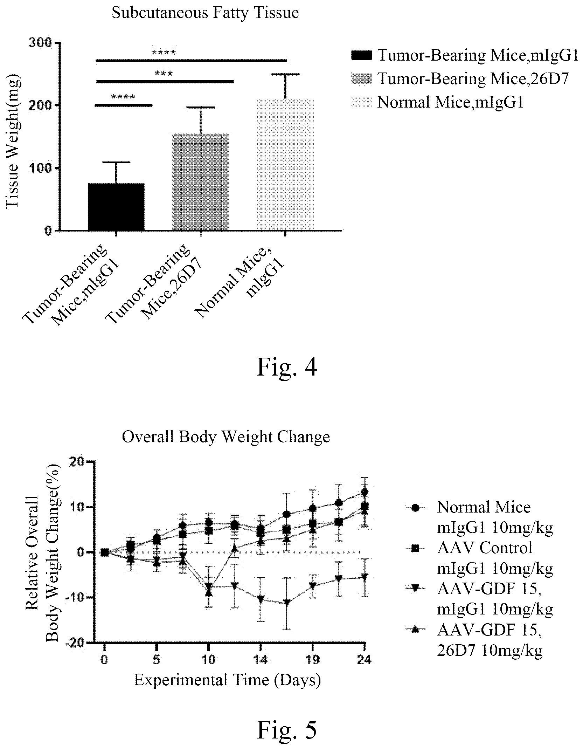

Meanwhile, at the end point of the experiment, after animals were euthanized according to animal welfare requirements, subcutaneous fat and gastrocnemius muscle of mice were isolated and weighed, and statistical analysis was performed using one way ANOVA- test. In the experiment, P < 0.05 was considered to be statistically significant. As shown in FIGs. 3 and 4, the experimental results show that anti-GFRAL antibody 26D7 can reverse the tumor-induced weight loss of the gastrocnemius muscle and subcutaneous fat in the tumor-bearing mice.

### 5.2 Evaluation of Effect of Murine Anti-GFRAL Antibodies in Reversing GDF-15-Induced Weight Loss in AAV-mGDF15 Model

An AAV-mGDF15 mouse model was constructed using 12-14 week old male C57BL/6 mice. An adenovirus-associated virus (rAAV) expressing GDF15 was first constructed with the brief protocols as follows.
1. Primers for use in PCR amplification were designed. A homologous sequence at the end of the linearized cloning vector is introduced at the 5' end of the primer. The primer sequence is designed for synthesis.
2. A bacterial solution containing the vector plasmid was incubated overnight. The plasmid was extracted from a fresh bacterial solution. Then, 1µg of fresh plasmid was taken and double-digested with the corresponding restriction enzyme. Gel recovery was performed after agarose gel electrophoresis assay of the digested product, and the concentration was determined after centrifugation of the filter column.
3. PCR amplification was carried out using diluted primers and templates. After amplification, the target gene was analyzed by agarose gel electrophoresis and recovered.
4. After determining the concentration of the recovery vector and the target fragment, the over-expression vector was ligated with the target fragment at 50°C using a HieffClone ^{™} recombinant reaction system. The ligation product was added to competent cells for transformation, and after incubation at 37 °C overnight, clones were screened for sequencing.
5. After extracting high-purity plasmids, the viral vector and auxiliary packaging element plasmids were co-introduced into AAV-293 cells using HG transgene reagents. After transfection, the cells were cultured for 48 hours, and the cells and supernatants were collected after exfoliation.

**Table 6: Primer Sequences**

| Primer name | Primer sequence (5' -3') |
|---|---|
| Forward primer | GCGAATTCGAAGTATACCTCGAGGCCACCATGGCCCCG |
| Reverse primer | GTGTCTGACTGTGTCGGATCCTCAAGCGCAGTGGCAGCCCCGG |

Based on the "Matched distribution" (StudyDirector^{™} software, version 3.1.399.19) method, the mice were randomly divided into 3 groups with 6 mice in each group based on mean body weights, wherein the mice of group 1 were not treated and the mice of groups 2 and 3 were administered a single tail vein injection of 1×10¹⁰vg of rAAV expressing mGDF15. Ten days after the rAAV injection, mice in Groups 1 and 2 were administered a 10 mg/kg dose of control antibody mIgG1 by subcutaneous injection, and mice in Group 3 were administered the same dose of anti-GFRAL antibody 26D7. Body weight was monitored three times a week and food intake was monitored twice a week. The body weight data are summarized as follows, and the data are expressed as the means±SEMs of the overall body weight and the percentage of body weight change (i.e., the percentage of body weight change relative to baseline body weight).

**Table 7: Improvement Effect of Anti-GFRAL Antibody 26D7 on Mouse Body Weight Loss in AAV-mGDF15 Mouse Model**

| **Group** | **Pre-treatment on Day 0** | **Treatment on Day 10 (10 mg/kg)** | **Day 0** | **Body Weight (g) Day 10** | **Day 24** | **Weight change ^{a} (%)** | | ***P^{b}*** |
|---|---|---|---|---|---|---|---|---|
| | | | | | | **Day 10** | **Day 24** | |
| 1 | NA | mIgG1 | 26.5±0.8 | 28.3±0.9 | 30.1±0.9 | 6.55±2.03 | 13.30±3.26 | < 0.001 |
| 2 | AAV-GDF15 | mIgG1 | 26.4±0.8 | 24.3±1.4 | 24.7±1.4 | -7.66±4.43 | -5.66±4.19 | - |
| 3 | AAV-GDF15 | 26D7 | 26.7±0.8 | 24.3±1.0 | 29.2±0.9 | -8.84±3.41 | 9.21±3.17 | < 0.001 |

| | | | | | | | | |
|---|---|---|---|---|---|---|---|---|
| Note: Data are expressed as means±SEMs; A. body weight change = [(body weight on day i - body weight on day 0)/body weight on day 0]* 100%; B. Independent t tests were performed by comparing body weight changes from day 0 to day 24 in different groups. | | | | | | | | |

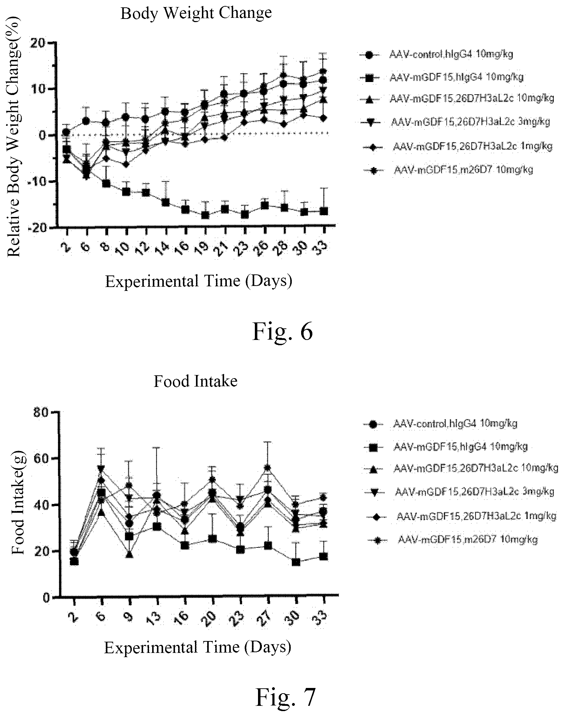

In analysis of the data in FIGs. 5 and 7, the experimental results show that anti-GFRAL antibody 26D7 can reverse the weight loss induced by GDF15 and effectively increase the weight of the mice by about 10%, which is statistically significant.

Taking the above experimental data together, it is shown that the murine anti-GFRAL antagonist antibody obtained in the present application can effectively block GFRAL/RET signal transduction and has excellent blocking activity, and that the anti-GFRAL antibody obtained in the present application can significantly reverse the weight loss induced by tumor or GDF15, as confirmed by the results from the tumor-bearing mouse model and the non-tumor-bearing mouse model experiment. The anti-GFRAL antibody potentially has a special therapeutic effect of treating or alleviating cachexia caused by cancer and other causes.

### EXAMPLE 6. Humanization of Murine Antibodies and Activity Assays

Murine antibodies 2D4 and 26D7 were selected for humanization design with the following brief protocols. The variable region structure of the murine antibody was first simulated by using softwares including Discovery Studio and Schrödinger Antibody Modeling, and the optimal structure model was selected to analyze the structure of the variable regions and the CDRs of the antibody.

Human heavy and light chain germline sequences highly homologous to the murine antibodies were screened respectively by aligning murine sequences with human germline sequences. The murine antibody CDR sequences were grafted into the corresponding positions in the human germline sequence. Based on the results of the murine antibody structure model analysis, the amino acid sequences of the murine antibody were replaced individually or in combination with the amino acid residues that play an important role in the formation of the CDR structure in the framework region, so that different heavy chain and light chain variable regions were designed (Table 8, Table 9, Table 10).

Humanized antibodies with the sequences shown in Table 8 were prepared as follows. Using human IgG4 constant region and kappa constant region, different humanized antibodies were constructed with different light and heavy chain variable region combinations (Table 11).

The antagonism of human GFRAL and murine GFRAL functions by humanized antibodies was detected using a reporter gene assay as described in Example 3, and the results are shown in Table 11.

**Table 8: Sequence Structure of Obtained Humanized Antibodies**

| **Antibody 1: FM1-CDR1-FM2-CDR2-FM3-CDR3-FM4** |
|---|
| ***2D4-mVH*** |
| EVNLVESGGGLVKPGGPLKLSCAASGFTFSSFVMSWVRQTPEKRLEWVASITSGGTTF YPDSVKGRFTISRDNARNILYLQMSNLRSGDTAMYYCARGPPQFSSLSMDYWGQGTS VTVSS （SEQ ID NO.71） |
| ***2D4-H6a*** |
| EVQLVESGGGLVKPGGSLRLSCAASGFTFSSFVMSWVRQAPGKGLEWVASITSGGTTF YPESVRGRFTISRDNAKNTLYLQMSSLRAEDTAVYYCARGPPQFSSLSMDYWGQGTT VTVSS （SEQ ID NO.90） |
| Heavy Chain CDR2: SITSGGTTFYPESVRG (SEQ ID NO.91) |
| ***2D4-H6b*** |
| EVQLVESGGGLVKPGGSLRLSCAASGFTFSSFVMSWVRQAPGKGLEWVASITSGGTTF YPDSVKGRFTISRDNAKNTLYLQMSSLRAEDTAVYYCARGPPQFSSLSMDYWGQGTT VTVSS （SEQ ID NO.92） |
| ***2D4-H6c*** |
| EVQLVESGGGLVKPGGSLRLSCAASGFTFSSFVMSWVRQAPGKGLEWVASITSGGTTF YPDSVKGRFTISRDNARNILYLQMSSLRAEDTAVYYCARGPPQFSSLSMDYWGQGTT VTVSS （SEQ ID NO.93） |
| ***2D4-mVL*** |
| DIVMTQSQKFMSTSVGDRITITCKASQNVGTAVSWFQQKPGQVPKLLIYSASNRYTGV PDRFTGSGSGTEFTLTISNIQSEDLADYFCQQYSSLFTFGSGTKLEIK （SEQ ID NO.74） |
| ***2D4-L6a*** |
| DIQMTQSPSSLSASVGDRVTITCRASQNVGTAVSWYQQKPGKAPKLLIYSASNRYTGV PSRFSGSGSGTDFTLTISSLQPEDFATYYCQQYSSLFTFGGGTKLEIK （SEQ ID NO.94） |
| Light Chain CDR1: RASQNVGTAVS (SEQ ID NO.95) |
| ***2D4-L6b*** |
| DIQMTQSPSSLSASVGDRVTITCKASQNVGTAVSWFQQKPGKAPKLLIYSASNRYTGV PSRFSGSGSGTDFTLTISSLQPEDFATYYCQQYSSLFTFGGGTKLEIK （SEQ ID NO.96） |
| ***2D4-L6c*** |
| DIQMTQSPSSLSASVGDRITITCKASQNVGTAVSWFQQKPGKAPKLLIYSASNRYTGVP DRFSGSGSGTDFTLTISSLQPEDFATYYCQQYSSLFTFGGGTKLEIK （SEQ ID NO.97） |

| **Antibody 2: FM1-CDR1-FM2-CDR2-FM3-CDR3-FM4** |
|---|
| ***26D7-mVH*** |
| QIQLVQSGPELKKPGETVKISCKVSGYTFTDYGMNWVKQTPGKDLKWVGWINTYTG EPTYADDFKDRFAFSLEASASTAYLQINNLKNEDTATYFCARRYGYDAGIDCWGQGTT LTVSS （SEQ ID NO.78） |
| ***26D7-H1a*** |
| QVQLVQSGAEVKKPGASVKVSCKASGYTFTDYGMNWVRQAPGQGLEWMGWINTY TGEPTYADDFKDRVTMTTDTSTSTAYMELRSLRSDDTAVYYCARRYGYDAGIDAWG QGTLVTVSS （SEQ ID NO.98） |
| Heavy Chain CDR3: RYGYDAGIDA (SEQ ID NO.99) |
| ***26D7-H1b*** |
| QVQLVQSGAEVKKPGASVKVSCKASGYTFTDYGMNWVRQAPGQGLEWMGWINTY TGEPTYADDFKDRVTFTLDASTSTAYMELRSLRSDDTAVYYCARRYGYDAGIDAWG QGTLVTVSS （SEQ ID NO.100） |
| ***26D7-H2a*** |
| QVQLVQSGAEVKKPGASVKVSCKVSGYTFTDYGMNWVRQAPGQGLEWVGWINTYT GEPTYADDFKDRFTFTLDTSTSTAYMELRSLRSDDTAVYFCARRYGYDAGIDAWGQG TLVTVSS （SEQ ID NO.101） |
| ***26D7-H2b*** |
| QVQLVQSGAEVKKPGASVKISCKASGYTFTDYGMNWVRQAPGQGLEWMGWINTYT GEPTYADDFKDRVTFTLDTSTSTAYMELRSLRSDDTAVYYCARRYGYDAGIDAWGQ GTLVTVSS （SEQ ID NO.102） |
| ***26D7-H2c*** |
| QVQLVQSGAEVKKPGASVKISCKASGYTFTDYGMNWVRQAPGQGLEWMGWINTYT GEPTYADDFKDRVTFTLDASTSTAYMELRSLRSDDTAVYYCARRYGYDAGIDAWGQ GTLVTVSS （SEQ ID NO.103） |
| ***26D7-H2d*** |
| QVQLVQSGAEVKKPGASVKISCKASGYTFTDYGMNWVRQAPGQGLKWMGWINTYT GEPTYADDFKDRVTFTLDASTSTAYMELRSLRSDDTAVYYCARRYGYDAGIDAWGQ GTLVTVSS （SEQ ID NO.104） |
| ***26D7-H3a*** |
| QVQLVQSGAEVKKPGASVKISCKVSGYTFTDYGMNWVRQTPGQGLKWVGWINTYT GEPTYADDFKDRFTFTLDASTSTAYMELRSLRSDDTAVYFCARRYGYDAGIDAWGQG TLVTVSS （SEQ ID NO.105） |
| ***26D7-H3h*** |
| QVQLVQSGAEVKKPGASVKISCKVSGYTFTDYGMNWVRQTPGQGLEWVGWINTYT GEPTYADDFKDRFTFTLDASTSTAYMELRSLRSDDTAVYFCARRYGYDAGIDAWGQG TLVTVSS （SEQ ID NO.106） |
| ***26D7-H3c*** |
| QVQLVQSGAEVKKPGASVKVSCKVSGYTFTDYGMNWVRQTPGQGLKWVGWINTYT GEPTYADDFKDRFTFTLDASTSTAYMELRSLRSDDTAVYFCARRYGYDAGIDAWGQG TLVTVSS （SEQ ID NO.107） |
| ***26D7 H3d*** |
| QVQLVQSGAEVKKPGASVKISCKVSGYTFTDYGMNWVRQAPGQGLKWVGWINTYT GEPTYADDFKDRFTFTLDASTSTAYMELRSLRSDDTAVYFCARRYGYDAGIDAWGQG TLVTVSS （SEQ ID NO.108） |
| ***26D7 H3e*** |
| QVQLVQSGAEVKKPGASVKISCKVSGYTFTDYGMNWVRQTPGQGLKWVGWINTYT GEPTYADDFKDRFTFTLDTSTSTAYMELRSLRSDDTAVYFCARRYGYDAGIDAWGQG TLVTVSS （SEQ ID NO.109） |
| ***26D7 H3f*** |
| QVQLVQSGAEVKKPGASVKVSCKVSGYTFTDYGMNWVRQAPGQGLKWVGWINTYT GEPTYADDFKDRFTFTLDASTSTAYMELRSLRSDDTAVYFCARRYGYDAGIDAWGQG TLVTVSS （SEQ ID NO.110） |
| ***26D7-H3g*** |
| QVQLVQSGAEVKKPGASVKVSCKVSGYTFTDYGMNWVRQAPGQGLEWVGWINTYT GEPTYADDFKDRFTFTLDASTSTAYMELRSLRSDDTAVYFCARRYGYDAGIDAWGQG TLVTVSS （SEQ ID NO.111） |
| ***26D7-H3h*** |
| QVQLVQSGAEVKKPGASVKISCKVSGYTFTDYGMNWVRQAPGQGLEWVGWINTYT GEPTYADDFKDRFTFTLDTSTSTAYMELRSLRSDDTAVYFCARRYGYDAGIDAWGQG TLVTVSS |
| （SEQ ID NO.112） |
| ***26D7-H3i*** |
| QVQLVQSGAEVKKPGASVKISCKVSGYTFTDYGMNWVRQAPGQGLEWVGWINTYT GEPTYADDFKDRFTFTLDASTSTAYMELRSLRSDDTAVYFCARRYGYDAGIDAWGQG TLVTVSS （SEQ ID NO.113） |
| ***26D7-H4a*** |
| QIQLVQSGAEVKKPGASVKISCKVSGYTFTDYGMNWVRQTPGQGLKWVGWINTYTG EPTYADDFKDRFTFTLDASTSTAYLEIRSLRSDDTAVYFCARRYGYDAGIDAWGQGTL LTVSS （SEQ ID NO.114） |
| ***26D7-H5a*** |
| QVQLVQSGAEVKKPGASVKVSCKASGYTFTDYGMNWVRQAPGQGLEWMGWINTY TGEPTYAQKLQGRVTMTTDTSTSTAYMELRSLRSDDTAVYYCARRYGYDAGIDAWG QGTTVTVSS （SEQ ID NO.115） |
| Heavy Chain CDR2: WINTYTGEPTYAQKLQG (SEQ ID NO.116) |
| ***26D7-H5b*** |
| QVQLVQSGAEVKKPGASVKVSCKVSGYTFTDYGMNWVRQAPGQGLEWMGWINTY TGEPTYAQKLQGRVTFTTDTSTSTAYMELRSLRSDDTAVYYCARRYGYDAGIDAWG QGTTVTVSS （SEQ ID NO.117） |
| ***26D7-H5c*** |
| QVQLVQSGAEVKKPGASVKVSCKVSGYTFTDYGMNWVRQAPGQGLEWVGWINTYT GEPTYADDFKDRVTFTTDTSTSTAYMELRSLRSDDTAVYYCARRYGYDAGIDAWGQ GTTVTVSS （SEQ ID NO.118） |
| ***26D7-H5d*** |
| QIQLVQSGAEVKKPGASVKISCKVSGYTFTDYGMNWVRQAPGQGLEWVGWINTYTG EPTYADDFKDRFTFTLDTSTSTAYLEIRSLRSDDTAVYYCARRYGYDAGIDAWGQGTT VTVSS （SEQ ID NO.119） |
| ***26D7-H5e*** |
| QVQLVQSGSELKKPGASVKVSCKVSGYTFTDYGMNWVRQAPGQGLEWVGWINTYT GEPTYADDFKDRFVFSLDTSVSTAYLQISSLKAEDTAVYYCARRYGYDAGIDAWGQG TTVTVSS （SEQ ID NO.120） |
| ***26D7-H5f*** |
| QIQLVQSGSELKKPGASVKISCKVSGYTFTDYGMNWVRQAPGQGLEWVGWINTYTG EPTYADDFKDRFVFSLDASVSTAYLQISSLKAEDTAVYYCARRYGYDAGIDAWGQGT TVTVSS （SEQ ID NO.121） |
| ***26D7-mVL*** |
| NIVLTQSPPSLAVSLGQRATISCRASESVDSYGNNLMHWYQQKPGQPPKLLIYLASYLE SGVPARFSGSGSRTDFTLTIDPVEADDAATYYCQQNNEDPWTFGGGTKLEIK （SEQ ID NO.81） |
| ***26D7-L1a*** |
| EIVLTQSPGTLSLSPGERATLSCRASESVDSYGNNLMHWYQQKPGQAPRLLIYLASYL |
| ESGIPDRFSGSGSGTDFTLTISRLEPEDFAVYYCQQNNESPWTFGQGTKVEIK （SEQ ID NO.122） |
| Light Chain CDR3: QQNNESPWT (SEQ ID NO.123) |
| ***26D7-L2a*** |
| EIVLTQSPGTLSLSPGERATLSCRASESVDSYGNNLMHWYQQKPGQAPRLLIYLASYL ESGVPDRFSGSGSGTDFTLTISRLEPEDFATYYCQQNNESPWTFGQGTKVEIK （SEQ ID NO.124） |
| ***26D7-L2h*** |
| EIVLTQSPGTLSLSPGERATLSCRASESVDAYGNNLMHWYQQKPGQAPRLLIYLASYL ESGVPDRFSGSGSGTDFTLTISRLEPEDFATYYCQQNNESPWTFGQGTKVEIK （SEQ ID NO.125） |
| Light Chain CDR1: RASESVDAYGNNLMH (SEQ ID NO.126) |
| ***26D7-L2c*** |
| EIVLTQSPGTLSLSPGERATLSCRASESVESYGNNLMHWYQQKPGQAPRLLIYLASYLE SGVPDRFSGSGSGTDFTLTISRLEPEDFATYYCQQNNESPWTFGQGTKVEIK （SEQ ID NO.127） |
| Light Chain CDR1: RASESVESYGNNLMH (SEQ ID NO.128) |
| ***26D7-L3a*** |
| EIVLTQSPGTLSLSPGERATISCRASESVDSYGNNLMHWYQQKPGQAPRLLIYLASYLE SGVPARFSGSGSRTDFTLTISRLEPEDFATYYCQQNNESPWTFGQGTKVEIK （SEQ ID NO.129） |
| ***26D7-L3b*** |
| EIVLTQSPGTLSLSPGERATISCRASESVDNYGNNLMHWYQQKPGQAPRLLIYLASYLE SGVPARFSGSGSRTDFTLTISRLEPEDFATYYCQQNNESPWTFGQGTKVEIK （SEQ ID NO.130） |
| ***26D7-L3c*** |
| EIVLTQSPGTLSLSPGERATISCRASESVDAYGNNLMHWYQQKPGQAPRLLIYLASYLE SGVPARFSGSGSRTDFTLTISRLEPEDFATYYCQQNNESPWTFGQGTKVEIK （SEQ ID NO.131） |
| ***26D7-L4a*** |
| EIVLTQSPGTLSVSPGERATISCRASESVDSYGNNLMHWYQQKPGQAPRLLIYLASYLE SGVPARFSGSGSRTDFTLTISRVEPEDFATYYCQQNNESPWTFGQGTKLEIK （SEQ ID NO.132） |
| ***26D7-L5a*** |
| DIQMTQSPSSLSASVGDRVTITCRASESVESYGNNLMHWYQQKPGKAPKLLIYLASYL ESGVPSRFSGSGSGTDFTLTISSLQPEDFATYYCQQNNESPWTFGGGTKLEIK （SEQ ID NO.133） |
| ***26D7-L5b*** |
| DIQLTQSPSSLSASVGDRATITCRASESVDSYGNNLMHWYQQKPGKAPKLLIYLASYL ESGVPSRFSGSGSGTDFTLTISSLQPEDFATYYCQQNNESPWTFGGGTKLEIK （SEQ ID NO.134） |
| ***26D7-L5c*** |
| DIVLTQSPASLAVSPGQRATITCRASESVDSYGNNLMHWYQQKPGQPPKLLIYLASYL ESGVPARFSGSGSGTDFTLTINPVEANDTANYYCQQNNESPWTFGGGTKLEIK （SEQ ID NO.135） |

| |
|---|
| **Note: The underlined parts show the CDRs** |

The humanized heavy and light chains described above were combined into humanized antibodies, which were tested for their IC₅₀ values at the cellular level as follows.

**Table 9: Humanization Design of26D7**

| | Humanization Design of26D7 | |
|---|---|---|
| Clone ID | Heavy chain | Light chain |
| 26D7H1aL1a | H1a ( SEQ ID NO.98 ) | L1a ( SEQ ID NO.122 ) |
| 26D7H1aL2a | H1a ( SEQ ID NO.98 ) | L2a ( SEQ ID NO.124 ) |
| 26D7H1bL2a | H1b ( SEQ ID NO.100 ) | L2a ( SEQ ID NO.124 ) |
| 26D7H2aL2a | H2a ( SEQ ID NO.101 ) | L2a ( SEQ ID NO.124 ) |
| 26D7H2bL2a | H2b ( SEQ ID NO.102 ) | L2a ( SEQ ID NO.124 ) |
| 26D7H2cL2a | H2c ( SEQ ID NO.103 ) | L2a ( SEQ ID NO.124 ) |
| 26D7H2dL2a | H2d ( SEQ ID NO.104 ) | L2a (SEQ ID NO.124 ) |
| 26D7H3aL2a | H3a ( SEQ ID NO.105 ) | L2a (SEQ ID NO.124 ) |
| 26D7H3hL2a | H3h ( SEQ ID NO.112 ) | L2a (SEQ ID NO.124 ) |
| 26D7H3iL2a | H3i ( SEQ ID NO.113 ) | L2a (SEQ ID NO.124 ) |
| 26D7H3aL2b | H3a ( SEQ ID NO.105 ) | L2b ( SEQ ID NO.125 ) |
| 26D7H3aL2c | H3a ( SEQ ID NO.105 ) | L2c ( SEQ ID NO.127 ) |
| 26D7H1bL3a | H1b ( SEQ ID NO.100 ) | L3a ( SEQ ID NO.129 ) |
| 26D7H2aL3a | H2a ( SEQ ID NO.101 ) | L3a ( SEQ ID NO.129 ) |
| 26D7H3aL3a | H3a ( SEQ ID NO.105 ) | L3a ( SEQ ID NO.129 ) |
| 26D7H3bL3a | H3b ( SEQ ID NO.106 ) | L3a ( SEQ ID NO.129 ) |
| 26D7H3cL3a | H3c ( SEQ ID NO.107 ) | L3a ( SEQ ID NO.129 ) |
| 26D7H3dL3a | H3d ( SEQ ID NO.108 ) | L3a ( SEQ ID NO.129 ) |
| 26D7H3eL3a | H3e ( SEQ ID NO.109 ) | L3a ( SEQ ID NO.129 ) |
| 26D7H3fL3a | H3f ( SEQ ID NO.110 ) | L3a ( SEQ ID NO.129 ) |
| 26D7H3gL3a | H3g ( SEQ ID NO.111 ) | L3a ( SEQ ID NO.129 ) |
| 26D7H3aL3b | H3a ( SEQ ID NO.105 ) | L3b ( SEQ ID NO.130 ) |
| 26D7H3aL3c | H3a ( SEQ ID NO.105 ) | L3c ( SEQ ID NO.131 ) |
| 26D7H1bL4a | H1b ( SEQ ID NO.100 ) | L4a ( SEQ ID NO.132 ) |
| 26D7H4aL4a | H4a ( SEQ ID NO.114 ) | L4a ( SEQ ID NO.132 ) |
| 26D7H5aL5a | H5a ( SEQ ID NO.115 ) | L5a ( SEQ ID NO.133 ) |
| 26D7H5bL5a | H5b ( SEQ ID NO.117 ) | L5a ( SEQ ID NO.133 ) |
| 26D7H5cL5a | H5c ( SEQ ID NO.118 ) | L5a ( SEQ ID NO.133 ) |
| 26D7H5dL5a | H5d ( SEQ ID NO.119) | L5a ( SEQ ID NO.133 ) |
| 26D7H5aL5b | H5a ( SEQ ID NO.115 ) | L5b ( SEQ ID NO.134 ) |
| 26D7H5bL5b | H5b ( SEQ ID NO.117 ) | L5b ( SEQ ID NO.134 ) |
| 26D7H5cL5b | H5c ( SEQ ID NO.118 ) | L5b ( SEQ ID NO.134 ) |
| 26D7H5dL5b | H5d ( SEQ ID NO.119 ) | L5b ( SEQ ID NO.134 ) |
| 26D7H5dL5c | H5d ( SEQ ID NO.119 ) | L5c ( SEQ ID NO.135 ) |
| 26D7H5eL5c | H5e ( SEQ ID NO.120 ) | L5c ( SEQ ID NO.135 ) |
| 26D7H5fL5c | H5f ( SEQ ID NO.121 ) | L5c ( SEQ ID NO.135 ) |

**Table 10: Humanization Design of 2D4**

| | Humanization Design of 2D4 | |
|---|---|---|
| Clone ID | Heavy chain | Light chain |
| 2D4H6aL6a | H6a ( SEQ ID NO.90 ) | L6a ( SEQ ID NO.94 ) |
| 2D4H6bL6a | H6b ( SEQ ID NO.92 ) | L6a ( SEQ ID NO.94 ) |
| 2D4H6aL6b | H6a ( SEQ ID NO.90 ) | L6b ( SEQ ID NO.96 ) |
| 2D4H6bL6b | H6b ( SEQ ID NO.92 ) | L6b ( SEQ ID NO.96 ) |
| 2D4H6aL6c | H6a ( SEQ ID NO.90 ) | L6c ( SEQ ID NO.97 ) |
| 2D4H6cL6c | H6c ( SEQ ID NO.93 ) | L6c ( SEQ ID NO.97 ) |

**Table 11 Cellular Experiments of Humanized Antibodies**

| | IC₅₀ (nM) | |
|---|---|---|
| Clone ID | Human GFRAL/human RET | Mouse GFRAL/mouse RET |
| 26D7H1aL2a | 2.6 | 7.7 |
| 26D7H2aL2a | 7.6 | 1.6 |
| 26D7H2dL2a | 6.5 | 3.0 |
| 26D7H3aL2c | 8.3 | 1.9 |
| 26D7H4aL4a | 5.7 | 0.1 |
| 26D7H5bL5a | 11.8 | 0.9 |
| 26D7H5eL5c | 8.3 | 0.7 |
| 26D7H5fL5c | 8.9 | 0.4 |
| 26D7-mVH+mVL | 8.7 | 0.7 |
| 2D4H6aL6b | 24.4 | 1.4 |
| 2D4H6cL6c | 22.5 | 1.3 |
| 2D4-mVH+mVL | 23.8 | 1.4 |

As shown in the results of Table 11 from the cellular experiments (only data from some of the humanized antibodies are shown), the majority of humanized antibodies have antagonistic activity against GFRAL similar to or better than murine antibodies.

Meanwhile, in order to verify the affinity level of the humanized anti-GFRAL antibodies, the method described in Example 4 was applied to humanized antibodies 26D7H3aL2c (SEQ ID NO.105+SEQ ID NO. 127) and 26D7H5bL5a (SEQ ID NO.117+SEQ ID NO. 133) for binding affinity assays. Specific protocols are as follows.

The surface of the CM5 chip was activated using 400mM EDC and 100mM NHS at a flow rate of 10 µl/min according to the instructions for the amino-coupled kit (Cat. No. BR100050, Cytiva). Then, the murine anti-human Fc antibody was immobilized in the test channel (Fc2) and the reference channel (Fc1) of the CM5 chip. Finally, the chip was blocked with 1 M ethanolamine at a flow rate of 10 µl/min; The humanized antibody (~300RU) diluted to 5 µg/ml was then captured on the assay channel (Fc2), and ligand capture were not carried out on the reference channel. Human, mouse or cynomolgus monkey GFRAL proteins (in a HBS-EP buffer) with 2-fold doubling dilutions was subsequently injected at a flow rate of 30 µL/min and the binding kinetics were evaluated at 25°C. The results are as follows (see Table 12)

**Table 12: Binding Kinetics Data for Humanized Antibodies**

| | Binding affinity | | | | | |
|---|---|---|---|---|---|---|
| Humanized antibodies | Human GFRAL | | Mouse GFRAL | | Cynomolgus monkey GFRAL | |
| Clone ID | KD (M) | K_{off}(1/s) | KD (nM) | K_{off}(1/s) | KD (M) | K_{off} (1/s) |
| 26D7H3aL2c | 4.65E-11 | 1.25E-04 | 1.34E-10 | 2.15E-04 | 1.31E-10 | 1.93E-04 |
| 26D7H5bL5a | 1.19E-10 | 2.68E-04 | 2.58E-10 | 3.14E-04 | 2.46E-10 | 2.31E-04 |

Thus, humanized anti-GFRAL antibodies 26D7H3aL2c and 26D7H5bL5a retained their high affinity to human and mouse GFRAL as murine antibodies, while demonstrating high binding activity to cynomolgus monkey GFRAL.

### EXAMPLE 7 Evaluation of Biological Function of Humanized Anti-GFRAL Antibodies in Animal Model Study

### 7.1 Evaluation of Effect of Humanized Anti-GFRAL Antibodies in Reversing GDF-15-Induced Weight Loss in AAV-mGDF15 Model

In order to further verify the *in vivo* efficacy of humanized anti-GFRAL antibodies, the alleviation effect of humanized antibodies on GDF15-induced weight loss in mice (non-tumor-bearing mice) with the AAV-mGDF15 model was studied with reference to the method described in Example 5.2. Specific protocols are as follows.

After random grouping according to mean body weights, mice were administered a single tail vein injection of 1×10¹⁰vg of rAAV expressing mGDF15 or control AAV. Starting on Day 6 after the rAAV injection, mice in individual groups were weekly given subcutaneous injections of 10 mg/kg doses of control antibody (hIgG4), 10 mg/kg doses of murine anti-GFRAL antibody (e.g., m26D7), or 10 mg/kg, 3 mg/kg, or 1 mg/kg doses of humanized anti-GFRAL antibody (e.g., 26D7H3aL2c, or 26D7H5bL5a), respectively. Body weights were monitored three times a week and food intake was monitored twice a week. The body weights are summarized below, and the data are expressed as the means±SEMs of the overall body weight and the percentage of body weight change (i.e., the percentage of body weight change relative to baseline body weight). In these experiments, P < 0.05 was considered statistically significant.

**Table 13: Improvement effect data of humanized anti-GFRAL antibody on weight loss in AAV-mGDF15 mouse model**

| **Group** | **Pretreatment Day 0** | **Antibody treatment Day 6** | **Body Weight (g)** | | | **Weight change ^{a} (%)** | | ***P^{b}*** |
|---|---|---|---|---|---|---|---|---|
| | | | **Day 0** | **Day 6** | **Day 33** | **Day 6** | **Day 33** | |
| 1 | AAV8- control | HIgG4, 10mg/kg | 26.1±0.9 | 26.9±1.4 | 29.1±1.8 | 3.04±2.87 | 11.4±4.60 | <0.0001 |
| 2 | AAV8-mGDF15 | HIgG4, 10mg/kg | 25.9±0.7 | 24.0±0.9 | 21.2±1.3 | -7.27±2.82 | -16.89±4.98 | |
| 3 | AAV8-mGDF15 | 26D7H3aL2c, 10mg/kg | 26.2±0.5 | 24.0±0.9 | 28.2±1.1 | -8.52±2.89 | 7.31±3.67 | <0.0001 |
| 4 | AAV8-mGDF15 | 26D7H3aL2c, 3mg/kg | 26.4±1.1 | 24.0±0.9 | 28.8±1.5 | -9.00±2.19 | 9.13±4.37 | <0.0001 |
| 5 | AAV8-mGDF15 | 26D7H3aL2c, 1mg/kg | 26.0±0.7 | 24.0±1.0 | 26.8±1.2 | -7.55±3.40 | 3.23±4.41 | <0.0001 |
| 6 | AAV8-mGDF15 | 26D7H5bL5a, 10mg/kg | 26.3±0.9 | 26.0±1.1 | 28.7±1.9 | -8.64±1.59 | 8.89±4.86 | <0.0001 |
| 7 | AAV8-mGDF15 | 26D7H5bL5a, 3mg/kg | 26.3±1.0 | 24.0±1.0 | 27.5±0.8 | -8.74±1.76 | 4.62±3.12 | 0.0095 |
| 8 | AAV8-mGDF15 | 26D7H5bL5a, 1mg/kg | 26.1±0.6 | 24.0±0.9 | 24.8±0.5 | -7.88±2.34 | -4.73±3.94 | 0.0002 |
| 9 | AAV8-mGDF15 | M26D7, 10mg/kg | 25.5±0.3 | 24.0±1.2 | 28.9±1.2 | -6.10±4.19 | 13.30±3.94 | <0.0001 |

| | | | | | | | | |
|---|---|---|---|---|---|---|---|---|
| Note: Data are expressed as means±SEMs; A. body weight change = [(body weight on day i - body weight on day 0)/body weight on day 0]* 100%; B. body weight changes from day 0 to day 33 in different groups were compared and an independent t test was performed. | | | | | | | | |

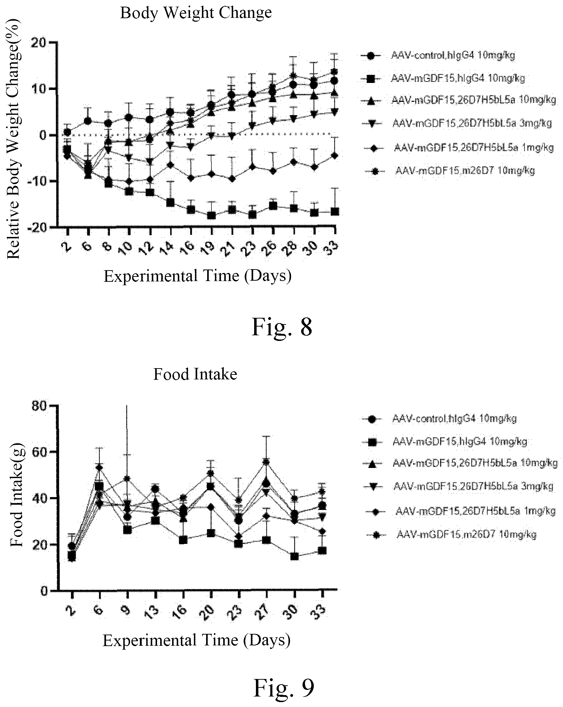

As shown by the experimental results in FIGs. 6-9 and Table 13, similar to the effect of the murine anti-GFRAL antibodies, the humanized anti-GFRAL antibodies were capable of reversing GDF 15-induced weight loss in a dose-dependent manner, effectively increasing the weight of the mice, and increasing the food intake of the mice. Both humanized antibodies 26D7H3aL2c and 26D7H5bL5a achieved the effect of reversing body weight loss, and 26D7H3aL2c performed better. This experimental result further confirms that the humanized anti-GFRAL antibodies retain the *in vivo* efficacy in the AAV-mGDF15 mouse model as murine antibodies, and are effective in improving cachexia symptoms induced by GDF-15.

### 7.2 Evaluation of Effect of Humanized Anti-GFRAL Antibodies in Amelioration of Cachexia Symptoms in Human Fibrosarcoma HT-1080 Tumor-Bearing Mouse Model

To further validate the *in vivo* efficacy of humanized anti-GFRAL antibodies, the alleviation effect of the humanized antibodies on GDF15-induced weight loss in mice was investigated using a human fibrosarcoma HT-1080 subcutaneous xenograft BALB/c nude female mouse model as described in Example 5.1. Specific protocols are as follows.

HT-1080 cells in the exponential growth phase were collected for subcutaneous tumor inoculation in nude mice, and a mouse subcutaneous xenograft tumor model was established. When the average tumor volume reached about 110.51 mm³, the mice were randomly divided into 4 groups according to the weight of the mice and the tumor size. Another group of non-tumor-bearing mice were involved. When the administration began, the experimental groups included three 26D7H3aL2c dosing groups (1 mg/kg, 3 mg/kg and 10 mg/kg), a hIgG4 dosing group (10 mg/kg) of tumor-bearing control mice, and a hIgG4 dosing group (10 mg/kg) of non-tumor-bearing control mice. Each group included eight mice, and each mouse was weekly administered by subcutaneous injection for 3 weeks. Daily body weight and food intake were recorded. Measured body weight data were recorded using Study Director^{™} software, and statistical analysis and plotting of overall body weight change and net body weight change were performed in GraphPad Prism 8 using a two-factor ANOVA Dunnett multiple comparison test where p values less than 0.05 were considered statistically significant.

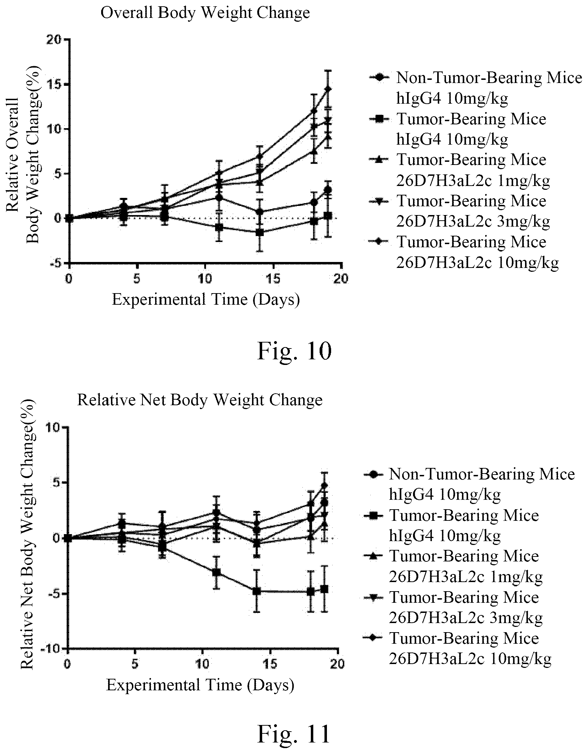

The results (see FIGs. 10, 11 and Tables 14, 15) show that, humanized anti-GFRAL antibody 26D7H3aL2c significantly alleviated the weight loss caused by cachexia in a human fibrosarcoma HT-1080 subcutaneous xenograft model at doses of 1, 3 and 10 mg/kg, and there were dose-dependent pharmacodynamic observations for 26D7H3aL2c.

**Table 14. Changes of Overall Body Weight in Human Fibrosarcoma HT-1080 Tumor-Bearing Model**

| **Group** | **Administration group** | **Dose (mg/kg)** | **Animal number (Experiment start/experiment end)** | **Mean body weight/g (MEANt SEM)** | | **Mean change rate of overall body weight (%) (MEAN±SEM)** | *P Value (vs control)* |
|---|---|---|---|---|---|---|---|
| | | | | **Day 0** | **Day 19** | **Day 19** | **Day 19** |
| **1** | **Non-tumor-bearing mouse** | 10 | 8/8 | 23.98 ± 0.37 | 24.73 ± 0.27 | 3.20±0.96 | 0.3618 |
| | **hIgG4** | | | | | | |
| **2** | **Tumor-bearing mouse** | 10 | 8/8 | 22.84 ± 0.69 | 22.83 ± 0.53 | 0.29±2.37 | **-** |
| | **hIgG4** | | | | | | |
| **3** | **Tumor-bearing mouse** | 1 | 8/8 | 22.84 ± 0.65 | 24.89 ± 0.46 | 9.22±1.35 | **<0.0001** |
| | **26D7H3aL2c** | | | | | | |
| **4** | **Tumor-bearing mouse** | 3 | 8/8 | 22.84 ± 0.54 | 25.31 ± 0.55 | 10.92±1.30 | **<0.0001** |
| | **26D7H3aL2c** | | | | | | |
| **5** | **Tumor-bearing mouse** | 10 | 8/8 | 22.84 ± 0.49 | 26.13 ± 0.59 | 14.50±2.03 | **<0.0001** |
| | **26D7H3aL2c** | | | | | | |

| | | | | | | | |
|---|---|---|---|---|---|---|---|
| Note: Data are expressed as "mean ± standard error"; Groups 1, 3, 4, 5 were compared with Group 2 using a two-factor ANOVA Dunnett multiple comparison test; ****p<0.0001 | | | | | | | |

**Table 15. Changes of Net Body Weight in Human Fibrosarcoma Ht-1080 Tumor-Bearing Model**

| **Group** | **Administration group** | **Dose (mg/kg)** | **Number of animals (start of experiment/end of experiment)** | **Mean body weight (net)/g (MEAN ± SEM)** | | **Weight (Net) Mean Rate of Change (%) (MEAN±SEM)** | *P value (vs control)* |
|---|---|---|---|---|---|---|---|
| | | | | **Day 0** | **Day 19** | **Day 19** | **Day 19** |
| **1** | **Non-tumor bearing mouse hIgG4** | 10 | 8/8 | 23.98 ± 0.37 | 24.73 ± 0.27 | 3.20±0.96 | **<0.0001** |
| **2** | **Tumor-bearing mouse hIgG4** | 10 | 8/8 | 22.73 ± 0.69 | 21.63 ± 0.55 | -4.57± - 2.07 | **-** |
| **3** | **Tumor-bearing mouse 26D7H3aL2c** | 1 | 8/8 | 22.74 ± 0.65 | 23.01 ± 0.63 | 1.39±1.67 | **0.0024** |
| **4** | **Tumor-bearing mouse 26D7H3aL2c** | 3 | 8/8 | 22.74 ± 0.54 | 23.18 ± 0.54 | 2.03±1.26 | **0.0006** |
| **5** | **Tumor-bearing mouse 26D7H3aL2c** | 10 | 8/8 | 22.74 ± 0.49 | 23.81 ± 0.51 | 4.76±1.14 | **<0.0001** |

| | | | | | | | |
|---|---|---|---|---|---|---|---|
| Note: Net mouse body weight = mouse body weight - mouse tumor weight, in which the mouse tumor weigh estimation method [1] was 1g/1000mm³. Data are expressed as "mean ± standard error"; Groups 1, 3, 4 and 5 were compared to Group 2 using a two factors ANOVADunnett multiple comparison test; **p<0.01, ***p<0.001, ****p<0.0001 | | | | | | | |

Taking the above experimental data analysis together, the anti-GFRAL antibody reversed the weight loss in cachexia symptoms in mice in both the HT-1080 tumor-bearing mouse model and the AAV-mGDF15 non-tumor-bearing mouse model, demonstrating that tumor-induced weight loss in mice in the HT-1080 tumor-bearing mouse model is directly related to the GDF15/GFRAL signaling pathway. Anti-GFRAL antibodies significantly improved tumor-induced weight loss in mice by blocking the GDF15/GFRAL signaling pathway. At the same time, the experimental results further confirm that the humanized anti-GFRAL antibody retains the high binding affinity to human GFRAL and mouse GFRAL and the *in vivo* drug efficacy as the murine antibody (based on the experimental results from the HT-1080 tumor-bearing mouse model and the AAV-mGDF15 mouse model), effectively alleviates or even reverses the cachexia symptoms induced by GDF-15, and achieves unexpected technical effects.

### REFERENCES

1. Rochette L, Zeller M, Cottin Y, Vergely C. Insights Into Mechanisms of GDF15 and Receptor GFRAL: Therapeutic Targets. Trends Endocrinol Metab. 2020 Dec;31 (12) :939-951.
2. Yang, L. et al. (2017) GFRAL is the receptor for GDF15 and is required for the anti-obesity effects of the ligand. Nat. Med. 23, 1158-1166
3. Mullican, S.E. et al. (2017) GFRAL is the receptor for GDF15 and the ligand promotes weight loss in mice and nonhuman primates. Nat. Med. 23, 1150-1157
4. Hsu J-Y, Crawley S, Chen M, Ayupova DA, Lindhout DA, Higbee J, et al. Non-homeostatic body weight regulation through a brainstem-restricted receptor for GDF15. Nature. 2017;550 (7675) :255-9.
5. Roeland EJ, Bohlke K, Baracos VE, Bruera E, Del Fabbro E, Dixon S, et al. Management of cancer cachexia: ASCO guideline. J Clin Oncol 2020; 38: 2438- 2453.

## Claims

1. An anti-GFRAL antibody or an antigen-binding portion thereof comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO. 116 or has at least 80%, 85%, 90% or 95% identity to the sequence as set forth in SEQ ID NO.76 or SEQ ID NO.116, the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99, the sequence of light chain CDR1 is the sequence as set forth in SEQ ID NO.79 or SEQ ID NO. 126 or SEQ ID NO. 128 or has at least 80%, 85%, 90% or 95% identity to the sequence as set forth in SEQ ID NO.79 or SEQ ID NO. 126 or SEQ ID NO.128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO.123 or has at least 80%, 85%, 90% or 95% identity to the sequence as set forth in SEQ ID NO.50 or SEQ ID NO. 123; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, the sequence of the heavy chain CDR3 is the sequence as set forth in SEQ ID NO.70 or has at least 80%, 85%, 90% or 95% identity to the sequence as set forth in SEQ ID NO.70, the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, the sequence of the heavy chain CDR3 is the sequence as set forth in SEQ ID NO.3 or has at least 80%, 85%, 90% or 95% identity to the sequence as set forth in SEQ ID NO.3, the sequence of the light chain CDR1 is the sequence as set forth in SEQ ID NO.5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO.10 or has at least 80%, 85%, 90% or 95% identity to the sequence as set forth in SEQ ID NO.10, the sequence of the heavy chain CDR3 is the sequence as set forth in SEQ ID NO.11 or has at least 80%, 85%, 90% or 95% identity to the sequence as set forth in SEQ ID NO. 11, the sequence of the light chain CDR1 is the sequence as set forth in SEQ ID NO.13 or has at least 80%, 85%, 90% or 95% identity to the sequence as set forth in SEQ ID NO.13, the sequence of the light chain CDR2 is the sequence as set forth in SEQ ID NO.14 or has at least 80%, 85%, 90% or 95% identity to the sequence as set forth in SEQ ID NO.14, and the sequence of the light chain CDR3 is the sequence as set forth in SEQ ID NO.15 or has at least 80%, 85%, 90% or 95% identity to the sequence as set forth in SEQ ID NO.15; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, the sequence of the heavy chain CDR3 is the sequence as set forth in SEQ ID NO.19 or has at least 80%, 85%, 90% or 95% identity to the sequence as set forth in SEQ ID NO.19, the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, the sequence of the heavy chain CDR3 is the sequence as set forth in SEQ ID NO.26 or has at least 80%, 85%, 90% or 95% identity to the sequence as set forth in SEQ ID NO.26, the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, the sequence of the heavy chain CDR3 is the sequence as set forth in SEQ ID NO.33 or has at least 80%, 85%, 90% or 95% identity to the sequence as set forth in SEQ ID NO.33, the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, the sequence of the heavy chain CDR3 is the sequence as set forth in SEQ ID NO.41 or has at least 80%, 85%, 90% or 95% identity to the sequence as set forth in SEQ ID NO.41, the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, the sequence of the heavy chain CDR3 is the sequence as set forth in SEQ ID NO.47 or has at least 80%, 85%, 90% or 95% identity to the sequence as set forth in SEQ ID NO.47, the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, the sequence of the heavy chain CDR3 is the sequence as set forth in SEQ ID NO.54 or has at least 80%, 85%, 90% or 95% identity to the sequence as set forth in SEQ ID NO.54, the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, the sequence of the heavy chain CDR3 is the sequence as set forth in SEQ ID NO.62 or has at least 80%, 85%, 90% or 95% identity to the sequence as set forth in SEQ ID NO.62, the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, the sequence of the heavy chain CDR3 is the sequence as set forth in SEQ ID NO.84 or has at least 80%, 85%, 90% or 95% identity to the sequence as set forth in SEQ ID NO.84, the sequence of the light chain CDR1 is the sequence as set forth in SEQ ID NO.86 or has at least 80%, 85%, 90% or 95% identity to the sequence as set forth in SEQ ID NO.86, the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the heavy chain CDR1 to 3 and the light chain CDR1 to 3 are defined according to the IMGT definition.

2. The anti-GFRAL antibody or antigen-binding portion thereof of claim 1, wherein the heavy chain variable region has the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121, or has an amino acid sequence having at least 80%, at least 90%, at least 95% or at least 99% identity to the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121.

3. The anti-GFRAL antibody or antigen-binding portion thereof of claim 1 or 2, wherein the light chain variable region has the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134 or 135, or has an amino acid sequence having at least 80%, at least 90%, at least 95% or at least 99% identity to the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134, or 135.

4. The anti-GFRAL antibody or antigen-binding portion thereof of any one of claims 1 to 3, wherein:
(1) the sequence of the heavy chain variable region is the sequence as set forth in SEQ ID NO.4, and the sequence of the light chain variable region is the sequence as set forth in SEQ ID NO.8;
(2) the sequence of the heavy chain variable region is the sequence as set forth in SEQ ID NO.12, and the sequence of the light chain variable region is the sequence as set forth in SEQ ID NO.16;
(3) the sequence of the heavy chain variable region is the sequence as set forth in SEQ ID NO.20, and the sequence of the light chain variable region is the sequence as set forth in SEQ ID NO.23;
(4) the sequence of the heavy chain variable region is the sequence as set forth in SEQ ID NO.27, and the sequence of the light chain variable region is the sequence as set forth in SEQ ID NO.30;
(5) the sequence of the heavy chain variable region is the sequence as set forth in SEQ ID NO.34, and the sequence of the light chain variable region is the sequence as set forth in SEQ ID NO.38;
(6) the sequence of the heavy chain variable region is the sequence as set forth in SEQ ID NO.42, and the sequence of the light chain variable region is the sequence as set forth in SEQ ID NO.44;
(7) the sequence of the heavy chain variable region is the sequence as set forth in SEQ ID NO.48, and the sequence of the light chain variable region is the sequence as set forth in SEQ ID NO.51;
(8) the sequence of the heavy chain variable region is the sequence as set forth in SEQ ID NO.52, and the sequence of the light chain variable region is the sequence as set forth in SEQ ID NO.51;
(9) the sequence of the heavy chain variable region is the sequence as set forth in SEQ ID NO.55, and the sequence of the light chain variable region is the sequence as set forth in SEQ ID NO.59;
(10) the sequence of the heavy chain variable region is the sequence as set forth in SEQ ID NO.63, and the sequence of the light chain variable region is the sequence as set forth in SEQ ID NO.67;
(11) the sequence of the heavy chain variable region is the sequence as set forth in SEQ ID NO.71, and the sequence of the light chain variable region is the sequence as set forth in SEQ ID NO.74;
(12) the sequence of the heavy chain variable region is the sequence as set forth in SEQ ID NO.78, and the sequence of the light chain variable region is the sequence as set forth in SEQ ID NO.81;
(13) the sequence of the heavy chain variable region is the sequence as set forth in SEQ ID NO.85, and the sequence of the light chain variable region is the sequence as set forth in SEQ ID NO.89;
(14) the sequence of the heavy chain variable region is the sequence as set forth in SEQ ID NO.98, and the sequence of the light chain variable region is the sequence as set forth in SEQ ID NO. 122;
(15) the sequence of the heavy chain variable region is the sequence as set forth in SEQ ID NO.98, and the sequence of the light chain variable region is the sequence as set forth in SEQ ID NO. 124;
(16) the sequence of the heavy chain variable region is the sequence as set forth in SEQ ID NO. 100, and the sequence of the light chain variable region is the sequence as set forth in SEQ ID NO. 124;
(17) the sequence of the heavy chain variable region is the sequence as set forth in SEQ ID NO. 101, and the sequence of the light chain variable region is the sequence as set forth in SEQ ID NO. 124;
(18) the sequence of the heavy chain variable region is the sequence as set forth in SEQ ID NO. 102, and the sequence of the light chain variable region is the sequence as set forth in SEQ ID NO. 124;
(19) the sequence of the heavy chain variable region is the sequence as set forth in SEQ ID NO. 103, and the sequence of the light chain variable region is the sequence as set forth in SEQ ID NO. 124;
(20) the sequence of the heavy chain variable region is the sequence as set forth in SEQ ID NO. 104, and the sequence of the light chain variable region is the sequence as set forth in SEQ ID NO. 124;
(21) the sequence of the heavy chain variable region is the sequence as set forth in SEQ ID NO. 105, and the sequence of the light chain variable region is the sequence as set forth in SEQ ID NO. 124;
(22) the sequence of the heavy chain variable region is the sequence as set forth in SEQ ID NO. 112, and the sequence of the light chain variable region is the sequence as set forth in SEQ ID NO. 124;
(23) the sequence of the heavy chain variable region is the sequence as set forth in SEQ ID NO. 113, and the sequence of the light chain variable region is the sequence as set forth in SEQ ID NO. 124;
(24) the sequence of the heavy chain variable region is the sequence as set forth in SEQ ID NO. 105, and the sequence of the light chain variable region is the sequence as set forth in SEQ ID NO. 125;
(25) the sequence of the heavy chain variable region is the sequence as set forth in SEQ ID NO. 105, and the sequence of the light chain variable region is the sequence as set forth in SEQ ID NO. 127;
(26) the sequence of the heavy chain variable region is the sequence as set forth in SEQ ID NO. 100, and the sequence of the light chain variable region is the sequence as set forth in SEQ ID NO. 129;
(27) the sequence of the heavy chain variable region is the sequence as set forth in SEQ ID NO. 101, and the sequence of the light chain variable region is the sequence as set forth in SEQ ID NO. 129;
(28) the sequence of the heavy chain variable region is the sequence as set forth in SEQ ID NO. 105, and the sequence of the light chain variable region is the sequence as set forth in SEQ ID NO. 129;
(29) the sequence of the heavy chain variable region is the sequence as set forth in SEQ ID NO. 106, and the sequence of the light chain variable region is the sequence as set forth in SEQ ID NO. 129;
(30) the sequence of the heavy chain variable region is the sequence as set forth in SEQ ID NO. 107, and the sequence of the light chain variable region is the sequence as set forth in SEQ ID NO. 129;
(31) the sequence of the heavy chain variable region is the sequence as set forth in SEQ ID NO. 108, and the sequence of the light chain variable region is the sequence as set forth in SEQ ID NO. 129;
(32) the sequence of the heavy chain variable region is the sequence as set forth in SEQ ID NO. 109, and the sequence of the light chain variable region is the sequence as set forth in SEQ ID NO. 129;
(33) the sequence of the heavy chain variable region is the sequence as set forth in SEQ ID NO. 110, and the sequence of the light chain variable region is the sequence as set forth in SEQ ID NO. 129;
(34) the sequence of the heavy chain variable region is the sequence as set forth in SEQ ID NO. 111, and the sequence of the light chain variable region is the sequence as set forth in SEQ ID NO. 129;
(35) the sequence of the heavy chain variable region is the sequence as set forth in SEQ ID NO. 105, and the sequence of the light chain variable region is the sequence as set forth in SEQ ID NO.130;
(36) the sequence of the heavy chain variable region is the sequence as set forth in SEQ ID NO. 105, and the sequence of the light chain variable region is the sequence as set forth in SEQ ID NO.131;
(37) the sequence of the heavy chain variable region is the sequence as set forth in SEQ ID NO. 100, and the sequence of the light chain variable region is the sequence as set forth in SEQ ID NO. 132;
(38) the sequence of the heavy chain variable region is the sequence as set forth in SEQ ID NO. 114, and the sequence of the light chain variable region is the sequence as set forth in SEQ ID NO. 132;
(39) the sequence of the heavy chain variable region is the sequence as set forth in SEQ ID NO. 115, and the sequence of the light chain variable region is the sequence as set forth in SEQ ID NO.133;
(40) the sequence of the heavy chain variable region is the sequence as set forth in SEQ ID NO. 117, and the sequence of the light chain variable region is the sequence as set forth in SEQ ID NO.133;
(41) the sequence of the heavy chain variable region is the sequence as set forth in SEQ ID NO. 118, and the sequence of the light chain variable region is the sequence as set forth in SEQ ID NO.133;
(42) the sequence of the heavy chain variable region is the sequence as set forth in SEQ ID NO. 119, and the sequence of the light chain variable region is the sequence as set forth in SEQ ID NO.133;
(43) the sequence of the heavy chain variable region is the sequence as set forth in SEQ ID NO. 115, and the sequence of the light chain variable region is the sequence as set forth in SEQ ID NO. 134;
(44) the sequence of the heavy chain variable region is the sequence as set forth in SEQ ID NO. 117, and the sequence of the light chain variable region is the sequence as set forth in SEQ ID NO. 134;
(45) the sequence of the heavy chain variable region is the sequence as set forth in SEQ ID NO. 118, and the sequence of the light chain variable region is the sequence as set forth in SEQ ID NO. 134;
(46) the sequence of the heavy chain variable region is the sequence as set forth in SEQ ID NO. 119, and the sequence of the light chain variable region is the sequence as set forth in SEQ ID NO. 134;
(47) the sequence of the heavy chain variable region is the sequence as set forth in SEQ ID NO. 119, and the sequence of the light chain variable region is the sequence as set forth in SEQ ID NO.135;
(48) the sequence of the heavy chain variable region is the sequence as set forth in SEQ ID NO. 120, and the sequence of the light chain variable region is the sequence as set forth in SEQ ID NO.135;
(49) the sequence of the heavy chain variable region is the sequence as set forth in SEQ ID NO. 121, and the sequence of the light chain variable region is the sequence as set forth in SEQ ID NO.135;
(50) the sequence of the heavy chain variable region is the sequence as set forth in SEQ ID NO.90, and the sequence of the light chain variable region is the sequence as set forth in SEQ ID NO.94;
(51) the sequence of the heavy chain variable region is the sequence as set forth in SEQ ID NO.92, and the sequence of the light chain variable region is the sequence as set forth in SEQ ID NO.94;
(52) the sequence of the heavy chain variable region is the sequence as set forth in SEQ ID NO.90, and the sequence of the light chain variable region is the sequence as set forth in SEQ ID NO.96;
(53) the sequence of the heavy chain variable region is the sequence as set forth in SEQ ID NO.92, and the sequence of the light chain variable region is the sequence as set forth in SEQ ID NO.96;
(54) the sequence of the heavy chain variable region is the sequence as set forth in SEQ ID NO.90, and the sequence of the light chain variable region is the sequence as set forth in SEQ ID NO.97; or
(55) the sequence of the heavy chain variable region is the sequence as set forth in SEQ ID NO.93, and the sequence of the light chain variable region is the sequence as set forth in SEQ ID NO.97.

5. The anti-GFRAL antibody or antigen-binding portion thereof of any one of claims 1 to 4, wherein the sequence of the heavy chain CDR1 is the sequence as set forth in SEQ ID NO.75, the sequence of the heavy chain CDR2 is the sequence as set forth in SEQ ID NO.76, the sequence of the heavy chain CDR3 is the sequence as set forth in SEQ ID NO.99, the sequence of the light chain CDR1 is the sequence as set forth in SEQ ID NO.128, the sequence of the light chain CDR2 is the sequence as set forth in SEQ ID NO.80, and the sequence of the light chain CDR3 is the sequence as set forth in SEQ ID NO. 123;
preferably, the sequence of the heavy chain variable region is the sequence as set forth in SEQ ID NO.105, and the sequence of the light chain variable region is the sequence as set forth in SEQ ID NO.127;
further preferably, the anti-GFRAL antibody or antigen-binding portion thereof comprises a human IgG1, IgG2 or IgG4 heavy chain constant region and a human kappa light chain constant region or lamda light chain constant region; and
most preferably, the anti-GFRAL antibody or antigen-binding portion thereof comprises a human IgG4 heavy chain constant region and a human kappa light chain constant region.

6. An anti-GFRAL antibody or an antigen-binding portion thereof, comprising a heavy chain variable region and a light chain variable region, wherein:
the heavy chain variable region has the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121, or has an amino acid sequence having at least 80%, at least 90%, at least 95% or at least 99% identity to the amino acid sequence as set forth in SEQ ID NO.4, 12, 20, 27, 34, 42, 48, 52, 55, 63, 71, 78, 85, 90, 92, 93, 98, 100, 101, 102, 103, 104, 105, 106, 107, 108, 109, 110, 111, 112, 113, 114, 115, 117, 118, 119, 120, or 121; and/or
the light chain variable region has the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134 or 135, or has an amino acid sequence having at least 80%, at least 90%, at least 95% or at least 99% identity to the amino acid sequence as set forth in SEQ ID NO.8, 16, 23, 30, 38, 44, 51, 59, 67, 74, 81, 89, 94, 96, 97, 122, 124, 125, 127, 129, 130, 131, 132, 133, 134, or 135.

7. An anti-GFRAL antibody or an antigen-binding portion thereof, comprising a heavy chain variable region, wherein the heavy chain variable region comprises a heavy chain CDR1 (HCDR1), a heavy chain CDR2 (HCDR2), and a heavy chain CDR3 (HCDR3), wherein:
(1) the sequence of the heavy chain CDR1 is the sequence as set forth in SEQ ID NO.75 or has at least 80%, 85%, 90% or 95% identity to the sequence as set forth in SEQ ID NO.75, the sequence of the heavy chain CDR2 is the sequence as set forth in SEQ ID NO.76 or SEQ ID NO.116 or has at least 80%, 85%, 90% or 95% identity to the sequence as set forth in SEQ ID NO.76 or SEQ ID NO.116, and the sequence of the heavy chain CDR3 is the sequence as set forth in SEQ ID NO.77 or SEQ ID NO.99 or has at least 80%, 85%, 90% or 95% identity to the sequence as set forth in SEQ ID NO.77 or SEQ ID NO.99; or
(2) the sequence of the heavy chain CDR1 is the sequence as set forth in SEQ ID NO.68 or has at least 80%, 85%, 90% or 95% identity to the sequence as set forth in SEQ ID NO.68, the sequence of the heavy chain CDR2 is the sequence as set forth in SEQ ID NO.69 or SEQ ID NO.91 or has at least 80%, 85%, 90% or 95% identity to the sequence as set forth in SEQ ID NO.69 or SEQ ID NO.91, and the sequence of the heavy chain CDR3 is the sequence as set forth in SEQ ID NO.70 or has at least 80%, 85%, 90% or 95% identity to the sequence as set forth in SEQ ID NO.70; or
(3) the sequence of the heavy chain CDR1 is the sequence as set forth in SEQ ID NO.1 or has at least 80%, 85%, 90% or 95% identity to the sequence as set forth in SEQ ID NO.1, the sequence of the heavy chain CDR2 is the sequence as set forth in SEQ ID NO.2 or has at least 80%, 85%, 90% or 95% identity to the sequence as set forth in SEQ ID NO.2, and the sequence of the heavy chain CDR3 is the sequence as set forth in SEQ ID NO.3 or has at least 80%, 85%, 90% or 95% identity to the sequence as set forth in SEQ ID NO.3; or
(4) the sequence of the heavy chain CDR1 is the sequence as set forth in SEQ ID NO.9 or has at least 80%, 85%, 90% or 95% identity to the sequence as set forth in SEQ ID NO.9, the sequence of the heavy chain CDR2 is the sequence as set forth in SEQ ID NO.10 or has at least 80%, 85%, 90% or 95% identity to the sequence as set forth in SEQ ID NO.10, and the sequence of the heavy chain CDR3 is the sequence as set forth in SEQ ID NO.11 or has at least 80%, 85%, 90% or 95% identity to the sequence as set forth in SEQ ID NO.11; or
(5) the sequence of the heavy chain CDR1 is the sequence as set forth in SEQ ID NO.17 or has at least 80%, 85%, 90% or 95% identity to the sequence as set forth in SEQ ID NO.17, the sequence of the heavy chain CDR2 is the sequence as set forth in SEQ ID NO.18 or has at least 80%, 85%, 90% or 95% identity to the sequence as set forth in SEQ ID NO.18, and the sequence of the heavy chain CDR3 is the sequence as set forth in SEQ ID NO.19 or has at least 80%, 85%, 90% or 95% identity to the sequence as set forth in SEQ ID NO.19; or
(6) the sequence of the heavy chain CDR1 is the sequence as set forth in SEQ ID NO.24 or has at least 80%, 85%, 90% or 95% identity to the sequence as set forth in SEQ ID NO.24, the sequence of the heavy chain CDR2 is the sequence as set forth in SEQ ID NO.25 or has at least 80%, 85%, 90% or 95% identity to the sequence as set forth in SEQ ID NO.25, and the sequence of the heavy chain CDR3 is the sequence as set forth in SEQ ID NO.26 or has at least 80%, 85%, 90% or 95% identity to the sequence as set forth in SEQ ID NO.26; or
(7) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.32 or has at least 80%, 85%, 90% or 95% identity to the sequence as set forth in SEQ ID NO.32, and the sequence of the heavy chain CDR3 is the sequence as set forth in SEQ ID NO.33 or has at least 80%, 85%, 90% or 95% identity to the sequence as set forth in SEQ ID NO.33; or
(8) the sequence of the heavy chain CDR1 is the sequence as set forth in SEQ ID NO.39 or has at least 80%, 85%, 90% or 95% identity to the sequence as set forth in SEQ ID NO.39, the sequence of the heavy chain CDR2 is the sequence as set forth in SEQ ID NO.40 or has at least 80%, 85%, 90% or 95% identity to the sequence as set forth in SEQ ID NO.40, and the sequence of the heavy chain CDR3 is the sequence as set forth in SEQ ID NO.41 or has at least 80%, 85%, 90% or 95% identity to the sequence as set forth in SEQ ID NO.41; or
(9) the sequence of the heavy chain CDR1 is the sequence as set forth in SEQ ID NO.45 or has at least 80%, 85%, 90% or 95% identity to the sequence as set forth in SEQ ID NO.45, the sequence of the heavy chain CDR2 is the sequence as set forth in SEQ ID NO.46 or has at least 80%, 85%, 90% or 95% identity to the sequence as set forth in SEQ ID NO.46, and the sequence of the heavy chain CDR3 is the sequence as set forth in SEQ ID NO.47 or has at least 80%, 85%, 90% or 95% identity to the sequence as set forth in SEQ ID NO.47; or
(10) the sequence of the heavy chain CDR1 is the sequence as set forth in SEQ ID NO.31 or has at least 80%, 85%, 90% or 95% identity to the sequence as set forth in SEQ ID NO.31, the sequence of the heavy chain CDR2 is the sequence as set forth in SEQ ID NO.53 or has at least 80%, 85%, 90% or 95% identity to the sequence as set forth in SEQ ID NO.53, and the sequence of the heavy chain CDR3 is the sequence as set forth in SEQ ID NO.54 or has at least 80%, 85%, 90% or 95% identity to the sequence as set forth in SEQ ID NO.54; or
(11) the sequence of the heavy chain CDR1 is the sequence as set forth in SEQ ID NO.60 or has at least 80%, 85%, 90% or 95% identity to the sequence as set forth in SEQ ID NO.60, the sequence of the heavy chain CDR2 is the sequence as set forth in SEQ ID NO.61 or has at least 80%, 85%, 90% or 95% identity to the sequence as set forth in SEQ ID NO.61, and the sequence of the heavy chain CDR3 is the sequence as set forth in SEQ ID NO.62 or has at least 80%, 85%, 90% or 95% identity to the sequence as set forth in SEQ ID NO.62; or
(12) the sequence of the heavy chain CDR1 is the sequence as set forth in SEQ ID NO.82 or has at least 80%, 85%, 90% or 95% identity to the sequence as set forth in SEQ ID NO.82, the sequence of the heavy chain CDR2 is the sequence as set forth in SEQ ID NO.83 or has at least 80%, 85%, 90% or 95% identity to the sequence as set forth in SEQ ID NO.83, and the sequence of the heavy chain CDR3 is the sequence as set forth in SEQ ID NO.84 or has at least 80%, 85%, 90% or 95% identity to the sequence as set forth in SEQ ID NO.84;
wherein the amino acid sequences of the heavy chain CDR1 to 3 are defined according to the IMGT definition.

8. An anti-GFRAL antibody or an antigen-binding portion thereof, comprising a light chain variable region, wherein the light chain variable region comprises a light chain CDR1 (HCDR1), a light chain CDR2 (HCDR2), and a light chain CDR3 (HCDR3), wherein:
(1) the sequence of the light chain CDR1 is the sequence as set forth in SEQ ID NO.79 or SEQ ID NO.126 or SEQ ID NO.128 or has at least 80%, 85%, 90% or 95% identity to the sequence as set forth in SEQ ID NO.79 or SEQ ID NO.126 or SEQ ID NO.128, the sequence of the light chain CDR2 is the sequence as set forth in SEQ ID NO.80 or has at least 80%, 85%, 90% or 95% identity to the sequence as set forth in SEQ ID NO.80, and the sequence of the light chain CDR3 is the sequence as set forth in SEQ ID NO.50 or SEQ ID NO.123 or has at least 80%, 85%, 90% or 95% identity to the sequence as set forth in SEQ ID NO.50 or SEQ ID NO.123; or
(2) the sequence of the light chain CDR1 is the sequence as set forth in SEQ ID NO.72 or SEQ ID NO.95 or has at least 80%, 85%, 90% or 95% identity to the sequence as set forth in SEQ ID NO.72 or SEQ ID NO.95, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.73 or has at least 80%, 85%, 90% or 95% identity to the sequence as set forth in SEQ ID NO.73; or
(3) the sequence of the light chain CDR1 is the sequence as set forth in SEQ ID NO. 5 or has at least 80%, 85%, 90% or 95% identity to the sequence as set forth in SEQ ID NO.5, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.7 or has at least 80%, 85%, 90% or 95% identity to the sequence as set forth in SEQ ID NO.7; or
(4) the sequence of the light chain CDR1 is the sequence as set forth in SEQ ID NO. 13 or has at least 80%, 85%, 90% or 95% identity to the sequence as set forth in SEQ ID NO. 13 the sequence of the light chain CDR2 is the sequence as set forth in SEQ ID NO. 14 or has at least 80%, 85%, 90% or 95% identity to the sequence as set forth in SEQ ID NO. 14, and the sequence of the light chain CDR3 is the sequence as set forth in SEQ ID NO. 15 or has at least 80%, 85%, 90% or 95% identity to the sequence as set forth in SEQ ID NO. 15; or
(5) the sequence of the light chain CDR1 is the sequence as set forth in SEQ ID NO.21 or has at least 80%, 85%, 90% or 95% identity to the sequence as set forth in SEQ ID NO.21, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(6) the sequence of the light chain CDR1 is the sequence as set forth in SEQ ID NO.28 or has at least 80%, 85%, 90% or 95% identity to the sequence as set forth in SEQ ID NO.28, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.29 or has at least 80%, 85%, 90% or 95% identity to the sequence as set forth in SEQ ID NO.29; or
(7) the sequence of the light chain CDR1 is the sequence as set forth in SEQ ID NO.35 or has at least 80%, 85%, 90% or 95% identity to the sequence as set forth in SEQ ID NO.35, the sequence of the light chain CDR2 is the sequence as set forth in SEQ ID NO.36 or has at least 80%, 85%, 90% or 95% identity to the sequence as set forth in SEQ ID NO.36, and the sequence of the light chain CDR3 is the sequence as set forth in SEQ ID NO.37 or has at least 80%, 85%, 90% or 95% identity to the sequence as set forth in SEQ ID NO.37; or
(8) the sequence of the light chain CDR1 is the sequence as set forth in SEQ ID NO.43 or has at least 80%, 85%, 90% or 95% identity to the sequence as set forth in SEQ ID NO.43, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.22 or has at least 80%, 85%, 90% or 95% identity to the sequence as set forth in SEQ ID NO.22; or
(9) the sequence of the light chain CDR1 is the sequence as set forth in SEQ ID NO.49 or has at least 80%, 85%, 90% or 95% identity to the sequence as set forth in SEQ ID NO.49, the sequence of the light chain CDR2 is the sequence as set forth in SEQ ID NO.6 or has at least 80%, 85%, 90% or 95% identity to the sequence as set forth in SEQ ID NO.6, and the sequence of the light chain CDR3 is the sequence as set forth in SEQ ID NO.50 or has at least 80%, 85%, 90% or 95% identity to the sequence as set forth in SEQ ID NO.50; or
(10) the sequence of the light chain CDR1 is the sequence as set forth in SEQ ID NO.56 or has at least 80%, 85%, 90% or 95% identity to the sequence as set forth in SEQ ID NO.56, the sequence of the light chain CDR2 is the sequence as set forth in SEQ ID NO.57 or has at least 80%, 85%, 90% or 95% identity to the sequence as set forth in SEQ ID NO.57, and the sequence of the light chain CDR3 is the sequence as set forth in SEQ ID NO.58 or has at least 80%, 85%, 90% or 95% identity to the sequence as set forth in SEQ ID NO.58; or
(11) the sequence of the light chain CDR1 is the sequence as set forth in SEQ ID NO.64 or has at least 80%, 85%, 90% or 95% identity to the sequence as set forth in SEQ ID NO.64, the sequence of the light chain CDR2 is the sequence as set forth in SEQ ID NO.65 or has at least 80%, 85%, 90% or 95% identity to the sequence as set forth in SEQ ID NO.65, and the sequence of the light chain CDR3 is the sequence as set forth in SEQ ID NO.66 or has at least 80%, 85%, 90% or 95% identity to the sequence as set forth in SEQ ID NO.66; or
(12) the sequence of the light chain CDR1 is the sequence as set forth in SEQ ID NO.86 or has at least 80%, 85%, 90% or 95% identity to the sequence as set forth in SEQ ID NO.86 the sequence of the light chain CDR2 is the sequence as set forth in SEQ ID NO.87 or has at least 80%, 85%, 90% or 95% identity to the sequence as set forth in SEQ ID NO.87, and the sequence of the light chain CDR3 is the sequence as set forth in SEQ ID NO.88 or has at least 80%, 85%, 90% or 95% identity to the sequence as set forth in SEQ ID NO.88;
wherein the amino acid sequences of the light chain CDR1 to 3 are defined according to the IMGT definition.

9. The antibody or antigen-binding portion thereof of any one of claims 1 to 8, wherein:
the anti-GFRAL antibody is an intact antibody, a single chain fragment variable (scFv), a bispecific antibody, or a multispecific antibody; and/or
the antigen-binding portion of the anti-GFRAL antibody is Fab, Fab', Fv or F(ab')2; and/or
the anti-GFRAL antibody is a murine antibody, a chimeric antibody, a humanized antibody, or a fully human antibody; and/or
the anti-GFRAL antibody is a monoclonal antibody; and/or
the anti-GFRAL antibody is of IgG1, IgG2 or IgG4 isotype; and/or
the anti-GFRAL antibody comprises a light chain constant region of kappa subtype or lamda subtype; and/or
the anti-GFRAL antibody comprises a human IgG1 heavy chain constant region and a human kappa light chain constant region; and/or
the anti-GFRAL antibody comprises a human IgG4 heavy chain constant region and a human kappa light chain constant region.

10. An antibody or an antigen-binding portion thereof of any one of claims 1 to 9 for use in medical therapy.

11. An isolated nucleic acid molecule encoding the anti-GFRAL antibody or antigen-binding portion thereof of any one of claims 1 to 10.

12. A vector comprising the nucleic acid molecule of claim 11.

13. A host cell comprising the nucleic acid molecule of claim 11 or the vector of claim 12.

14. An antibody-drug conjugate comprising the anti-GFRAL antibody or antigen-binding portion thereof of any one of claims 1 to 10 conjugated with a therapeutic agent.

15. The antibody-drug conjugate of claim 14, wherein the therapeutic agent is an anti-tumor drug, e.g., a cytotoxic drug, an immunopotentiator, or a radioisotope.

16. A pharmaceutical composition comprising the anti-GFRAL antibody or antigen-binding portion thereof of any one of claims 1 to 10 or the antibody-drug conjugate of claim 14 or 15, and a pharmaceutically acceptable carrier.

17. A pharmaceutical composition of claim 16 for the treatment or prevention of a GFRAL-mediated or GFRAL-RET-GDF15 signaling pathway-mediated disease or abnormality, such as a cachexia syndrome (e.g. a cachexia syndrome caused by a tumor), a tumor (including a cancer), a cardiovascular disease, a renal disease, an ischemic disease, a metabolic disorder, a neurodegenerative disease, anorexia or weight loss caused by anorexia.

18. Use of the antibody or antigen-binding portion thereof of any one of claims 1 to 10, the nucleic acid molecule of claim 11, the vector of claim 12, the host cell of claim 13 or the antibody-drug conjugate of claim 14 or 15 for the manufacture of a medicament for the treatment or prevention of a GFRAL-mediated or GFRAL-RET-GDF15 signaling pathway-mediated disease or abnormality, such as a cachexia syndrome (e.g. a cachexia syndrome caused by a tumor), a tumor (including a cancer), a cardiovascular disease, a renal disease, an ischemic disease, a metabolic disorder, a neurodegenerative disease, anorexia or weight loss caused by anorexia.

19. A method of treating a GFRAL-mediated or GFRAL-RET-GDF15 signaling pathway-mediated disease or abnormality in a subject, comprising administering to the subject a therapeutically effective amount of the antibody or antigen-binding portion thereof of any one of claims 1 to 10, the antibody-drug conjugate of claim 14 or 15, or the pharmaceutical composition of claim 16, for example, the disease or abnormality is a cachexia syndrome (e.g. a cachexia syndrome caused by a tumor), a tumor (including a cancer), a cardiovascular disease, a renal disease, an ischemic disease, a metabolic disorder, a neurodegenerative disease, anorexia or weight loss caused by anorexia.

20. The pharmaceutical composition of claim 17 or the use of claim 18 or the method of claim 19, wherein the tumor (including a cancer) is a hematologic tumor or a solid tumor.

21. The pharmaceutical composition, use or method of claim 20, wherein the hematologic tumor is a lymphoma or leukemia, such as myeloma, B-cell lymphoma, mantle cell lymphoma, non-Hodgkin B-cell lymphoma, non-Hodgkin T-cell lymphoma, cutaneous lymphoma, anaplastic large cell lymphoma, multiple myeloma, inert non-Hodgkin lymphoma, plasmacytoma, chronic lymphocytic leukemia, small lymphocyte lymphoma, follicular lymphoma; optionally, the hematologic tumor is recurrent or refractory.

22. The pharmaceutical composition, use or method of claim 20, wherein the solid tumor is selected from the group consisting of a respiratory tumor, a digestive tract tumor, a urinary system tumor, a male organ tumor, a female organ tumor, skin cancer, an endothelial cell tumor, a brain tumor, a nervous system tumor, and an endocrine organ tumor.

23. The pharmaceutical composition, use or method of claim 20, wherein the solid tumor is selected from the group consisting of bladder cancer, brain cancer, breast cancer, cervical cancer, a thoracic tumor, endometrial cancer, esophageal squamous cancer, gastric cancer, a head tumor, pancreatic cancer, bile duct cancer, colorectal cancer, ocular cancer, head and neck squamous cancer, urothelial cancer, renal cancer, liver cancer, lymph node cancer, lung cancer, oral cancer, a neck tumor, ovarian cancer, prostate cancer, testicular cancer, laryngeal cancer, uterine cancer, melanoma, salivary adenocarcinoma, fibrosarcoma, soft tissue sarcoma and osteosarcoma; optionally, the solid tumor is recurrent or refractory.

24. A conjugate comprising the anti-GFRAL antibody or antigen-binding portion thereof of any one of claims 1 to 10 and a detectable label.

25. A fusion protein comprising the anti-GFRAL antibody or antigen-binding portion thereof of any one of claims 1 to 10.
